# EUROPEAN PATENT SPECIFICATION

(11) **EP 1 754 052 B1**
(45) Date of publication and mention of the grant of the patent: **22.07.2015**
(21) Application number: 05746849.8
(22) Date of filing: 03.06.2005
(51) Int. Cl.: G01N 33/50, C07K 7/00, C12N 5/00, C12N 15/10, C12Q 1/68

(54) **PEPTIDE MODULATORS OF CELLULAR PHENOTYPE AND BI-NUCLEIC ACID FRAGMENT LIBRARY**
MODULATOREN BIOCHEMISCHER EIGENSCHAFTEN
MODULATEURS DE CARACTERISTIQUES BIOCHIMIQUES

(30) Priority: 03.06.2004 AU 2004903002
(43) Date of publication of application: 21.02.2007
(73) Proprietor: Phylogica Limited, Crawley, WA 3009 (AU)
(72) Inventor: WATT, Paul, Michael, Mount Claremont, Western Australia 6010 (AU); HOPKINS, Richard, North Perth, Western Australia 6006 (AU); FEAR, Mark, Kardinya, Western Australia 6163 (AU); MILECH, Nadia, Marian, Dorothy, Daglish, Western Australia 6008 (AU)
(74) Representative: Leonard, Thomas Charles
(86) International application number: PCT/AU2005/000801
(87) International publication number: WO 2005/119244

(56) References cited:
- EP-A1- 1 277 835
- WO-A-00/76308
- WO-A-01/11086
- WO-A1-00/76308
- WO-A1-01/25461
- WO-A1-03/004528
- WO-A1-03/010540
- WO-A2-01/11086
- WO-A2-01/75178
- WO-A2-02/29101
- WO-A2-98/15172
- WO-A2-02/060946
- WO-A2-02/086111
- WO-A2-02/088314
- WO-A2-03/012055
- WO-A2-03/040168
- WO-A2-03/046147
- WO-A2-03/076621
- AU-B2- 756 617
- AU-B2- 771 534
- US-A- 5 834 247
- US-A1- 2002 155 564
- US-A1- 2002 164 735
- US-B1- 6 225 530
- US-B1- 6 436 694
- US-B1- 6 475 726
- US-B1- 6 720 413
- US-B1- 6 846 625
- US-B2- 6 521 425
- TORTOSA PABLO ET AL: "Characterization of ylbF, a new gene involved in competence development and sporulation in Bacillus subtilis" MOLECULAR MICROBIOLOGY, vol. 35, no. 5, March 2000 (2000-03), pages 1110-1119, XP002517610 ISSN: 0950-382X
- ANDRE S ET AL: 'Identification of peptide ligands for malignancy- and growth-regulating galectins using random phage-display and designed combinatorial peptide libraries.' BIOINORGANIC & MEDICINAL CHEMISTRY. vol. 13, no. 2, 17 January 2005, pages 563 - 573, XP004681540
- MILLER VL ET AL: 'Identification of regions of Ail required for the invasion and serum resistance phenotypes.' MOLECULAR MICROBIOLOGY. vol. 41, no. 5, September 2001, pages 1053 - 1062, XP008115273
- TORTOSA P ET AL: 'Characterization of ylbF, a new gene involved in competence development and sporulation in Bacillus subtilis.' MOLECULAR MICROBIOLOGY. vol. 35, no. 5, March 2000, pages 1110 - 1119, XP002517610
- LAYNE MD ET AL: 'Aortic carboxypeptidase-like protein, a novel protein with discoidin and carboxypeptidase-like domains, is up-regulated during vascular smooth muscle cell differentiation' THE JOURNAL OD BIOLOGICAL CHEMISTRY. vol. 273, no. 25, 19 June 1998, pages 15654 - 15660, XP008115452
- HALEY KJ ET AL: 'Tumor necrosis factor induces neuroendocrine differentiation in small cell lung cancer cell lines.' AMERICAN JOURNAL OF PHYSIOLOGY. vol. 275, no. 2 PT 1, August 1998, pages L311 - 21, XP000853970
- VALENTINI SR ET AL: 'Glucocorticoid-regulated gene in transformed to normal phenotypic reversion.' BRAZILIAN JOURNAL OF MEDICAL AND BIOLOGICAL RESEARCH. vol. 27, no. 2, February 1994, pages 541 - 546, XP008115274
- BRODIN NT ET AL: 'Rat monoclonal antibodies produced against rat colorectal adenocarcinomas define tumor- and colon-associated, auto-immunogenic antigens.' INTERNATIONAL JOURNAL OF CANCER. vol. 45, no. 5, 15 May 1990, pages 902 - 910, XP008115792
- FUTCH WS ET AL: 'Dissection of macrophage tumoricidal and protozoacidal activities using T-cell hybridomas and recombinant lymphokines.' INFECTION AND IMMUNITY. vol. 50, no. 3, December 1985, pages 709 - 715, XP008115275
- URBANEK M ET AL: 'Variation in resistin gene promoter not associated with polycystic ovary syndrome.' DIABETES. vol. 52, January 2003, pages 214 - 217, XP008115276
- YANG P ET AL: 'Direct activation of the fission yeast PAK Shk1 by the novel SH3 domain protein, Skb5.' THE JOURNAL OF BIOLOGICAL CHEMISTRY. vol. 274, 17 December 1999, pages 36052 - 36057, XP008115317
- CHOI Y ET AL: 'Identification of bioactive molecules by adipogenesis profiling of organic compounds.' FASEB JOURNAL. vol. 17, no. 4-5, March 2003, page A605, XP008115318
- KOO JH ET AL: 'Purification and characterization of Bex, an OMP parter.' FASEB JOURNAL. vol. 15, no. 5, 08 March 2001, page A894, XP008115453

### SEQUENCE LISTING

<110> Phylogica Limited
<120> Modulators of biochemical characteristics
<130> 122839
<150> 2004903002 <151> 2004-06-03
<160> 63
<170> PatentIn version 3.3
<210> 1
   <211> 6
   <212> DNA
   <213> synthetic nucleic acid Kozak sequence
<220>
   <221> misc_feature
   <222> (2)..(3)
   <223> n is a, c, g, or t
<400> 1
   rnnatg 6
<210> 2
   <211> 8
   <212> DNA
   <213> synthetic nucleic acid eukaryotic Kozak sequence
<400> 2
   ccrccatg 8
<210> 3
   <211> 11
   <212> DNA
   <213> synthetic nucleic acid eukaryotic Kozak sequence
<400> 3
   gccagccatg g 11
<210> 4
   <211> 8
   <212> DNA
   <213> synthetic nucleic acid plant Kozak sequence
<400> 4
   ctaccatg 8
<210> 5
   <211> 10
   <212> DNA
   <213> synthetic nucleic acid Shine Dalgarno sequence
<400> 5
   gaagaagata 10
<210> 6
   <211> 7
   <212> DNA
   <213> synthetic nucleic acid eukaryotic translational slippage sequence
<400> 6
   aaaaaac 7
<210> 7
   <211> 7
   <212> DNA
   <213> synthetic nucleic acid eukaryotic translational slippage sequence
<400> 7
   aaattta 7
<210> 8
   <211> 7
   <212> DNA
   <213> synthetic nucleic acid eukaryotic translational slippage sequence.
<400> 8
   aaatttt 7
<210> 9
   <211> 7
   <212> DNA
   <213> synthetic nucleic acid eukaryotic translational slippage sequence
<400> 9
   gggaaac 7
<210> 10
   <211> 7
   <212> DNA
   <213> synthetic nucleic acid eukaryotic translational slippage sequence
<400> 10
   gggcccc 7
<210> 11
   <211> 7
   <212> DNA
   <213> synthetic nucleic acid eukaryotic translational slippage sequence
<400> 11
   gggttta 7
<210> 12
   <211> 7
   <212> DNA
   <213> synthetic nucleic acid eukaryotic translational slippage sequence
<400> 12
   gggtttt 7
<210> 13
   <211> 7
   <212> DNA
   <213> synthetic nucleic acid eukaryotic translational slippage sequence
<400> 13
   tttaaac 7
<210> 14
   <211> 7
   <212> DNA
   <213> synthetic nucleic acid eukaryotic translational slippage sequence
<400> 14
   tttaaat 7
<210> 15
   <211> 6
   <212> DNA
   <213> synthetic nucleic acid eukaryotic translational slippage sequence
<400> 15
   ttttta 6
<210> 16
   <211> 7
   <212> DNA
   <213> synthetic nucleic acid eukaryotic translational slippage sequence
<400> 16
   ggattta 7
<210> 17
   <211> 7
   <212> DNA
   <213> synthetic nucleic acid yeast translational slippage sequence
<400> 17
   cttaggc 7
<210> 18
   <211> 7
   <212> DNA
   <213> synthetic nucleic acid yeast translational slippage sequence
<400> 18
   gcgagtt 7
<210> 19
   <211> 7
   <212> DNA
   <213> synthetic nucleic acid mammalian translational slippage sequence
<400> 19
   tcctgat 7
<210> 20
   <211> 7
   <212> DNA
   <213> synthetic nucleic acid prokaryotic translational slippage sequence
<400> 20
   aaaaaag 7
<210> 21
   <211> 7
   <212> DNA
   <213> synthetic nucleic acid prokaryotic translational slippage sequence
<400> 21
   aaaaaaa 7
<210> 22
   <211> 7
   <212> DNA
   <213> synthetic nucleic acid prokaryotic translational slippage sequence
<400> 22
   aaaaaac 7
<210> 23
   <211> 7
   <212> DNA
   <213> synthetic nucleic acid prokaryotic translational slippage sequence
<400> 23
   gggaaag 7
<210> 24
   <211> 7
   <212> DNA
   <213> synthetic nucleic acid prokaryotic translational slippage sequence
<400> 24
   aaaaggg 7
<210> 25
   <211> 7
   <212> DNA
   <213> synthetic nucleic acid prokaryotic translational slippage sequence
<400> 25
   gggaaaa 7
<210> 26
   <211> 7
   <212> DNA
   <213> synthetic nucleic acid prokaryotic translational slippage sequence
<400> 26
   tttaaag 7
<210> 27
   <211> 7
   <212> DNA
   <213> synthetic nucleic acid prokaryotic translational slippage sequence
<400> 27
   aaagggg 7
<210> 28
   <211> 3
   <212> DNA
   <213> synthetic nucleic acid bacterial translational slippage sequence
<400> 28
   ctt 3
<210> 29
   <211> 17
   <212> PRT
   <213> synthetic or recombinant peptide Drosophila penetratin
<400> 29
   Cys Arg Gln Ile Lys Ile Trp Phe Gln Asn Arg Arg Met Lys Trp Lys
   1 5 10 15
   Lys
<210> 30
   <211> 21
   <212> PRT
   <213> synthetic or recombinant peptide Pep1
<400> 30
   Lys Glu Thr Trp Trp Glu Thr Trp Trp Thr Glu Trp Ser Gln Lys Lys
   1 5 10 15
   Lys Lys Arg Lys Val
   20
<210> 31
   <211> 13
   <212> PRT
   <213> synthetic or recombinant peptide HIV-1 Tat
<400> 31
   Gly Arg Lys Lys Arg Arg Gln Arg Arg Arg Pro Pro Gln
   1 5 10
<210> 32
   <211> 27
   <212> PRT
   <213> synthetic or recombinant peptide - signal sequence based peptide 1
<400> 32
   Gly Ala Leu Phe Leu Gly Trp Leu Gly Ala Ala Gly Ser Thr Met Gly
   1 5 10 15
   Ala Trp Ser Gln Pro Lys Lys Lys Arg Lys Val
   20 25
<210> 33
   <211> 16
   <212> PRT
   <213> synthetic or recombinant peptide - signal sequence based peptide 2
<400> 33
   Ala Ala Val Ala Leu Leu Pro Ala Val Leu Leu Ala Leu Leu Ala Pro
   1 5 10 15
<210> 34
   <211> 26
   <212> PRT
   <213> synthetic or recombinant peptide - transportan
<400> 34
   Gly Trp Thr Leu Asn Ser Ala Gly Tyr Leu Leu Lys Ile Asn Leu Lys
   1               5                   10                  15
   Ala Leu Ala Ala Leu Ala Lys Lys Ile Leu
               20                  25
<210> 35
   <211> 18
   <212> PRT
   <213> synthetic or recombinant peptide - amphiphilic model peptide
<400> 35
   Lys Leu Ala Leu Lys Leu Ala Leu Lys Ala Leu Lys Ala Ala Leu Lys
   1               5                   10                  15
   Leu Ala
<210> 36
   <211> 11
   <212> PRT
   <213> synthetic or recombinant peptide - polyarginine
<400> 36
   Arg Arg Arg Arg Arg Arg Arg Arg Arg Arg Arg
   1               5                   10
<210> 37
   <211> 1212
   <212> DNA
   <213> Homo sapiens Aurora A kinase (Aurora 2 kinase, AIK1, STK15 or BTAK)
<220>
   <221> CDS
   <222> (1)..(1212)
<400> 37
   atg gac cga tct aaa gaa aac tgc att tca gga cct gtt aag gct aca       48
   Met Asp Arg Ser Lys Glu Asn Cys Ile Ser Gly Pro Val Lys Ala Thr
   1               5                   10                  15
   gct cca gtt gga ggt cca aaa cgt gtt ctc gtg act cag caa att cct       96
   Ala Pro Val Gly Gly Pro Lys Arg Val Leu Val Thr Gln Gln Ile Pro
               20                  25                  30
   tgt cag aat cca tta cct gta aat agt ggc cag gct cag cgg gtc ttg      144
   Cys Gln Asn Pro Leu Pro Val Asn Ser Gly Gln Ala Gln Arg Val Leu
           35                  40                  45
   tgt cct tca aat tct tcc cag cgc gtt cct ttg caa gca caa aag ctt      192
   Cys Pro Ser Asn Ser Ser Gln Arg Val Pro Leu Gln Ala Gln Lys Leu
       50                  55                  60
   gtc tcc agt cac aag ccg gtt cag aat cag aag cag aag caa ttg cag      240
   Val Ser Ser His Lys Pro Val Gln Asn Gln Lys Gln Lys Gln Leu Gln
   65                  70                  75                  80
   gca acc agt gta cct cat cct gtc tcc agg cca ctg aat aac acc caa      288
   Ala Thr Ser Val Pro His Pro Val Ser Arg Pro Leu Asn Asn Thr Gln
                   85                  90                  95
   aag agc aag cag ccc ctg cca tcg gca cct gaa aat aat cct gag gag      336
   Lys Ser Lys Gln Pro Leu Pro Ser Ala Pro Glu Asn Asn Pro Glu Glu
               100                 105                 110
   gaa ctg gca tca aaa cag aaa aat gaa gaa tca aaa aag agg cag tgg      384
   Glu Leu Ala Ser Lys Gln Lys Asn Glu Glu Ser Lys Lys Arg Gln Trp
           115                 120                 125
   gct ttg gaa gac ttt gaa att ggt cgc cct ctg ggt aaa gga aag ttt      432
   Ala Leu Glu Asp Phe Glu Ile Gly Arg Pro Leu Gly Lys Gly Lys Phe
       130                 135                 140
   ggt aat gtt tat ttg gca aga gaa aag caa agc aag ttt att ctg gct      480
   Gly Asn Val Tyr Leu Ala Arg Glu Lys Gln Ser Lys Phe Ile Leu Ala
   145                 150                 155                 160
   ctt aaa gtg tta ttt aaa gct cag ctg gag aaa gcc gga gtg gag cat      528
   Leu Lys Val Leu Phe Lys Ala Gln Leu Glu Lys Ala Gly Val Glu His
                   165                 170                 175
   cag ctc aga aga gaa gta gaa ata cag tcc cac ctt cgg cat cct aat      576
   Gln Leu Arg Arg Glu Val Glu Ile Gln Ser His Leu Arg His Pro Asn
               180                 185                 190
   att ctt aga ctg tat ggt tat ttc cat gat gct acc aga gtc tac cta      624
   Ile Leu Arg Leu Tyr Gly Tyr Phe His Asp Ala Thr Arg Val Tyr Leu
           195                 200                 205
   att ctg gaa tat gca cca ctt gga aca gtt tat aga gaa ctt cag aaa      672
   Ile Leu Glu Tyr Ala Pro Leu Gly Thr Val Tyr Arg Glu Leu Gln Lys
       210                 215                 220
   ctt tca aag ttt gat gag cag aga act gct act tat ata aca gaa ttg      720
   Leu Ser Lys Phe Asp Glu Gln Arg Thr Ala Thr Tyr Ile Thr Glu Leu
   225                 230                 235                 240
   gca aat gcc ctg tct tac tgt cat tcg aag aga gtt att cat aga gac      768
   Ala Asn Ala Leu Ser Tyr Cys His Ser Lys Arg Val Ile His Arg Asp
                   245                 250                 255
   att aag cca gag aac tta ctt ctt gga tca gct gga gag ctt aaa att      816
   Ile Lys Pro Glu Asn Leu Leu Leu Gly Ser Ala Gly Glu Leu Lys Ile
               260                 265                 270
   gca gat ttt ggg tgg tca gta cat gct cca tct tcc agg agg acc act      864
   Ala Asp Phe Gly Trp Ser Val His Ala Pro Ser Ser Arg Arg Thr Thr
           275                 280                 285
   ctc tgt ggc acc ctg gac tac ctg ccc cct gaa atg att gaa ggt cgg      912
   Leu Cys Gly Thr Leu Asp Tyr Leu Pro Pro Glu Met Ile Glu Gly Arg
       290                 295                 300
   atg cat gat gag aag gtg gat ctc tgg agc ctt gga gtt ctt tgc tat      960
   Met His Asp Glu Lys Val Asp Leu Trp Ser Leu Gly Val Leu Cys Tyr
   305                 310                 315                 320
   gaa ttt tta gtt ggg aag cct cct ttt gag gca aac aca tac caa gag     1008
   Glu Phe Leu Val Gly Lys Pro Pro Phe Glu Ala Asn Thr Tyr Gln Glu
                   325                 330                 335
   acc tac aaa aga ata tca cgg gtt gaa ttc aca ttc cct gac ttt gta     1056
   Thr Tyr Lys Arg Ile Ser Arg Val Glu Phe Thr Phe Pro Asp Phe Val
               340                 345                 350
   aca gag gga gcc agg gac ctc att tca aga ctg ttg aag cat aat ccc     1104
   Thr Glu Gly Ala Arg Asp Leu Ile Ser Arg Leu Leu Lys His Asn Pro
           355                 360                 365
   agc cag agg cca atg ctc aga gaa gta ctt gaa cac ccc tgg atc aca     1152
   Ser Gln Arg Pro Met Leu Arg Glu Val Leu Glu His Pro Trp Ile Thr
       370                 375                 380
   gca aat tca tca aaa cca tca aat tgc caa aac aaa gaa tca gct agc     1200
   Ala Asn Ser Ser Lys Pro Ser Asn Cys Gln Asn Lys Glu Ser Ala Ser
   385                 390                 395                 400
   aaa cag tct tag 1212
   Lys Gln Ser
<210> 38
   <211> 403
   <212> PRT
   <213> Homo sapiens Aurora A kinase (Aurora 2 kinase, AIK1, STK15 or BTAK)
<400> 38
   Met Asp Arg Ser Lys Glu Asn Cys Ile Ser Gly Pro Val Lys Ala Thr
   1               5                   10                  15
   Ala Pro Val Gly Gly Pro Lys Arg Val Leu Val Thr Gln Gln Ile Pro
               20                  25                  30
   Cys Gln Asn Pro Leu Pro Val Asn Ser Gly Gln Ala Gln Arg Val Leu
           35                  40                  45
   Cys Pro Ser Asn Ser Ser Gln Arg Val Pro Leu Gln Ala Gln Lys Leu
       50                  55                  60
   Val Ser Ser His Lys Pro Val Gln Asn Gln Lys Gln Lys Gln Leu Gln
   65                  70                  75                  80
   Ala Thr Ser Val Pro His Pro Val Ser Arg Pro Leu Asn Asn Thr Gln
                   85                  90                  95
   Lys Ser Lys Gln Pro Leu Pro Ser Ala Pro Glu Asn Asn Pro Glu Glu
               100                 105                 110
   Glu Leu Ala Ser Lys Gln Lys Asn Glu Glu Ser Lys Lys Arg Gln Trp
           115                 120                 125
   Ala Leu Glu Asp Phe Glu Ile Gly Arg Pro Leu Gly Lys Gly Lys Phe
       130                 135                 140
   Gly Asn Val Tyr Leu Ala Arg Glu Lys Gln Ser Lys Phe Ile Leu Ala
   145                 150                 155                 160
   Leu Lys Val Leu Phe Lys Ala Gln Leu Glu Lys Ala Gly Val Glu His
                   165                 170                 175
   Gln Leu Arg Arg Glu Val Glu Ile Gln Ser His Leu Arg His Pro Asn
               180                 185                 190
   Ile Leu Arg Leu Tyr Gly Tyr Phe His Asp Ala Thr Arg Val Tyr Leu
           195                 200                 205
   Ile Leu Glu Tyr Ala Pro Leu Gly Thr Val Tyr Arg Glu Leu Gln Lys
       210                 215                 220
   Leu Ser Lys Phe Asp Glu Gln Arg Thr Ala Thr Tyr Ile Thr Glu Leu
   225                 230                 235                 240
   Ala Asn Ala Leu Ser Tyr Cys His Ser Lys Arg Val Ile His Arg Asp
                   245                 250                 255
   Ile Lys Pro Glu Asn Leu Leu Leu Gly Ser Ala Gly Glu Leu Lys Ile
               260                 265                 270
   Ala Asp Phe Gly Trp Ser Val His Ala Pro Ser Ser Arg Arg Thr Thr
                   275                 280                 285
   Leu Cys Gly Thr Leu Asp Tyr Leu Pro Pro Glu Met Ile Glu Gly Arg
       290                 295                 300
   Met His Asp Glu Lys Val Asp Leu Trp Ser Leu Gly Val Leu Cys Tyr
   305                 310                 315                 320
   Glu Phe Leu Val Gly Lys Pro Pro Phe Glu Ala Asn Thr Tyr Gln Glu
                   325                 330                 335
   Thr Tyr Lys Arg Ile Ser Arg Val Glu Phe Thr Phe Pro Asp Phe Val
               340                 345                 350
   Thr Glu Gly Ala Arg Asp Leu Ile Ser Arg Leu Leu Lys His Asn Pro
           355                 360                 365
   Ser Gln Arg Pro Met Leu Arg Glu Val Leu Glu His Pro Trp Ile Thr
       370                 375                 380
   Ala Asn Ser Ser Lys Pro Ser Asn Cys Gln Asn Lys Glu Ser Ala Ser
   385                 390                 395                 400
   Lys Gln Ser
<210> 39
   <211> 1188
   <212> DNA
   <213> Homo sapiens cyclin E
<220>
   <221> CDS
   <222> (1)..(1188)
<400> 39
   atg aag gag gac ggc ggc gcg gag ttc tcg gct cgc tcc agg aag agg      48
   Met Lys Glu Asp Gly Gly Ala Glu Phe Ser Ala Arg Ser Arg Lys Arg
   1               5                   10                  15
   aag gca aac gtg acc gtt ttt ttg cag gat cca gat gaa gaa atg gcc      96
   Lys Ala Asn Val Thr Val Phe Leu Gln Asp Pro Asp Glu Glu Met Ala
               20                  25                  30
   aaa atc gac agg acg gcg agg gac cag tgt ggg agc cag cct tgg gac     144
   Lys Ile Asp Arg Thr Ala Arg Asp Gln Cys Gly Ser Gln Pro Trp Asp
           35                  40                  45
   aat aat gca gtc tgt gca gac ccc tgc tcc ctg atc ccc aca cct gac     192
   Asn Asn Ala Val Cys Ala Asp Pro Cys Ser Leu Ile Pro Thr Pro Asp
       50                  55                  60
   aaa gaa gat gat gac cgg gtt tac cca aac tca acg tgc aag cct cgg     240
   Lys Glu Asp Asp Asp Arg Val Tyr Pro Asn Ser Thr Cys Lys Pro Arg
   65                  70                  75                  80
   att att gca cca tcc aga ggc tcc ccg ctg cct gta ctg agc tgg gca     288
   Ile Ile Ala Pro Ser Arg Gly Ser Pro Leu Pro Val Leu Ser Trp Ala
                   85                  90                  95
   aat aga gag gaa gtc tgg aaa atc atg tta aac aag gaa aag aca tac     336
   Asn Arg Glu Glu Val Trp Lys Ile Met Leu Asn Lys Glu Lys Thr Tyr
               100                 105                 110
   tta agg gat cag cac ttt ctt gag caa cac cct ctt ctg cag cca aaa      384
   Leu Arg Asp Gln His Phe Leu Glu Gln His Pro Leu Leu Gln Pro Lys
           115                 120                 125
   atg cga gca att ctt ctg gat tgg tta atg gag gtg tgt gaa gtc tat      432
   Met Arg Ala Ile Leu Leu Asp Trp Leu Met Glu Val Cys Glu Val Tyr
       130                 135                 140
   aaa ctt cac agg gag acc ttt tac ttg gca caa gat ttc ttt gac cgg      480
   Lys Leu His Arg Glu Thr Phe Tyr Leu Ala Gln Asp Phe Phe Asp Arg
   145                 150                 155                 160
   tat atg gcg aca caa gaa aat gtt gta aaa act ctt tta cag ctt att      528
   Tyr Met Ala Thr Gln Glu Asn Val Val Lys Thr Leu Leu Gln Leu Ile
                   165                 170                 175
   ggg att tca tct tta ttt att gca gcc aaa ctt gag gaa atc tat cct      576
   Gly Ile Ser Ser Leu Phe Ile Ala Ala Lys Leu Glu Glu Ile Tyr Pro
               180                 185                 190
   cca aag ttg cac cag ttt gcg tat gtg aca gat gga gct tgt tca gga      624
   Pro Lys Leu His Gln Phe Ala Tyr Val Thr Asp Gly Ala Cys Ser Gly
           195                 200                 205
   gat gaa att ctc acc atg gaa tta atg att atg aag gcc ctt aag tgg      672
   Asp Glu Ile Leu Thr Met Glu Leu Met Ile Met Lys Ala Leu Lys Trp
       210                 215                 220
   cgt tta agt ccc ctg act att gtg tcc tgg ctg aat gta tac atg cag      720
   Arg Leu Ser Pro Leu Thr Ile Val Ser Trp Leu Asn Val Tyr Met Gln
   225                 230                 235                 240
   gtt gca tat cta aat gac tta cat gaa gtg cta ctg ccg cag tat ccc      768
   Val Ala Tyr Leu Asn Asp Leu His Glu Val Leu Leu Pro Gln Tyr Pro
                   245                 250                 255
   cag caa atc ttt ata cag att gca gag ctg ttg gat ctc tgt gtc ctg      816
   Gln Gln Ile Phe Ile Gln Ile Ala Glu Leu Leu Asp Leu Cys Val Leu
               260                 265                 270
   gat gtt gac tgc ctt gaa ttt cct tat ggt ata ctt gct gct tcg gcc      864
   Asp Val Asp Cys Leu Glu Phe Pro Tyr Gly Ile Leu Ala Ala Ser Ala
           275                 280                 285
   ttg tat cat ttc tcg tca tct gaa ttg atg caa aag gtt tca ggg tat      912
   Leu Tyr His Phe Ser Ser Ser Glu Leu Met Gln Lys Val Ser Gly Tyr
       290                 295                 300
   cag tgg tgc gac ata gag aac tgt gtc aag tgg atg gtt cca ttt gcc      960
   Gln Trp Cys Asp Ile Glu Asn Cys Val Lys Trp Met Val Pro Phe Ala
   305                 310                 315                 320
   atg gtt ata agg gag acg ggg agc tca aaa ctg aag cac ttc agg ggc     1008
   Met Val Ile Arg Glu Thr Gly Ser Ser Lys Leu Lys His Phe Arg Gly
                   325                 330                 335
   gtc gct gat gaa gat gca cac aac ata cag acc cac aga gac agc ttg     1056
   Val Ala Asp Glu Asp Ala His Asn Ile Gln Thr His Arg Asp Ser Leu
               340                 345                 350
   gat ttg ctg gac aaa gcc cga gca aag aaa gcc atg ttg tct gaa caa     1104
   Asp Leu Leu Asp Lys Ala Arg Ala Lys Lys Ala Met Leu Ser Glu Gln
           355                 360                 365
   aat agg gct tct cct ctc ccc agt ggg ctc ctc acc ccg cca cag agc      1152
   Asn Arg Ala Ser Pro Leu Pro Ser Gly Leu Leu Thr Pro Pro Gln Ser
       370                 375                 380
   ggt aag aag cag agc agc ggg ccg gaa atg gcg tga                      1188
   Gly Lys Lys Gln Ser Ser Gly Pro Glu Met Ala
   385                 390                 395
<210> 40
   <211> 395
   <212> PRT
   <213> Homo sapiens cyclin E
<400> 40
   Met Lys Glu Asp Gly Gly Ala Glu Phe Ser Ala Arg Ser Arg Lys Arg
   1               5                   10                  15
   Lys Ala Asn Val Thr Val Phe Leu Gln Asp Pro Asp Glu Glu Met Ala
               20                  25                  30
   Lys Ile Asp Arg Thr Ala Arg Asp Gln Cys Gly Ser Gln Pro Trp Asp
           35                  40                  45
   Asn Asn Ala Val Cys Ala Asp Pro Cys Ser Leu Ile Pro Thr Pro Asp
       50                  55                  60
   Lys Glu Asp Asp Asp Arg Val Tyr Pro Asn Ser Thr Cys Lys Pro Arg
   65                  70                  75                  80
   Ile Ile Ala Pro Ser Arg Gly Ser Pro Leu Pro Val Leu Ser Trp Ala
                   85                  90                  95
   Asn Arg Glu Glu Val Trp Lys Ile Met Leu Asn Lys Glu Lys Thr Tyr
               100                 105                 110
   Leu Arg Asp Gln His Phe Leu Glu Gln His Pro Leu Leu Gln Pro Lys
           115                 120                 125
   Met Arg Ala Ile Leu Leu Asp Trp Leu Met Glu Val Cys Glu Val Tyr
       130                 135                 140
   Lys Leu His Arg Glu Thr Phe Tyr Leu Ala Gln Asp Phe Phe Asp Arg
   145                 150                 155                 160
   Tyr Met Ala Thr Gln Glu Asn Val Val Lys Thr Leu Leu Gln Leu Ile
                   165                 170                 175
   Gly Ile Ser Ser Leu Phe Ile Ala Ala Lys Leu Glu Glu Ile Tyr Pro
               180                 185                 190
   Pro Lys Leu His Gln Phe Ala Tyr Val Thr Asp Gly Ala Cys Ser Gly
           195                 200                 205
   Asp Glu Ile Leu Thr Met Glu Leu Met Ile Met Lys Ala Leu Lys Trp
       210                 215                 220
   Arg Leu Ser Pro Leu Thr Ile Val Ser Trp Leu Asn Val Tyr Met Gln
   225                 230                 235                 240
   Val Ala Tyr Leu Asn Asp Leu His Glu Val Leu Leu Pro Gln Tyr Pro
                   245                 250                 255
   Gln Gln Ile Phe Ile Gln Ile Ala Glu Leu Leu Asp Leu Cys Val Leu
   260 265 270
   Asp Val Asp Cys Leu Glu Phe Pro Tyr Gly Ile Leu Ala Ala Ser Ala
   275 280 285
   Leu Tyr His Phe Ser Ser Ser Glu Leu Met Gln Lys Val Ser Gly Tyr
   290 295 300
   Gln Trp Cys Asp Ile Glu Asn Cys Val Lys Trp Met Val Pro Phe Ala
   305 310 315 320
   Met Val Ile Arg Glu Thr Gly Ser Ser Lys Leu Lys His Phe Arg Gly
   325 330 335
   Val Ala Asp Glu Asp Ala His Asn Ile Gln Thr His Arg Asp Ser Leu
   340 345 350
   Asp Leu Leu Asp Lys Ala Arg Ala Lys Lys Ala Met Leu Ser Glu Gln
   355 360 365
   Asn Arg Ala Ser Pro Leu Pro Ser Gly Leu Leu Thr Pro Pro Gln Ser
   370 375 380
   Gly Lys Lys Gln Ser Ser Gly Pro Glu Met Ala
   385 390 395
<210> 41
   <211> 2023
   <212> DNA
   <213> homo sapiens cyclin dependent kinase 2 (CDK2)
<220>
   <221> CDS
   <222> (1)..(897)
<400> 41
   atg gag aac ttc caa aag gtg gaa aag atc gga gag ggc acg tac gga 48
   Met Glu Asn Phe Gln Lys Val Glu Lys Ile Gly Glu Gly Thr Tyr Gly
   1 5 10 15
   gtt gtg tac aaa gcc aga aac aag ttg acg gga gag gtg gtg gcg ctt 96
   Val Val Tyr Lys Ala Arg Asn Lys Leu Thr Gly Glu Val Val Ala Leu
   20 25 30
   aag aaa atc cgc ctg gac act gag act gag ggt gtg ccc agt act gcc 144
   Lys Lys Ile Arg Leu Asp Thr Glu Thr Glu Gly Val Pro Ser Thr Ala
   35 40 45
   atc cga gag atc tct ctg ctt aag gag ctt aac cat cct aat att gtc 192
   Ile Arg Glu Ile Ser Leu Leu Lys Glu Leu Asn His Pro Asn Ile Val
   50 55 60
   aag ctg ctg gat gtc att cac aca gaa aat aaa ctc tac ctg gtt ttt 240
   Lys Leu Leu Asp Val Ile His Thr Glu Asn Lys Leu Tyr Leu Val Phe
   65 70 75 80
   gaa ttt ctg cac caa gat ctc aag aaa ttc atg gat gcc tct gct ctc 288
   Glu Phe Leu His Gln Asp Leu Lys Lys Phe Met Asp Ala Ser Ala Leu
   85 90 95
   act ggc att cct ctt ccc ctc atc aag agc tat ctg ttc cag ctg ctc 336
   Thr Gly Ile Pro Leu Pro Leu Ile Lys Ser Tyr Leu Phe Gln Leu Leu
   100 105 110
   cag ggc cta gct ttc tgc cat tct cat cgg gtc ctc cac cga gac ctt      384
   Gln Gly Leu Ala Phe Cys His Ser His Arg Val Leu His Arg Asp Leu
           115                 120                 125
   aaa cct cag aat ctg ctt att aac aca gag ggg gcc atc aag cta gca      432
   Lys Pro Gln Asn Leu Leu Ile Asn Thr Glu Gly Ala Ile Lys Leu Ala
       130                 135                 140
   gac ttt gga cta gcc aga gct ttt gga gtc cct gtt cgt act tac acc      480
   Asp Phe Gly Leu Ala Arg Ala Phe Gly Val Pro Val Arg Thr Tyr Thr
   145                 150                 155                 160
   cat gag gtg gtg acc ctg tgg tac cga gct cct gaa atc ctc ctg ggc      528
   His Glu Val Val Thr Leu Trp Tyr Arg Ala Pro Glu Ile Leu Leu Gly
                   165                 170                 175
   tgc aaa tat tat tcc aca gct gtg gac atc tgg agc ctg ggc tgc atc      576
   Cys Lys Tyr Tyr Ser Thr Ala Val Asp Ile Trp Ser Leu Gly Cys Ile
               180                 185                 190
   ttt gct gag atg gtg act cgc cgg gcc cta ttc cct gga gat tct gag      624
   Phe Ala Glu Met Val Thr Arg Arg Ala Leu Phe Pro Gly Asp Ser Glu
           195                 200                 205
   att gac cag ctc ttc cgg atc ttt cgg act ctg ggg acc cca gat gag      672
   Ile Asp Gln Leu Phe Arg Ile Phe Arg Thr Leu Gly Thr Pro Asp Glu
       210                 215                 220
   gtg gtg tgg cca gga gtt act tct atg cct gat tac aag cca agt ttc      720
   Val Val Trp Pro Gly Val Thr Ser Met Pro Asp Tyr Lys Pro Ser Phe
   225                 230                 235                 240
   ccc aag tgg gcc cgg caa gat ttt agt aaa gtt gta cct ccc ctg gat      768
   Pro Lys Trp Ala Arg Gln Asp Phe Ser Lys Val Val Pro Pro Leu Asp
                   245                 250                 255
   gaa gat gga cgg agc ttg tta tcg caa atg ctg cac tac gac cct aac      816
   Glu Asp Gly Arg Ser Leu Leu Ser Gln Met Leu His Tyr Asp Pro Asn
               260                 265                 270
   aag cgg att tcg gcc aag gca gcc ctg gct cac cct ttc ttc cag gat      864
   Lys Arg Ile Ser Ala Lys Ala Ala Leu Ala His Pro Phe Phe Gln Asp
           275                 280                 285
   gtg acc aag cca gta ccc cat ctt cga ctc tga tagccttctt gaagccccca    917
   Val Thr Lys Pro Val Pro His Leu Arg Leu
       290                 295
   gccctaatct caccctctcc tccagtgtgg gcttgaccag gcttgcgctt gggctatttg    977
   gactcaggtg ggccctctga acttgcctta aacactcacc ttctagtctt ggccagccaa   1037
   ctctgggaat acaggggtga aaggggggaa ccagtgaaaa tgaaaggaag tttcagtatt  1097
   agatgcactt aagttagcct ccaccaccct ttcccccttc tcttagttat tgctgaagag    1157
   ggttggtata aaaataattt taaaaaagcc ttcctacacg ttagatttgc cgtaccaatc   1217
   tctgaatgcc ccataattat tatttccagt gtttgggatg accaggatcc caagcctcct   1277
   gctgccacaa tgtttataaa ggccaaatga tagcgggggc taagttggtg cttttgagaa   1337
   ccaagtaaaa caaaaccact gggaggagtc tattttaaag aattcggttg aaaaaaatag   1397
   atccaatcag tttataccct agttagtgtt ttgcctcacc taataggctg ggagactgaa   1457
   gactcagccc gggtggctgc agaaaaatga ttggccccag tccccttgtt tgtcccttct   1517
   acaggcatga ggaatctggg aggccctgag acagggattg tgcttcattc caatctattg   1577
   cttcaccatg gccttatgag gcaggtgaga gatgtttgaa tttttctctt ccttttagta   1637
   ttcttagttg ttcagttgcc aaggatccct gatcccattt tcctctgacg tccacctcct   1697
   accccatagg agttagaagt tagggtttag gcatcatttt gagaatgctg acactttttc   1757
   agggctgtga ttgagtgagg gcatgggtaa aaatatttct ttaaaagaag gatgaacaat   1817
   tatatttata tttcaggtta tatccaatag tagagttggc tttttttttt tttttttggt   1877
   catagtgggt ggatttgttg ccatgtgcac cttggggttt tgtaatgaca gtgctaaaaa   1937
   aaaagcattt tttttttatg atttgtctct gtcacccttg tccttgagtg ctcttgctat   1997
   taacgttatt tgtaatttag tttgta                                         2023
<210> 42
   <211> 298
   <212> PRT
   <213> homo sapiens cyclin dependent kinase 2 (CDK2)
<400> 42
   Met Glu Asn Phe Gln Lys Val Glu Lys Ile Gly Glu Gly Thr Tyr Gly
   1               5                   10                  15
   Val Val Tyr Lys Ala Arg Asn Lys Leu Thr Gly Glu Val Val Ala Leu
               20                  25                  30
   Lys Lys Ile Arg Leu Asp Thr Glu Thr Glu Gly Val Pro Ser Thr Ala
           35                  40                  45
   Ile Arg Glu Ile Ser Leu Leu Lys Glu Leu Asn His Pro Asn Ile Val
       50                  55                  60
   Lys Leu Leu Asp Val Ile His Thr Glu Asn Lys Leu Tyr Leu Val Phe
   65                  70                  75                  80
   Glu Phe Leu His Gln Asp Leu Lys Lys Phe Met Asp Ala Ser Ala Leu
                   85                  90                  95
   Thr Gly Ile Pro Leu Pro Leu Ile Lys Ser Tyr Leu Phe Gln Leu Leu
               100                 105                 110
   Gln Gly Leu Ala Phe Cys His Ser His Arg Val Leu His Arg Asp Leu
           115                 120                 125
   Lys Pro Gln Asn Leu Leu Ile Asn Thr Glu Gly Ala Ile Lys Leu Ala
       130                 135                 140
   Asp Phe Gly Leu Ala Arg Ala Phe Gly Val Pro Val Arg Thr Tyr Thr
   145                 150                 155                 160
   His Glu Val Val Thr Leu Trp Tyr Arg Ala Pro Glu Ile Leu Leu Gly
                   165                 170                 175
   Cys Lys Tyr Tyr Ser Thr Ala Val Asp Ile Trp Ser Leu Gly Cys Ile
               180                 185                 190
   Phe Ala Glu Met Val Thr Arg Arg Ala Leu Phe Pro Gly Asp Ser Glu
           195                 200                 205
   Ile Asp Gln Leu Phe Arg Ile Phe Arg Thr Leu Gly Thr Pro Asp Glu
       210                 215                 220
   Val Val Trp Pro Gly Val Thr Ser Met Pro Asp Tyr Lys Pro Ser Phe
   225                 230                 235                 240
   Pro Lys Trp Ala Arg Gln Asp Phe Ser Lys Val Val Pro Pro Leu Asp
                   245                 250                 255
   Glu Asp Gly Arg Ser Leu Leu Ser Gln Met Leu His Tyr Asp Pro Asn
               260                 265                 270
   Lys Arg Ile Ser Ala Lys Ala Ala Leu Ala His Pro Phe Phe Gln Asp
           275                 280                 285
   Val Thr Lys Pro Val Pro His Leu Arg Leu
       290                 295
<210> 43
   <211> 40
   <212> DNA
   <213> synthetic oligonucleotide
<400> 43
   agaggaattc aggtcagact acaaggacga cgacgacaag 40
<210> 44
   <211> 27
   <212> DNA
   <213> synthetic oligonucleotide
<400> 44
   cagaagctta aggacgacga cgacaag 27
<210> 45
   <211> 27
   <212> DNA
   <213> synthetic oligonucleotide
<400> 45
   cagaagctta aggacgacga cgacaag 27
<210> 46
   <211> 28
   <212> DNA
   <213> synthetic oligonucleotide
<400> 46
   caggaattcc aaggacgacg acgacaag 28
<210> 47
   <211> 29
   <212> DNA
   <213> synthetic oligonucleotide
<400> 47
   caggaattca caaggacgac cgacgacaag 29
<210> 48
   <211> 16
   <212> DNA
   <213> synthetic oligonucleotide
<400> 48
   aattcgaacc ccttcg 16
<210> 49
   <211> 12
   <212> DNA
   <213> synthetic oligonucleotide
<400> 49
   cgaaggggtt cg 12
<210> 50
   <211> 17
   <212> DNA
   <213> synthetic oligonucleotide
<400> 50
   aattcgaacc ccttcgc 17
<210> 51
   <211> 13
   <212> DNA
   <213> synthetic oligonucleotide
<400> 51
   gcgaaggggt tcg 13
<210> 52
   <211> 18
   <212> DNA
   <213> synthetic oligonucleotide
<400> 52
   aattcgaacc ccttcgcg 18
<210> 53
   <211> 14
   <212> DNA
   <213> synthetic oligonucleotide
<400> 53
   cgcgaagggg ttcg 14
<210> 54
   <211> 16
   <212> DNA
   <213> synthetic oligonucleotide
<400> 54
   agctcgaagg ggttcg 16
<210> 55
   <211> 12
   <212> DNA
   <213> synthetic oligonucleotide
<400> 55
   cgaacccctt cg 12
<210> 56
   <211> 600
   <212> DNA
   <213> Homo sapiens Aurora interacting protein (AIP)
<220>
   <221> CDS
   <222> (1)..(600)
<400> 56
   atg ctc ctg ggg cgc ctg act tcc cag ctg ttg agg gcc gtt cct tgg     48
   Met Leu Leu Gly Arg Leu Thr Ser Gln Leu Leu Arg Ala Val Pro Trp
   1               5                   10                  15
   gca ggc ggc cgc ccg cct tgg ccc gtc tct gga gtg ctg ggc agc cgg     96
   Ala Gly Gly Arg Pro Pro Trp Pro Val Ser Gly Val Leu Gly Ser Arg
               20                  25                  30
   gtc tgc ggg ccc ctt tac agc aca tcg ccg gcc ggc cca ggt agg gcg    144
   Val Cys Gly Pro Leu Tyr Ser Thr Ser Pro Ala Gly Pro Gly Arg Ala
           35                  40                  45
   gcc tct ctc cct cgc aag ggg gcc cag ctg gag ctg gag gag atg ctg    192
   Ala Ser Leu Pro Arg Lys Gly Ala Gln Leu Glu Leu Glu Glu Met Leu
       50                  55                  60
   gtc ccc agg aag atg tcc gtc agc ccc ctg gag agc tgg ctc acg gcc    240
   Val Pro Arg Lys Met Ser Val Ser Pro Leu Glu Ser Trp Leu Thr Ala
   65                  70                  75                  80
   cgc tgc ttc ctg ccc aga ctg gat acc ggg acc gca ggg act gtg gct    288
   Arg Cys Phe Leu Pro Arg Leu Asp Thr Gly Thr Ala Gly Thr Val Ala
                   85                  90                  95
   cca ccg caa tcc tac cag tgt ccg ccc agc cag ata ggg gaa ggg gcc    336
   Pro Pro Gln Ser Tyr Gln Cys Pro Pro Ser Gln Ile Gly Glu Gly Ala
               100                 105                 110
   gag cag ggg gat gaa ggc gtc gcg gat gcg cct caa att cag tgc aaa    384
   Glu Gln Gly Asp Glu Gly Val Ala Asp Ala Pro Gln Ile Gln Cys Lys
           115                 120                 125
   aac gtg ctg aag atc cgc cgg cgg aag atg aac cac cac aag tac cgg    432
   Asn Val Leu Lys Ile Arg Arg Arg Lys Met Asn His His Lys Tyr Arg
       130                 135                 140
   aag ctg gtg aag aag acg cgg ttc ctg cgg agg aag gtc cag gag gga    480
   Lys Leu Val Lys Lys Thr Arg Phe Leu Arg Arg Lys Val Gln Glu Gly
   145                 150                 155                 160
   cgc ctg aga cgc aag cag atc aag ttc gag aaa gac ctg agg cgc atc    528
   Arg Leu Arg Arg Lys Gln Ile Lys Phe Glu Lys Asp Leu Arg Arg Ile
                   165                 170                 175
   tgg ctg aag gcg ggg cta aag gaa gcc ccc gaa ggc tgg cag acc ccc    576
   Trp Leu Lys Ala Gly Leu Lys Glu Ala Pro Glu Gly Trp Gln Thr Pro
               180                 185                 190
   aag atc tac ctg cgg ggc aaa tga                                    600
   Lys Ile Tyr Leu Arg Gly Lys
           195
<210> 57
   <211> 199
   <212> PRT
   <213> Homo sapiens Aurora interacting protein (AIP)
<400> 57
   Met Leu Leu Gly Arg Leu Thr Ser Gln Leu Leu Arg Ala Val Pro Trp
   1               5                   10                  15
   Ala Gly Gly Arg Pro Pro Trp Pro Val Ser Gly Val Leu Gly Ser Arg
               20                  25                  30
   Val Cys Gly Pro Leu Tyr Ser Thr Ser Pro Ala Gly Pro Gly Arg Ala
           35                  40                  45
   Ala Ser Leu Pro Arg Lys Gly Ala Gln Leu Glu Leu Glu Glu Met Leu
       50                  55                  60
   Val Pro Arg Lys Met Ser Val Ser Pro Leu Glu Ser Trp Leu Thr Ala
   65                  70                  75                  80
   Arg Cys Phe Leu Pro Arg Leu Asp Thr Gly Thr Ala Gly Thr Val Ala
                   85                  90                  95
   Pro Pro Gln Ser Tyr Gln Cys Pro Pro Ser Gln Ile Gly Glu Gly Ala
               100                 105                 110
   Glu Gln Gly Asp Glu Gly Val Ala Asp Ala Pro Gln Ile Gln Cys Lys
           115                 120                 125
   Asn Val Leu Lys Ile Arg Arg Arg Lys Met Asn His His Lys Tyr Arg
       130                 135                 140
   Lys Leu Val Lys Lys Thr Arg Phe Leu Arg Arg Lys Val Gln Glu Gly
   145                 150                 155                 160
   Arg Leu Arg Arg Lys Gln Ile Lys Phe Glu Lys Asp Leu Arg Arg Ile
                   165                 170                 175
   Trp Leu Lys Ala Gly Leu Lys Glu Ala Pro Glu Gly Trp Gln Thr Pro
               180                 185                 190
   Lys Ile Tyr Leu Arg Gly Lys
           195
<210> 58
   <211> 21
   <212> DNA
   <213> synthetic oligonucleotide for amplifying AIP
<400> 58
   cgctgccgat cggggccgac t 21
<210> 59
   <211> 21
   <212> DNA
   <213> synthetic oligonucleotide for amplifying AIP
<400> 59
   cgctgccgat cggggccgac t 21
<210> 60
   <211> 495
   <212> DNA
   <213> p21
<220>
   <221> CDS
   <222> (1)..(495)
<400> 60
   atg tca gaa ccg gct ggg gat gtc cgt cag aac cca tgc ggc agc aag      48
   Met Ser Glu Pro Ala Gly Asp Val Arg Gln Asn Pro Cys Gly Ser Lys
   1               5                   10                  15
   gcc tgc cgc cgc ctc ttc ggc cca gtg gac agc gag cag ctg agc cgc      96
   Ala Cys Arg Arg Leu Phe Gly Pro Val Asp Ser Glu Gln Leu Ser Arg
               20                  25                  30
   gac tgt gat gcg cta atg gcg ggc tgc atc cag gag gcc cgt gag cga      144
   Asp Cys Asp Ala Leu Met Ala Gly Cys Ile Gln Glu Ala Arg Glu Arg
           35                  40                  45
   tgg aac ttc gac ttt gtc acc gag aca cca ctg gag ggt gac ttc gcc      192
   Trp Asn Phe Asp Phe Val Thr Glu Thr Pro Leu Glu Gly Asp Phe Ala
       50                  55                  60
   tgg gag cgt gtg cgg ggc ctt ggc ctg ccc aag ctc tac ctt ccc acg      240
   Trp Glu Arg Val Arg Gly Leu Gly Leu Pro Lys Leu Tyr Leu Pro Thr
   65                  70                  75                  80
   ggg ccc cgg cga ggc cgg gat gag ttg gga gga ggc agg cgg cct ggc      288
   Gly Pro Arg Arg Gly Arg Asp Glu Leu Gly Gly Gly Arg Arg Pro Gly
                   85                  90                  95
   acc tca cct gct ctg ctg cag ggg aca gca gag gaa gac cat gtg gac      336
   Thr Ser Pro Ala Leu Leu Gln Gly Thr Ala Glu Glu Asp His Val Asp
               100                 105                 110
   ctg tca ctg tct tgt acc ctt gtg cct cgc tca ggg gag cag gct gaa      384
   Leu Ser Leu Ser Cys Thr Leu Val Pro Arg Ser Gly Glu Gln Ala Glu
           115                 120                 125
   ggg tcc cca ggt gga cct gga gac tct cag ggt cga aaa cgg cgg cag      432
   Gly Ser Pro Gly Gly Pro Gly Asp Ser Gln Gly Arg Lys Arg Arg Gln
       130                 135                 140
   acc agc atg aca gat ttc tac cac tcc aaa cgc cgg ctg atc ttc tcc      480
   Thr Ser Met Thr Asp Phe Tyr His Ser Lys Arg Arg Leu Ile Phe Ser
   145                 150                 155                 160
   aag agg aag ccc taa                                                  495
   Lys Arg Lys Pro
<210> 61
   <211> 164
   <212> PRT
   <213> p21
<400> 61
   Met Ser Glu Pro Ala Gly Asp Val Arg Gln Asn Pro Cys Gly Ser Lys
   1               5                   10                  15
   Ala Cys Arg Arg Leu Phe Gly Pro Val Asp Ser Glu Gln Leu Ser Arg
               20                  25                  30
   Asp Cys Asp Ala Leu Met Ala Gly Cys Ile Gln Glu Ala Arg Glu Arg
           35                  40                  45
   Trp Asn Phe Asp Phe Val Thr Glu Thr Pro Leu Glu Gly Asp Phe Ala
       50                  55                  60
   Trp Glu Arg Val Arg Gly Leu Gly Leu Pro Lys Leu Tyr Leu Pro Thr
   65                  70                  75                  80
   Gly Pro Arg Arg Gly Arg Asp Glu Leu Gly Gly Gly Arg Arg Pro Gly
                   85                  90                  95
   Thr Ser Pro Ala Leu Leu Gln Gly Thr Ala Glu Glu Asp His Val Asp
               100                 105                 110
   Leu Ser Leu Ser Cys Thr Leu Val Pro Arg Ser Gly Glu Gln Ala Glu
           115                 120                 125
   Gly Ser Pro Gly Gly Pro Gly Asp Ser Gln Gly Arg Lys Arg Arg Gln
       130                 135                 140
   Thr Ser Met Thr Asp Phe Tyr His Ser Lys Arg Arg Leu Ile Phe Ser
   145                 150                 155                 160
   Lys Arg Lys Pro
<210> 62
   <211> 31
   <212> DNA
   <213> synthetic oligonucleotide - T7MfeN9
<220>
   <221> misc_feature
   <222> (23)..(31)
   <223> n is a, c, g, or t
<400> 62
   gtaatacgac tcatacaatt gcnnnnnnnn n 31
<210> 63
   <211> 22
   <212> DNA
   <213> synthetic oligonucleotide - T7Mfe
<400> 63
   gtaatacgac tcatacaatt gc 22

## Description

### Field of the invention

The present invention relates to non-hybrid screening methods for the identification and/or isolation of a peptide that is capable of modulating a phenotype in a cell, tissue or organism.

### Background of the invention

### General

This specification contains nucleotide and amino acid sequence information prepared using PatentIn Version 3.3, presented herein after the claims. Each nucleotide sequence is identified in the sequence listing by the numeric indicator <210> followed by the sequence identifier (e.g. <210>1, <210>2, <210>3, etc). The length and type of sequence (DNA, protein (PRT), etc), and source organism for each nucleotide sequence, are indicated by information provided in the numeric indicator fields <211>, <212> and <213>, respectively. Nucleotide sequences referred to in the specification are defined by the term "SEQ ID NO:", followed by the sequence identifier (eg. SEQ ID NO: 1 refers to the sequence in the sequence listing designated as <400>1).

The designation of nucleotide residues referred to herein are those recommended by the IUPAC-IUB Biochemical Nomenclature Commission, wherein A represents Adenine, C represents Cytosine, G represents Guanine, T represents thymine, Y represents a pyrimidine residue, R represents a purine residue, M represents Adenine or Cytosine, K represents Guanine or Thymine, S represents Guanine or Cytosine, W represents Adenine or Thymine, H represents a nucleotide other than Guanine, B represents a nucleotide other than Adenine, V represents a nucleotide other than Thymine, D represents a nucleotide other than Cytosine and N represents any nucleotide residue.

As used herein the term "derived from" shall be taken to indicate that a specified integer may be obtained from a particular source albeit not necessarily directly from that source.

Throughout this specification, unless the context requires otherwise, the word "comprise", or variations such as "comprises" or "comprising", will be understood to imply the inclusion of a stated step or element or integer or group of steps or elements or integers but not the exclusion of any other step or element or integer or group of elements or integers.

Throughout this specification, unless specifically stated otherwise or the context requires otherwise, reference to a single step, composition of matter, group of steps or group of compositions of matter shall be taken to encompass one and a plurality (i.e., one or more) of those steps, compositions of matter, groups of steps or group of compositions of matter.

Each embodiment described herein is to be applied *mutatis mutandis* to each and every other embodiment unless specifically stated otherwise.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations or any two or more of said steps or features.

The present invention is not to be limited in scope by the specific embodiments described herein, which are intended for the purpose of exemplification only. Functionally-equivalent products, compositions and methods are clearly within the scope of the invention, as described herein. The subject matter for which protection is sought is as defined in the claims.

The present invention is performed without undue experimentation using, unless otherwise indicated, conventional techniques of molecular biology, microbiology, virology, recombinant DNA technology, peptide synthesis in solution, solid phase peptide synthesis, and immunology. Such procedures are described, for example, in the following texts:
1. Sambrook, J. and Russell, D. W., Molecular Cloning: A Laboratory Manual, Cold Spring Harbour Laboratory Press, Cold Spring Harbour, New York. Third Edition (2001), whole of Vols I, II, and III;
2. DNA Cloning: A Practical Approach, Vols. I and II (D. N. Glover, ed., 1985), IRL Press, Oxford, whole of text;
3. Oligonucleotide Synthesis: A Practical Approach (M. J. Gait, ed., 1984) IRL Press, Oxford, whole of text, and particularly the papers therein by Gait, pp1-22; Atkinson *et al*., pp35-81; Sproat *et al.*, pp 83-115; and Wu *et al*., pp 135-151;
4. Nucleic Acid Hybridization: A Practical Approach (B. D. Hames & S. J. Higgins, eds., 1985) IRL Press, Oxford, whole of text;
5. Animal Cell Culture: Practical Approach, Third Edition (John R.W. Masters, ed., 2000), ISBN 0199637970, whole of text;
6. Immobilized Cells and Enzymes: A Practical Approach (1986) IRL Press, Oxford, whole of text;
7. Perbal, B., A Practical Guide to Molecular Cloning (1984);
8. Methods In Enzymology (S. Colowick and N. Kaplan, eds., Academic Press, Inc.), whole of series;
9. J.F. Ramalho Ortigao, "The Chemistry of Peptide Synthesis" In: Knowledge database of Access to Virtual Laboratory website (Interactiva, Germany);
10. Sakakibara, D., Teichman, J., Lien, E. Land Fenichel, R.L. (1976). Biochem. Biophys. Res. Commun. 73 336-342
11. Merrifield, R.B. (1963). J. Am. Chem. Soc. 85, 2149-2154.
12. Barany, G. and Merrifield, R.B. (1979) in The Peptides (Gross, E. and Meienhofer, J. eds.), vol. 2, pp. 1-284, Academic Press, New York.
13. Wünsch, E., ed. (1974) Synthese von Peptiden in Houben-Weyls Metoden der Organischen Chemie (Müler, E., ed.), vol. 15, 4th edn., Parts 1 and 2, Thieme, Stuttgart.
14. Bodanszky, M. (1984) Principles of Peptide Synthesis, Springer-Verlag, Heidelberg.
15. Bodanszky, M. & Bodanszky, A. (1984) The Practice of Peptide Synthesis, Springer-Verlag, Heidelberg.
16. Bodanszky, M. (1985) Int. J. Peptide Protein Res. 25, 449-474.
17. Handbook of Experimental Immunology, Vols. I-IV (D. M. Weir and C. C. Blackwell, eds., 1986, Blackwell Scientific Publications).
18. Hogan et al Manipulating the Mouse Embryo. A Laboratory Manual, 2nd Edition. Cold Spring Harbour Laboratory. ISBN: 0879693843, 1994.
19. Ausubel, F. M., Brent, R, Kingston, R. E., Moore, D. D., Seidman, J. G., and Struhl, K. (Editors). Current Protocols in Molecular Biology, John Wiley and Sons, New York (1987), whole of volumes.
20. Scopes Protein purification: principles and practice, Third Edition, Springer Verlag, 1994

### Background of the related art

As a response to the increasing demand for new lead compounds and new target identification and validation reagents, the pharmaceutical industry has increased its screening of various sources for new lead compounds having a unique activity or specificity in therapeutic applications, such as, for example, in the treatment of neoplastic disorders, infection, modulating immunity, autoimmunity, inflammation or fertility, amongst others.

A large number of diseases, including those listed *supra* are caused by or linked to a genetic modification or mutation. Substantial effort is expended to determine therapeutic compounds that suppress or compensate for such mutant genes at the transcriptional, translational or functional level.

One class of such therapeutic compounds comprises therapeutic peptides, such as, for example, a random peptide aptamer. However, random peptide aptamers often show little or none of the secondary or tertiary structure required to efficiently bind to a target molecule. Furthermore, random peptide aptamers are often unstable. International Application No. PCT/AU00/00414. describes libraries of peptides that overcome problems associated with random peptide aptamers. The peptides described in PCT/AU00/00414 are derived from natural sources and mimic the native structure of a domain or subdomain of a natural protein. Such natural protein domains or subdomains have been selected in nature to form stable secondary structures that enable them to bind to, for example, other proteins or nucleic acids with high affinity.

It is known to identify a candidate therapeutic peptide or "lead" using hybrid screening of peptide libraries. Such hybrid screening is useful for determining a peptide that binds to a target (forward hybrid screening) or a peptide that inhibits the interaction of two or more targets (reverse hybrid screening). Hybrid screening methods generally require the formulation of known drug targets into binding partners that interact to reconstitute a molecule, e.g., a transcription factor, capable of regulating the expressionof a reporter molecule.

For example, in a conventional forward two-hybrid screen the protein of interest is expressed as a fusion protein with the DNA binding domain (DBD) of a transcription factor. A transcriptional activation domain (AD) of the transcription factor is expressed separately as a fusion with each member of a library of peptides. The fusion proteins are then expressed in a cell that comprises a reporter gene, the expression of which is under control of the transcription factor (i.e., comprising the DBD and AD). When the appropriate association between binding partners occurs in the cell, a functional transcription factor is reconstituted and the reporter gene is expressed. The cell expressing a peptide that binds the target is then isolated and/or identified.

Reverse two-hybrid screening methods also express a fusion protein comprising a first protein of interest fused to a DBD. A protein that is known to interact with the first protein is expressed as a fusion with an AD. These two proteins are introduced into a cell that comprises a reporter gene that is expressed in the presence of a reconstituted transcription factor. However, a reverse two-hybrid screen differs from a forward N-hybrid screen by providing a selection against the interaction of the two proteins, for example, by expressing a counter-selectable reporter gene when the two proteins interact. Accordingly, by introducing a peptide into the cell and selecting against cells that express the counter-selectable reporter gene an inhibitor of the protein interaction is identified.

The skilled person will also be aware of numerous variations of standard hybrid screens, e.g., a one-hybrid screen, a three-hybrid screen, a split-hybrid screen, a Sos recruitment screen or an ubiquitin-based split protein sensor screen.

All forms of hybrid screen known in the art require prior knowledge of at least one member of a protein-DNA or protein-protein interaction. Accordingly, hybrid screens generally permit the identification of candidate therapeutic peptides that modulate known targets. This clearly limits the applicability of hybrid screens to the identification of peptides that are therapeutic of a disease or disorder in which a specific protein-DNA or protein-protein interaction is known to be a causative factor.

Furthermore, N-hybrid screening requires prior cloning and expression of nucleic acid encoding at least one protein target Accordingly, such screens are labor intensive and time consuming.

International patent application published as WO 01/11086 relates to methods of diagnosis of angiogenesis and compositions and methods of screening for angiogenesis modulators. The method of screening drug candidates comprises providing a cell that expresses an expression profile gene which encodes a particular protein, adding a drug candidate to said cell, and determining the effect of said drug candidate on the expression of said expression profile gene.

International application published as WO 00/76308 relates to animal models and methods for analysis of lipid metabolism and screening of pharmaceutical and pesticidal agents that modulate lipid metabolism. Drosophilia melanogaster and C. *elegans* were genetically modified to express or mis-express proteins involved in the sterol regulatory element binding protein (SREBP) pathway. The animal models have phenotypes for studying lipid metabolism.

Tortosa et al, Molecular Microbiology, 35(5), 1110-1119 (2000) describe characterisation of *ylbF,* a gene involved in competence development and sporulation in *Bacillus subtilis.* The authors used mini Tn*10* transposition to generate a library of *Bacillus subtilis* insertion mutants, with a goal of identifying and characterising new competence genes. The disruption of one of these genes (*ylbF*) leads to a dramatic decrease in the expression of *comK,* encoding the competence transcription factor.

Clearly, there is a need in the art for a means of rapidly identifying candidate peptide leads that modulate cellular phenotypes, without prior knowledge of the precise cellular mechanisms involved, i.e., without prior knowledge of the target protein, nucleic acid, biochemical pathway or regulatory pathway responsible for expression of the phenotype. There is also considerable benefit to be derived from a simplified screening protocol that delivers lead peptides at lower cost than standard procedures.

### Summary of invention

In work leading up to the present invention the inventors sought to avoid the time consuming and labor-intensive steps assocated with hybrid screens, yet identify a peptide capable of modulating a phenotype of interest.

The subject matter for which protection is sought is as defined in the claims.

The screening method of the invention is a non-hybrid screening method for identifying a peptide that rescues a cell, tissue or non-human organism from death induced by expression of a heterologous peptide, polypeptide, protein or allele, said method comprising:
(i) using a cell, tissue or non-human organism which has been obtained or selected, wherein death of the cell, tissue or non-human organism is induced by expression of a heterologous peptide, polypeptide, protein or allele;
(ii) before inducing death of the cell, tissue or non-human organism which has been obtained and selected at (i), expressing in the cell, tissue or non-human organism or introducing into the cell, tissue or non-human organism or contacting the cell, tissue or non-human organism with a candidate peptide wherein said candidate peptide is encoded by a genome of a prokaryote organism having a genome size of less than 1700 mega-base pairs (Mbp)
(iii) inducing death of the cell, tissue or non-human organism and selecting a cell, tissue or non-human organism that survives;
(iv) identifying from a cell, tissue or non-human organism selected at (iii) the expressed or introduced peptide; and
(v) identifying a peptide that is not contained within a protein that induces survival when expressed in a cell, tissue or animal expressing the heterologous peptide, polypeptide, protein or allele,
   wherein the cell is a yeast, insect, plant or mammalian cell.

By screening on this basis, the inventors enrich for peptides that have the biological activity of interest. Because the screens identified by the inventors detect or measure a phenotype of a cell, tissue or organism, it is not necessary to identify a gene or protein that is associated with or causative of the phenotype as a preliminary step.

As exemplified herein, in one embodiment of the invention, the inventors have produced a screen to identify a peptide that mimics the structure of a protein domain or subdomain and that is capable of modulating the tumorigenic state of a cell. This peptide is identified by overexpressing Aurora-A kinase protein in a yeast cell that also expresses a peptide that mimics the structure of a protein domain. Overexpression of Aurora-A kinase in yeast cells causes cell death. Those cells that survive and grow are considered to express a peptide capable of rescuing the defect associated with Aurora-A overexpression. As overexpression of this protein is also observed in various human tumors, the identified peptides are also considered to be capable of modulating the tumorigenic state of a cell.

Other exemplified embodiments of the disclosure provide methods for determining a peptide that induces or prevents cytokine signaling. For example, methods are provided to determine a peptide that induces interlekin-3 (IL-3) signaling, granulocyte-colony stimulating factor (G-CSF), granulocyte/macrophage- colony stimulating factor (GM-CSF) or erythropoietin (epo).

These and other exemplified embodiments provide a model for identifying a peptide that is capable of modulating any phenotype, e.g., a phenotype associated with a disease and/or disorder. Such a peptide is useful not only for the development of new therapeutics, but also for the identification of new drug targets (e.g., a protein with which a peptide identified using the screening method of the present invention interacts). Methods of the invention identify peptides that rescuse a cell, tissue or non-human organism from death by expression of a heterologous peptide, polypeptide, protein or allele.

Without being bound by theory or mode of action, this screen is based on the inventors' understanding that protein interactions within a specific organism have often been selected to be transitional or in dynamic equilibrium. By using peptides encoded by nucleic acid derived from an organism that is unrelated to the organism in which the phenotype of interest occurs, the number of peptides that bind to cellular components with high affinity and thereby efficiently modulate a phenotype is enriched.

The term "unrelated" shall be understood to mean that the organisms are unrelated at the taxonomic level. For example, it is preferable that the two organisms are from different taxonomic classes or phyla/divisions. However, to enrich for peptides that are capable of binding to a cellular component with high affinity it is preferred that the peptide is derived from one or more organisms that are from a different taxonomic kingdom to the cell, tissue or organism used to perform the screening method. For example, should the screen be performed in a mammalian cell, tissue or organism, the peptide is preferably produced from (or a library of peptides is produced from) one or more organisms from a kingdom such as, for example, Prokaryotae/Monera (e.g., bacterium), Protista (e.g., a protozoan), Fungi or Plantae. However, should the screen be performed in a yeast, the peptide is preferably produced from (or a library of peptides is produced from) one or more organisms from a kingdom such as, for example, Prokaryotae/Monera (e.g., bacterium), Protista (e.g., a protozoan), Plantae or Animalia.

In embodiments of the invention, the peptide or library of peptides screened using the method of the invention is derived from a prokaryote organism having a compact genome with a size of less than 1700 Mbp. The advantages of such libraries of peptides are described further herein. For example, a library of peptides derived from one or more organisms having a compact genome have a large number of naturally occurring protein domains that are considered to be capable of modulating a phenotype of interest.

In this regard, it is preferable that the peptide or library of peptides is screened using a cell, tissue or organism having a complex genome, (e.g., the peptide is derived from a bacterium and is screened using a mammalian cell).

The term "complex genome" shall be taken to mean a genome that comprises more than about 1700 mega-base pairs (Mbp), preferably, more than about 1000 Mbp, and even more preferably, more than about 500 Mbp.

In another embodiment, a complex genome comprises a large degree of repetitive nucleic acid. For example, a complex genome comprises more repetitive nucleic acid than a yeast or a bacteria or *Takifugu rubripes.* For example, a complex genome comprises a similar level of repetitive nucleic acid to that observed in a human. Such information can be determined from information from NCBI or TIGR.

As used herein the term "NCBI" shall be taken to mean the database of the National Center for Biotechnology Information at the National Library of Medicine at the National Institutes of Health of the Government of the United States of America, Bethesda, MD, 20894.

As used herein the term "TIGR" shall be taken to mean the database of The Institute of Genomic Research, Rockville, MD, 20850.

In a further embodiment, a complex genome has a low level of gene density. For example, less than about 15% of the genome of the cell, tissue or organism having a complex genome comprises an open reading frame. By way of example, *T. rubripes* has a gene density of about 16% compared to humans, who have a gene density of about 3%. Preferably, less than about 12% of the genome of a complex genome comprises an open reading frame; more preferably, less than about 10%, even more preferably, less than about 7%.

Suitable organisms comprising a complex genome will be apparent to the skilled artisan. For example, as many bacteria comprise compact genomes, it is preferable that the screening method of the invention is performed in a eukaryotic cell. Suitable cells, tissues and/or organisms will be apparent to the skilled person and include, for example, an insect cell, an insect, a plant cell, a plant, a mammalian cell or a mammal.

As will be apparent from the foregoing a "compact genome" comprises less than about 1700 mega-base pairs (Mbp), preferably, less than about 1000 Mbp, more preferably, less than about 500 Mbp, even more preferably, less than about 100 Mbp, still more preferably, less than about 50 Mbp and still more preferably, less than about 13 Mbp.

In another embodiment, a compact genome has a high level of gene density. For example, more than about 15% of the genome comprises an open reading frame, e.g., more than about 20% or 30% or 40% or 50% or 60% or 70% or 80% of the genome comprises an open reading frame.

Suitable eukaryotic and/or prokaryotic genomes will be apparent to the skilled person based on the description herein. For example, a suitable compact prokaryotic genome is a bacterial genome.

As used herein, the term "non-hybrid" shall be taken to mean that the screen of the instant invention does not make use of any known hybrid screening method, such as, for example, a forward N-hybrid, reverse N-hybrid, a split-hybrid, a tribrid system, a PolIII hybrid system, a repressor hybrid, Sos recruitment screen or a ubiquitin-based split protein sensor system. Such screening system generally requires producing a fusion protein between a test protein and a protein, polypeptide or peptide that is capable of binding to DNA (a DNA binding domain) and/or that comprises or consists of a transcriptional activation domain. The present invention does not require the production of such a fusion protein. Accordingly, the present invention clearly provides an advantage over hybrid systems, in that it is not necessary to isolate one or more proteins of interest and produce a fusion protein prior to performing a screen to identify a peptide.

As will be apparent to the skilled artisan, hybrid screens require at least one member of a protein interaction being studied is known. In contrast to hybrid screens, the present invention requires no prior knowledge about cell components that confer a phenotype of interest. Merely, a phenotype of interest is known and/or detectable and/or measurable. By detecting or measuring the phenotype or a change in the phenotype a peptide of interest is determined.

Nor does the present invention require reconstitution of a transcription factor to induce expression of a reporter gene. Rather, the peptide being tested modulates a cell component to thereby modulate the phenotype of interest.

The term "phenotype" is to be taken in its broadest context to mean any biochemical or physical characteristic of an organism or cell. Accordingly, the term "phenotype" shall also encompass any biochemical or physical characteristic of an organism or cell that is determined by the genetic composition of a cell, tissue or organism or both the genetic composition of the organism or cell and the environment in which the organism or cell subsists. Preferred phenotypes are those that are measurable, such as, for example, cell death and/or survival, cell proliferation, gene expression, metabolism and signal transduction, amongst others.

A cell, tissue or organism that is "capable of expressing" the phenotype of interest encompasses a cell, tissue or organism having the phenotype or a cell tissue or organism having the potential to have the phenotype. For example, in the case of the phenotype being cell death induced by expression of a gene a cell, tissue or organism that comprises the gene and capable of expressing the gene is capable of expressing the phenotype of interest. Clearly, in the case of such a phenotype it is beneficial to express a modulatory peptide prior to inducing expression of the gene that causes cell death.

As used herein, the term "protein domain" shall be taken to mean a discrete portion or region of a protein that assumes a secondary structure or conformation sufficient to permit said portion to perform a specific function in the context of another protein or a nucleic acid. In particular, the secondary structure of the protein domain facilitates high affinity binding to another protein or nucleic acid in a cell and thereby facilitates modulation of a phenotype of the cell and/or an animal. Preferred protein domains are not required to be constrained within a scaffold structure to bind to the target nucleic acid or target protein, or for said binding to be enhanced.

The term "protein domain" or "domain" or similar shall be taken to include an independently folding peptide structure (i.e., a "subdomain") unless the context requires otherwise. For example, a protein subdomain consisting of a 19-residue fragment from the C-loop of the fourth epidermal growth factor-like domain of thrombomodulin has been described by Alder et al, J. Biol. Chem., 270: 23366-23372, 1995. Accordingly, the skilled artisan is aware of the meaning of the term "protein subdomain".

By "native environment" of a peptide in meant the protein encoded by the gene from which the nucleic acid fragment was isolated. Accordingly, it is the aim of the present invention to identify those polypeptides that display a novel function, for example by binding to a target protein or nucleic acid to which it cannot bind in the context of the protein in which it naturally occurs. Suitable methods for determining the native environment of a peptide and/or the native function of a peptide will be apparent to the skilled person and/or described herein.

The screen of the invention is performed to identify a peptide that is capable of rescuing a cell, tissue or non-human organism from death induced by expression of a heterologous peptide, polypeptide, protein or allele. A peptide that rescues such a phenotype is capable of preventing cell death. Such a rescue screen is particularly amenable to a high-throughput screening platform. This is because only those cells that express a peptide with a desired biological activity are capable of surviving, thereby reducing analysis time.

The present disclosure also provides a rescue non-hybrid screen for the identification of a peptide that modulates a phenotype. The present disclosure provides a non-hybrid method for identifying a peptide capable of modulating a phenotype in a cell, tissue or organism, said method comprising:
(i) selecting or obtaining a cell, tissue or organism capable of expressing the phenotype, wherein the phenotype is death and/or reduced growth of the cell, tissue or organism;
(ii) expressing in the cell, tissue or organism (i) or introducing into the cell tissue or organsism (i) or contacting the cell, tissue or organsism (i) a peptide that mimics the structure of a domain or subdomain of a native protein;
(iii) selecting a cell, tissue or organism at (ii) that survives and/or is capable of growing; and
(iv) identifying the expressed or introduced peptide that induces survival and/or growth of the selected cell, tissue or organism (iii), wherein the peptide does not induce survival or growth of the cell, tissue or organism in its native environment.

In accordance with each of the embodiments described *supra* it is preferable that the phenotype is associated with or caused by an allele. Accordingly, preferred phenotypes are those that are caused by the presence of one or more alleles in a cell, tissue or organism. In this regard, the allele may be, for example, reduced or enhanced expression of a gene product, a polymorphism, expression of a mutant form of a gene product or expression of a heterologous gene product. Preferably, the allele is associated with a disease phenotype.

The various embodiments of the invention described *supra* are to be taken to apply *mutatis mutandis* to the instant screening method (e.g., as described in the previous two paragraphs).

In the invention, a peptide used in the screening assay of the present invention is encoded by nucleic acid that is derived from an organism with a compact genome. Accordingly, in one embodiment, the candidate peptide that mimics the structure of a domain of a native protein is produced by a method comprising:
(i) producing fragments from nucleic acids derived from two or more microorganisms and/or eukaryotes containing compact genomes, each of said microorganisms or eukaryotes having a substantially sequenced genome;
(ii) inserting the nucleic acid fragments at (i) into a suitable expression construct thereby producing recombinant constructs, wherein each fragment is in operable connection with a promoter sequence that is capable of conferring expression of that fragment; and
(iii) expressing the polypeptide encoded by the recombinant construct (ii).

Preferably, each of the contributing genomes or transcriptomes used in the production of the candidate peptide is used in an amount that is proportional to the complexity and size of the genome (or transcriptome), such as, for example, in comparison to the complexity and size of another genome in the mixture of genomes. This process results in approximately equal representation of the genome fragments.

The present invention additionally contemplates isolating, providing or producing the identified peptide and/or nucleic acid encoding same.

Furthermore, the present disclosure contemplates using an identified, isolated, produced or provided peptide or nucleic acid in the manufacture of a medicament for the treatment of a disease or disorder.

### Brief description of the drawings

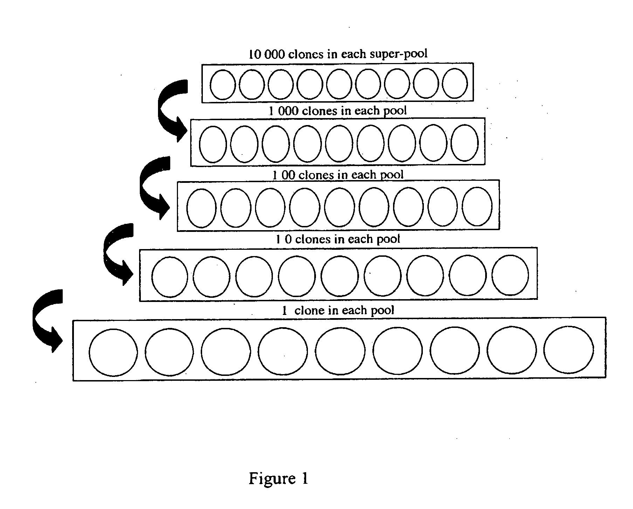

Figure 1 is a schematic representation of a number of pools of candidate peptides of the present invention. 10 individual peptides (or nucleic acid encoding same) are pooled. These pools are then pooled (to produce a pool of 100 clones). Ten of these pools are combined to produce a pool of 1000 clones. Ten of these pools are combined to produce a pool of 10000 clones. Clearly the pool sizes may differ. This method allows for the screening of large numbers of peptides at the same time, and by using the initial smaller pools, specific peptides that modulate a phenotype of interest are identified.

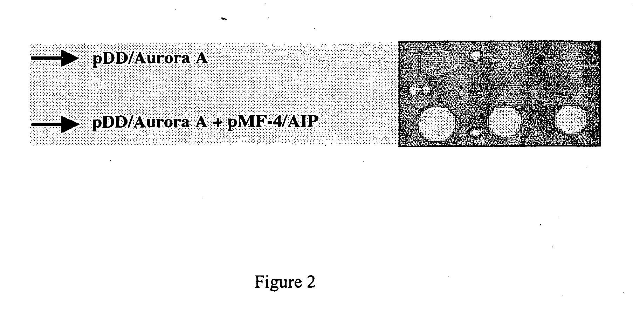

Figure 2 is a copy of a photographic representation showing rescue of Aurora A kinase induced lethality in yeast using the Aurora Interacting Protein (a repressor of Aurora A) in yeast grown on galactose media. Top row: Poor yeast growth associated with toxic expression of Aurora A. Bottom Row: Repression of Aurora A toxicity in yeast co-expressing the Aurora Interacting Protein

### Detailed description of the preferred embodiments

### Candidate peptides

In one embodiment, the candidate peptide that mimics the structure of a domain of a native protein is produced by a method comprising:
(i) producing fragments from nucleic acids derived from two or more prokaryotes containing compact genomes, each of said prokaryotes having a substantially sequenced genome;
(ii) inserting the nucleic acid fragments at (i) into a suitable expression construct thereby producing recombinant constructs, wherein each fragment is in operable connection with a promoter sequence that is capable of conferring expression of that fragment; and
(iii) expressing the polypeptide encoded by the recombinant construct (ii).

The term "fragment" as used herein, shall be understood to mean a nucleic acid that is the same as part of, but not all of a nucleic acid that forms a gene.

As used herein, the term "gene" means the segment of nucleic acid, specifically DNA, capable of encoding a peptide or polypeptide, in the present context, a "nucleic acid fragment" is include regions preceding and/or following the coding region of a naturally occurring gene, e.g. 5' untranslated or 3' untranslated sequences, as well as intervening sequences between individual coding sequences.

It will be apparent from the disclosure herein that the nucleic acid fragments used to produce the expression libraries in accordance with the present invention do not necessarily encode the same protein or peptide as in their native context (i.e. the gene from which they were derived). In fact, the nucleic acid fragments will generally encode a hitherto unknown peptide, particularly if derived from a non-coding region of a native gene. All that is required is an open reading frame of sufficient length to encode a peptide or protein domain.

Nucleic acid fragments are generated by one or more of a variety of methods known to those skilled in the art. Such methods include, for example, a method of producing nucleic acid fragments selected from the group consisting of mechanical shearing (e.g. by sonication or passing the nucleic acid through a fine gauge needle), digestion with a nuclease (e.g. Dnase 1), digestion with one or more restriction enzymes, preferably frequent cutting enzymes that recognize 4-base restriction enzyme sites and treating the DNA samples with radiation (e.g. gamma radiation or ultra-violet radiation).

In another embodiment, copies of nucleic acid fragments isolated from one or two or more organisms are generated by polymerase chain reaction (PCR) using, for example, random or degenerate oligonucleotides. Such random or degenerate oligonucleotides include restriction enzyme recognition sequences to allow for cloning of the amplified nucleic acid into an appropriate nucleic acid vector. Methods of generating oligonucleotides are known in the art and are described, for example, in Oligonucleotide Synthesis: A Practical Approach (M. J. Gait, ed., 1984) IRL Press, Oxford, whole of text, and particularly the papers therein by Gait, pp1-22; Atkinson *et al*., pp35-81; Sproat *et al*., pp 83-115; and Wu *et al*., pp 135-151. Methods of performing PCR are also described in detail by McPherson et al., In: PCR A Practical Approach., IRL Press, Oxford University Press, Oxford, United Kingdom, 1991.

In one embodiment, a candidate peptide comprises or consists of an amino acid sequence substantially identical to that of a protein domain, wherein the candidate peptide is not an antibody or fragment thereof that retains the activity of the antibody, nor is the peptide a random peptide (rather it is derived from a natural source).

As used herein "substantially identical" shall be taken to mean at least about 80% identical, more preferably 85% identical, even more preferably, 85% to 90% identical, and even more preferably, 95% to 99% identical.

As will be apparent to the skilled person from the foregoing, the present invention is useful for screening libraries of peptides. Such libraries are constructed, for example, from nucleic acid fragments comprising genomic DNA, cDNA, or amplified nucleic acid derived from one or two or more well-characterized genomes. The well-characterized genomes used in the production of an expression library can be a compact genome of a eukaryote (e.g., a protist, a dinoflagellate, an alga, a plant, a fungus, a mould, a invertebrate, a vertebrate, amongst others) such as, for example, a eukaryote selected from the group consisting of *Arabidopsis thaliana, Anopheles gambiae*, *Caenorhabditis elegans, Danio rerio, Drosophila melanogaster, Takifugu rubripes, Cryptosporidium parvum, Giardia duodenalis, Trypanosoma cruzii, Saccharomyces cerevesiae*, and *Schizosaccharomyces pombe.* In embodiments of the invention, one or more well-characterized genomes is a compact genome of a prokaryote (i.e. bacteria, eubacteria, cyanobacteria, etc) such as, for example a prokaryote selected from the group consisting of *Archaeoglobus fulgidis, Aquifex aeolicus, Aeropyrum pernix*, *Bacillus subtilis, Bordetella pertussis TOX6, Borrelia burgdorferi, Chlamydia trachomatis, Desulfobacterium autotrophicum, Escherichia coli K12, Haemophilus influenzae (rd), Halobacterium salinarium, Haloferax volcanii, Helicobacter pylori, Methanobacterium thermoautotrophicum, Methanococcus jannaschii, Mycoplasma pneumonia, Neisseria meningitidis, Pirellula Species 1 (rhodopirellula baltica), Pseudomonas aeruginosa, Pyrococcus horikoshii, Synechocystis PCC 6803, Thermoplasma volcanium*, *Thermotoga maritima, Thermus thermophilus* and *Desulfovibrio vulgaris.*

As used herein, the term "well characterized genome" shall be taken to mean that a genome has been substantially sequenced. As used herein a "substantially sequenced genome" shall be taken to mean that at least about 60% of the genome has been sequenced. More preferably at least about 70% of the genome has been sequenced, and more preferably at least about 75% of the genome has been sequenced. Even more preferably at least about 80% of the genome has been sequenced.

Methods for determining the amount of a genome that has been sequenced are known in the art. Furthermore, information regarding those sequences that have been sequenced is readily obtained from publicly available sources, such as, for example, the databases of NCBI or TIGR, thereby facilitating determination of the diversity of the genome.

Organisms having a substantially sequenced genome include, for example, an organism selected from the group consisting of *Actinobacillus pleuropneumoniae serovar*, *Aeropyrum pernix, Agrobacterium tumeficians, Anopheles gambiae, Aquifex aeolicus, Arabidopsis thaliana, Archeglobus fulgidis, Bacillus anthracis, bacillus cereus, Baccilus halodurans, Bacillus subtilis, Bacteroides thetaiotaomicron, Bdellovibrio bacteriovorus, Bifidobacterium longuin, Bordetella bronchiseptica, Bordetella para pertussis*, *Borrelia burgdorferi*, *Bradyrhizobium japonicum, Brucella melitensis, Brucella suis, Bruchnera aphidicola*, *Brugia malayi, Caenorhabditis elegans, Campylobacter jejuni, Candidatus blochmannia floridanus, Caulobacter crescentus, Chlamydia muridarum, Chlamydia trachomatis, Chlamydophilia caviae, Chlamydia pneumoniae, Chlorobium tepidum, Chromobacterium violaceum, Clostridium acetobutylicum, Clostridium perfringens, Clostridium tetani, Corynebacterium diphtheriae, Corynebacterium efficiens, Corynebacterium glutamicum, Coxiella burnetii, Danio rerio, Dechloromonas aromatica, Deinococcus radiodurans, Drosophila melanogaster, Eimeria tenella, Eimeria acervulina, Entamoeba histolytica, Enterococcus faecalis, Escherichia coli, Fusobacterium nucleatum, Geobacter sulfurreducens, Gloeobacter violaceus, Haemophilis ducreyi, Haemophilus influenzae, Halobacterium, Helicobacter hepaticus, Helicobacter pylori, Lactobacillus johnsonii, Lactobacillus plantarum, Lactococcus lactis, Leptospira interrogans serovar lai, Listeria innocua, Listeria monocytogenes, Mesorhizobium loti, Methanobacterium thermoautotrophicum, Methanocaldocossus jannaschii, Methanococcoides burtonii, Methanopyrus kandleri, Methanosarcina acetivorans, Methanosarcina mazei Goel, Methanothermobacter thermautotrophicus, Mycobacterium avium, Mycobacterium bovis, Mycobacterium leprae, Mycobacterium tuberculosis, Mycoplasma gallisepticum strain R, Mycoplasma genitalium, Mycoplasma penetrans, Mycoplasma pneumoniae, Mycoplasma pulmonis, Nanoarchaeum equitans, Neisseria meningitidis, Nitrosomonas europaea, Nostoc, Oceanobacillus iheyensis, Onion yellows phytoplasma, Oryzias latipes, Oryza sativa, Pasteurella multocida, Photorhabdus luminescens, Pirellula, Plasmodium falciparum, Plasmodium vivax, Plasmodium yoelii, Porphyromonas gingivalis, Prochlorococcus marinus, Prochlorococcus marinus, Prochlorococcus, Pseudomonas aeruginosa, Pseudomonas putida, Pseudomonas syringae, Pyrobaculum aerophilum, Pyrococcus abyssi, Pyrococcus furiosus, Pyrococcus horikoshii, Ralstonia solanacearum, Rhodopseudomonas palustris, Rickettsia conorii, Rickettsia prowazekii, Rickettsia rickettsii*, *Saccharomyces cerevisiae, Salmonella enterica, Salmonella typhimurium, Sarcocystis cruzi, Schistosoma mansoni, Schizosaccharomyces pombe, Shewanella oneidensis, Shigella flexneri, Sinorhizobium meliloti, Staphylococcus aureus, Staphylococcus epidermidis, Streptococcus agalactiae, Streptococcus agalactiae, Streptococcus mutans*, *Streptococcus pneumoniae, Streptococcus pyogenes, Streptomyces avermitilis, Streptomyces coelicolor, Sulfolobus solfataricus, Sulfolobus tokodaii, Synechocystis sp., Takifugu rubripes, Tetraodon fluviatilis, Theileria parva*, *Thermoanaerobacter tengcongensis, Thermoplasma acidophilum*, *Thermoplasma volcanium, Thermosynechococcus elongatus, Thermotoga maritima, Toxoplasma gondii*, *Treponema denticola, Treponema pallidum*, *Tropheryma whipplei*, *Tryponosoma brucei*, *Trypanosoma cruzi*, *Ureaplasma urealyticum*, *Vibrio cholerae*, *Vibro parahaemolyticus, Vibro vulnificus*, *Wigglesworthia brevipalpis*, *Wolbachia endosymbiont of Drosophilia melanogaster*, *Wolinella succinogenes*, *Xanthomonas axonopodis pv. Citri*, *Xanthomonas campestris pv. Campestris*, *Xylella fastidiosa,* and *Yersinia pestis.*

In the present disclosure, nucleic acid fragments are derived from a virus having a substantially sequenced genomes. Virus' with a substantially sequenced genomes are known in the art and include, for example, a virus selected from the group consisting of T7 phage, HIV, equine arteritis virus, lactate dehydrogenase-elevating virus, lelystad virus, porcine reproductive and respiratory syndrome virus, simian hemorrhagic fever virus, avian nephritis virus 1, turkey astrovirus 1, human asterovirus type 1, 2 or 8, mink astrovirus 1, ovine astrovirus 1, avian infectious bronchitis virus, bovine coronavirus, human coronavirus, murine hepatitis virus, porcine epidemic diarrhea virus, SARS coronavirus, transmissible gastroenteritis virus, acute bee paralysis virus, aphid lethal paralysis virus, black queen cell virus, cricket paralysis virus, Drosophila C virus, himetobi P virus, kashmir been virus, plautia stali intestine virus, rhopalosiphum padi virus, taura syndrome virus, triatoma virus, alkhurma virus, apoi virus, cell fusing agent virus, deer tick virus, dengue virus type 1, 2, 3 or 4, Japanese encephalitis virus, Kamiti River virus, kunjin virus, langat virus, louping ill virus, modoc virus, Montana myotis leukoencephalitis virus, Murray Valley encephalitis virus, omsk hemorrhagic fever virus, powassan virus, Rio Bravo virus, Tamana bat virus, tick-borne encephalitis virus, West Nile virus, yellow fever virus , yokose virus, Hepatitis C virus, border disease virus, bovine viral diarrhea virus 1 or 2, classical swine fever virus, pestivirus giraffe, pestivirus reindeer, GB virus C, hepatitis G virus, hepatitis GB virus, bacteriophage M11, bacteriophage Qbeta, bacteriophage SP, enterobacteria phage MX1, enterobacteria NL95, bacteriophage AP205, enterobacteria phage fr, enterobacteria phage GA, enterobacteria phage KU1, enterobacteria phage M12, enterobacteria phage MS2, pseudomonas phage PP7, pea enation mosaic virus-1, barley yellow dwarf virus, barley yellow dwarf virus-GAV, barley yellow dwarf virus-MAW, barley yellow dwarf virus-PAS, barley yellow dwarf virus-PAV, bean leafroll virus, soybean dwarf virus, beet chlorosis virus, beet mild yellowing virus, beet western yellows virus, cereal yellow dwarf virus-RPS, cereal yellow dwarf virus-RPV, cucurbit aphid-borne yellows virus, potato leafroll virus, turnip yellows virus, sugarcane yellow leaf virus, equine rhinitis A virus, foot-and-mouth disease virus, encephalomyocarditis virus, theilovirus, bovine enterovirus, human enterovirus A, B, C, D or E, poliovirus, porcine enterovirus A or B, unclassified enterovirus, equine rhinitis B virus, hepatitis A virus, aichi virus, human parechovirus 1, 2 or 3, ljungan virus, equine rhinovirus 3, human rhinovirus A and B, porcine teschovirus 1, 2-7, 8, 9, 10 or 11, avian encephalomyelitis virus, kakugo virus, simian picornavirus 1, aura virus, barmah forest virus, chikungunya virus, eastern equine encephalitis virus, igbo ora virus, mayaro virus, ockelbo virus, onyong-nyong virus, Ross river virus, sagiyama virus, salmon pancrease disease virus, semliki forest virus, sindbis virus, sindbus-like virus, sleeping disease virus, Venezuelan equine encephalitis virus, Western equine encephalomyelitis virus, rubella virus, grapevine fleck virus, maize rayado fino virus, oat blue dwarf virus, chayote mosaic tymovirus, eggplant mosaic virus, erysimum latent virus, kennedya yellow mosaic virus, ononis yellow mosaic virus, physalis mottle virus, turnip yellow mosaic virus and poinsettia mosaic virus.

Information regarding those viral sequences that have been sequenced is readily obtained from publicly available sources, such as, for example, the databases of VirGen and/or NCBI, thereby facilitating determination of the diversity of the genome.

As used herein, the term "VirGen" shall be taken to mean the vial genome resource of the Bioinformatics Centre, University of Pune, Pune 411 007, India.

In one preferred embodiment of the invention, the nucleic acid fragments are derived from an organism a compact genome so as to facilitate identification of one or more modulatory peptides in a complex genome.

More preferably, the nucleic acid fragments are derived from an organism that is from a different kingdom to the cell, tissue or organism in which the peptide is screened. Alternatively, or in addition, the nucleic acid fragments are derived from an organism that is from a different kingdom to the organism in which the phenotype occurs in nature.

In a preferred embodiment, the nucleic acid fragments are derived from one or more bacterium. For example, the nucleic acid fragments are derived from one or more bacterium having a compact genome. In accordance with this embodiment, when screening for a peptide derived from a bacterium, it is preferable that the screen is performed in a non-bacterial cell (e.g., a eukaryotic cell, e.g., a yeast cell or a mammalian cell).

In a preferred embodiment, the nucleic acid fragments are derived from one or more prokaryotes selected from the group consisting of *Aeropyrum pernix*, *Aquifex aeolicus*, *Archaeoglobus fulgidis, Bacillus subtilis, Bordetella pertussis, Borrelia burgdorferi, Chlamydia trachomatis, Escherichia coli, Haemophilus influenzae, Helicobacter pylori, Methanobacterium thermoautotrophicum, Methanococcus jannaschii*, *Mycoplasma pneumoniae, Neisseria meningitidis, Pseudomonas aeruginosa, Pyrococcus horikoshii*, *Synechocystis* PCC 6803, *Thermoplasma volcanium*, *Thermotoga maritima*, *Thermus thermophilus* and *Desulfovibrio vulgaris*.

In another preferred embodiment, the nucleic acid fragments are derived from the prokaryotes *Aeropyrum pernix, Aquifex aeolicus, Archaeoglobus fulgidis, Bacillus subtilis, Bordetella pertussis, Borrelia burgdorferi, Chlamydia trachomatis, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis* PCC 6803, *Thermoplasma volcanium*, *Thermotoga maritima, Thermus thermophilus* and *Desulfovibrio vulgaris*.

In a preferred embodiment, the nucleic acid fragments are derived from one or more prokaryotes selected from the group consisting of *Aeropyrum pernix, Aquifex aeolicus, Archaeoglobus fulgidis, Bacillus subtilis, Bordetella pertussis, Borrelia burgdorferi, Chlamydia trachomatis, Desulfobacterium autotrophicum, Escherichia coli, Haemophilus influenzae, Halobacterium salinarium, Haloferax volcanii Helicobacter pylori, Methanobacterium thermoautotrophicum, Methanococcus jannaschii, Mycoplasma pneumoniae, Neisseria meningitidis, Pirellula Species 1 (rhodopirellula baltica), Pseudomonas aeruginosa, Pyrococcus horikoshii, Synechocystis PCC 6803, Thermoplasma volcanium and Thermotoga maritima.Escherichia coli, Haemophilus influenzae, Helicobacter pylori, Methanobacterium thermoautotrophicum*, *Methanococcus jannaschii, Mycoplasma pneumoniae, Neisseria meningitidis, Pseudomonas aeruginosa, Pyrococcus horikoshii, Synechocystis PCC 6803, Thermoplasma volcanium* and *Thermotoga maritima*.

In another preferred embodiment, the nucleic acid fragments are derived from the prokaryotes *Aeropyrum pernix, Aquifex aeolicus, Archaeoglobus fulgidis, Bacillus subtilis, Bordetella pertussis, Borrelia burgdorferi, Chlamydia trachomatis, Desulfobacterium autotrophicum, Escherichia coli, Haemophilus influenzae*, *Halobacterium salinarium, Haloferax volcanii Helicobacter pylori, Methanobacterium thermoautotrophicum, Methanococcus jannaschii, Mycoplasma pneumoniae, Neisseria meningitidis, Pirellula Species 1 (rhodopirellula baltica), Pseudomonas aeruginosa, Pyrococcus horikoshii, Synechocystis PCC 6803, Thermoplasma volcanium and Thermotoga maritime, Escherichia coli and Haemophilus.*

In a further preferred embodiment, the nucleic acid fragments are derived from one or more prokaryotes selected from the group consisting of *Archaeoglobus fulgidus*, *Aquifex aeolicus, Aeropyrum pernix, Bacillus subtilis, Bordetella pertussis, Borrelia burgdorferi, Chlamydia trachomatis, Escherichia coli K12, Haemophilus influenzae, Helicobacter pylori, Methanobacterium thermoautotrophicum., Methanococcus jannashii, Neisseria meningitidis, Pyrococcus horikoshii, Pseudomonas aeruginosa, Synechocystis PCC 6803, Thermoplasma volcanicum, Thermotoga maritima, Acidobacterium capsulatum, Halobacterium salinarum, Desulfobacterium autotrophicum, Haloferax volcanii, Rhodopirellula baltica, Thermus thermophilus HB27 and Prochlorococcus marinus MED4.*

In a further preferred embodiment, the nucleic acid fragments are derived from the prokaryotes *Archaeoglobus fulgidus, Aquifex aeolicus, Aeropyrum pernix, Bacillus subtilis, Bordetella pertussis, Borrelia burgdorferi, Chlamydia trachomatis, Escherichia coli K12, Haemophilus influenzae, Helicobacter pylori, Methanobacterium thermoautotrophicum., Methanococcus jannashii, Neisseria meningitidis, Pyrococcus horikoshii, Pseudomonas aeruginosa, Synechocystis PCC 6803, Thermoplasma volcanicum, Thermotoga maritima, Acidobacterium capsulatum, Halobacterium salinarum, Desulfobacterium autotrophicum, Haloferax volcanii, Rhodopirellula baltica, Thermus thermophilus HB27 and Prochlorococcus marinus MED4.*

Methods of isolating genomic DNA from eukaryotic organisms are known in the art and are described in, for example, Ausubel et al (In: Current Protocols in Molecular Biology. Wiley Interscience, ISBN 047 150338, 1987) or (Sambrook et al (In: Molecular Cloning: A Laboratory Manual, Cold Spring Harbor Laboratories, New York, Third Edition 2001).

In a further embodiment of the present invention, the nucleic acid fragments are derived from complementary DNA (cDNA). Those skilled in the art will be aware that cDNA is generated by reverse transcription of RNA using, for example, avian reverse transcriptase (AMV) reverse transcriptase or Moloney Murine Leukemia Virus (MMLV) reverse transcriptase. Such reverse transcriptase enzymes and the methods for their use are known in the art, and are obtainable in commercially available kits, such as, for example, the Powerscript kit (Clontech), the Superscript II kit (Invitrogen), the Thermoscript kit (Invitrogen), the Titanium kit (Clontech), or Omniscript (Qiagen).

Methods of isolating mRNA from a variety of organisms are known in the art and are described for example in, Ausubel et al (In: Current Protocols in Molecular Biology. Wiley Interscience, ISBN 047 150338, 1987) or Sambrook et al (In: Molecular Cloning: A Laboratory Manual, Cold Spring Harbor Laboratories, New York, Third Edition 2001).

Methods of generating cDNA from isolated RNA are also commonly known in the art and are described in for example, Ausubel et al (In: Current Protocols in Molecular Biology. Wiley Interscience, ISBN 047 150338, 1987) or (Sambrook et al (In: Molecular Cloning: A Laboratory Manual, Cold Spring Harbor Laboratories, New York, Third Edition 2001).

In a preferred embodiment, the nucleic acid fragments generated from RNA or cDNA are normalized to reduce any bias toward more highly expressed genes. Methods of normalizing nucleic acids are known in the art, and are described for example in, Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001) and Soares et al Curr. Opinion Biotechnol 8, 542-546, 1997, and references cited therein. One of the methods described by Soares uses reasssociation-based kinetics to reduce the bias of the library toward highly expressed sequences. Alternatively, cDNA is normalized through hybridization to genomic DNA that has been bound to magnetic beads, as described in Kopczynski et al, Proc. Natl. Acad. Sci. USA, 95(17), 9973-9978, 1998. This provides an approximately equal representation of cDNA sequences in the eluant from the magnetic beads. Normalized expression libraries produced using cDNA from one or two or more prokaryotes or compact eukaryotes are clearly contemplated by the present invention.

In a preferred embodiment, nucleic acid fragments are selected that have sufficiently different or divergent nucleotide sequences to thereby enhance nucleotide sequence diversity among the selected fragments compared to the diversity of sequences in the genome from which they were derived.

In one embodiment a nucleic acid fragment is selected such that the encoded polypeptide varies by one or more amino acids with regard to the amino acid sequence of the polypeptide encoded by another fragment in the library, a process that is facilitated using genomes that are substantially sequenced.

In an alternative embodiment, the nucleotide sequence of a nucleic acid fragment is mutated by a process such that the encoded peptide varies by one or more amino acids compared to the "template" nucleic acid fragment. The "template" may have the same nucleotide sequence as the original nucleic acid fragment in its native context (i.e., in the gene from which it was derived). Alternatively, the template may itself be an intermediate variant that differs from the original nucleic acid fragment as a consequence of mutagenesis. Mutations include at least one nucleotide difference compared to the sequence of the original fragment. This nucleic acid change may result in for example, a different amino acid in the encoded peptide, or the introduction or deletion of a stop codon. Accordingly, the diversity of the nucleic acids of the expression library and the encoded polypeptides is enhanced by such mutation processes.

In one embodiment, the nucleic acid fragments are modified by a process of mutagenesis selected from the group consisting of, mutagenic PCR, expressing the nucleic acid fragment in a bacterial cell that induces a random mutation, site directed mutagenesis and expressing a nucleic acid fragment in a host cell exposed to a mutagenic agent such as for example radiation, bromo-deoxy-uridine (BrdU), ethylnitrosurea (ENU), ethylmethanesulfonate (EMS) hydroxylamine, or trimethyl phosphate amongst others.

In a preferred embodiment, the nucleic acid fragments are modified by amplifying a nucleic acid fragment using mutagenic PCR. Such methods include, for example, a process selected from the group consisting of: (i) performing the PCR reaction in the presence of manganese; and (ii) performing the PCR in the presence of a concentration of dNTPs sufficient to result in misincorporation of nucleotides.

Methods of inducing random mutations using PCR are known in the art and are described, for example, in Dieffenbach (ed) and Dveksler (ed) (In: PCR Primer: A Laboratory Manual, Cold Spring Harbour Laboratories, NY, 1995). Furthermore, commercially available kits for use in mutagenic PCR are obtainable, such as, for example, the Diversify PCR Random Mutagenesis Kit (Clontech) or the GeneMorph Random Mutagenesis Kit (Stratagene).

In one embodiment, PCR reactions are performed in the presence of at least about 200µM manganese or a salt thereof, more preferably at least about 300µM manganese or a salt thereof, or even more preferably at least about 500µM or at least about 600µM manganese or a salt thereof. Such concentrations manganese ion or a manganese salt induce from about 2 mutations per 1000 base pairs (bp) to about 10 mutations every 1000 bp of amplified nucleic acid (Leung et al Technique 1, 11-15, 1989).

In another embodiment, PCR reactions are performed in the presence of an elevated or increased or high concentration of dGTP. It is preferred that the concentration of dGTP is at least about 25µM, or more preferably between about 50µM and about 100µM. Even more preferably the concentration of dGTP is between about 100µM and about 150µM, and still more preferably between about 150µM and about 200µM. Such high concentrations of dGTP result in the misincorporation of nucleotides into PCR products at a rate of between about 1 nucleotide and about 3 nucleotides every 1000 bp of amplified nucleic acid (Shafkhani et al BioTechniques 23, 304-306, 1997).

PCR-based mutagenesis is preferred for the mutation of the nucleic acid fragments of the present invention, as increased mutation rates is achieved by performing additional rounds of PCR.

In another preferred embodiment, the nucleic acid of the expression library is mutated by inserting said nucleic acid into a host cell that is capable of mutating nucleic acid. Such host cells are deficient in one or more enzymes, such as, for example, one or more recombination or DNA repair enzymes, thereby enhancing the rate of mutation to a rate that is rate approximately 5,000 to 10,000 times higher than for non-mutant cells.

Strains particularly useful for the mutation of nucleic acids carry alleles that modify or inactivate components of the mismatch repair pathway. Examples of such alleles include alleles selected from the group consisting of *mutY*, *mutM*, *mutD*, *mutT*, *mutA*, *mutC* and *mutS*. Bacterial cells that carry alleles that modify or inactivate components of the mismatch repair pathway are known in the art, such as, for example the XL-1Red, XL-*mutS* and XL-*mutS*-Kan^{r} bacterial cells (Stratagene).

In a further preferred embodiment the mutated nucleic acid fragments are combined with the non-mutated fragments from which they were derived, for subcloning into an expression vector. In this way, the nucleotide diversity of the expression library is enhanced, as is the diversity of the conformations of the expressed peptides and proteins.

In a further embodiment, a significant proportion of the nucleic acid fragments are cloned into a gene construct in at least two forward open reading frames, and preferably three forward open reading frames, to thereby enhance the number of divergent peptides or proteins that are encoded by a particular nucleic acid fragment. In this context, the term "significant proportion" means at least about 30% to 50%, preferably at least about 40% to 60%, more preferably at least about 50% to 70%, still more preferably at least about 60% to 80% and still more preferably greater than about 70% or 80% of the total nucleic acid fragments that are subcloned successfully into a suitable gene construct such that more than one open reading frame can be utilized for expression. As will be known to those skilled in the art, procedures for cloning a single nucleic acid into a gene construct in multiple reading frames are known.

Preferred methods of subcloning a nucleic acid fragment in multiple three reading frames comprise a process selected from the group consisting of:
(a) ligating the nucleic acid fragment to a linker or adaptor, such as for example, one or more linkers modified to contain an additional one or two or three base pairs, or a multiple of one or two or three nucleotides;
(b) Placing each nucleic acid fragment operably under the control of a Kozak consensus sequence and at different distances therefrom (e.g. one or two or three nucleotides or a multiple of one or two or three nucleotides) from said Kozak consensus sequence;
(c) Placing a fragment under control of sequences that confer transcriptional and/or translational slippage.

By ligating the nucleic acid fragment to a linker or adaptor, the number of introduced nucleotides can be varied such that a significant proportion of the nucleic acid fragments are introduced into an expression vector or gene construct in at least two and preferably three reading frames. Linkers or adaptors are ligated to the 5'-end of the nucleic acid fragment such that, on average, a different length linker or adaptor is added to each nucleic acid fragment having the same sequence. This is generally achieved by varying the relative proportions of each linker/adaptor to the nucleic acid fragments. Naturally, each linker/adaptor of differing length is generally in equimolar concentration in the ligation reaction, and the total concentration of linker/adaptor 3'-ends is held in equimolar concentration to the total concentration of 5'-ends of the nucleic acid fragments being ligated. Methods of ligating adaptors to nucleic acids are known in the art and are described in for example, Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001).

As an alternative to separately adding the linkers/adaptors to the nucleic acid fragments prior to subcloning into a suitable gene construct, a suitable gene construct is used that comprises additional nucleotides 3' of a translation initiation signal, and provides for sub-cloning of nucleic acid fragments in each reading frame. As will be known to those skilled in the art, each reading frame in a gene construct is generally accessed by digesting the gene construct with a different restriction endonuclease and then sub-cloning nucleic acid fragments into the digested, linearized vector. By "sub-cloning" means a process involving or comprising a ligation reaction.

Alternatively, site directed mutagenesis is used to introduce additional nucleotides after the translation initiation site of the gene construct. Methods of site-directed mutagenesis are known in the art, and are described for example, in Dieffenbach (eds) and Dveksler (ed) (In: PCR Primer: A Laboratory Manual, Cold Spring Harbour Laboratories, NY, 1995). Furthermore, kits containing instruction and reagents necessary for site-directed mutagenesis are commercially available, such as, for example, the Quikchange site directed mutagenesis kit (Stratagene).

Furthermore, expression vectors are commercially available that have been modified to include an additional one or two nucleotides after the transcription start codon to allow for cloning of a nucleic acid in at least two and preferably three reading frames. Such vectors include, for example, the pcDNA (A, B, or C) vector suite (Invitrogen).

By positioning each nucleic acid fragment so that expression is placed operably under the control of a Kozak consensus sequence and at different distances therefrom, a significant proportion of the nucleic acid fragments is inserted into the vector in at least two and preferably three reading frames. A preferred Kozak sequence has the core sequence RNNATG (SEQ ID NO: 1), wherein R is a purine (i.e. A or G) and N is any nucleotide. A particularly preferred Kozak sequence for expression of a polypeptide in eukaryotic cells comprises the sequence CCRCCATG (SEQ ID NO: 2) or GCCAGCCATGG (SEQ ID NO: 3). A preferred Kozak sequence for the expression of polypeptides in plants is CTACCATG (SEQ ID NO: 4).

A Kozak consensus sequence is generated using synthetic oligonucleotides in a process that is known in the art and described, for example, in, Oligonucleotide Synthesis: A Practical Approach (M. J. Gait, ed., 1984) IRL Press, Oxford, whole of text, and particularly the papers therein by Gait, pp1-22; Atkinson *et al*., pp35-81; Sproat *et al*., pp 83-115; and Wu *et al*., pp 135-151. Alternatively a Kozac sequence is isolated from a natural or recombinant source using methods known in the art, such as for example using from the group, restriction enzyme digestion or PCR.

In one embodiment, the Kozak sequence is generated as an oligonucleotide or nucleic acid fragment and then ligated 5' of the nucleic acid fragment (i.e., the nucleic acid fragment being sub-cloned). Methods of ligating such oligonucleotides or fragments are known in the art and are described in for example, Ausubel et al (In: Current Protocols in Molecular Biology. Wiley Interscience, ISBN 047 150338, 1987) or (Sambrook et al (In: Molecular Cloning: Molecular Cloning: A Laboratory Manual, Cold Spring Harbor Laboratories, New York, Third Edition 2001). As with other ligations, the total concentration of nucleic acid of each ligating species (i.e., the Kozak containing fragment and the nucleic acid) should preferably be equimolar. Naturally, to ensure that a significant proportion of nucleic acid fragments are ligated in each reading frame, the Kozak-containing fragments of differing length should also be present in approximately equimolar concentration.

As an alternative to separately adding the Kozak consensus sequence oligonucleotide or fragment to the nucleic acid fragment prior to subcloning into a suitable vector, an expression vector is used that comprises a translation start site and provides for subcloning of nucleic acid fragments in each reading frame. As will be known to those skilled in the art, each reading frame in such a vector is generally accessed by digesting the vector with a different restriction enzyme and then subcloning fragments into the digested, linearized vector.

When the nucleic acid fragment of the present invention is to be expressed in prokaryotic cells, it is particularly preferred that the Kozak sequence of the above embodiments is replaced with a ribosome binding sequence, or Shine Dalgarno sequence. A particularly preferred Shine Dalgarno sequence consists of nucleic acids having the nucleotide sequence GAAGAAGATA (SEQ ID NO: 5).

By placing a fragment under control of sequences that confer transcriptional and/or translational slippage is meant that the fidelity of the start site for transcription and/or translation is reduced such that translation is initiated at different sites. Accordingly, such a sequence is cause the expression of several different polypeptides.

In one embodiment translational slippage (or translational frameshifting) is induced using nucleic acid comprising of the consensus sequence N₁N₁N₁N₂N₂N₂N₃, wherein N represents any nucleotide and all nucleotides represented by N₁ are the same nucleotide, all nucleotides represented by N₂ are the same nucleotide. In accordance with this embodiment, N₁ and/or N₂ and/or N₃ are the same or different. A particularly preferred translational slippage sequence for use in a eukaryote will comprise a sequence selected from the group consisting of: AAAAAAC (SEQ ID NO: 6), AAATTTA (SEQ ID NO: 7), AAATTTT (SEQ ID NO: 8), GGGAAAC (SEQ ID NO: 9), GGGCCCC (SEQ ID NO: 10), GGGTTTA (SEQ ID NO: 11), GGGTTTT (SEQ ID NO: 12), TTTAAAC (SEQ ID NO: 13), TTTAAAT (SEQ ID NO: 14), TTTTTA (SEQ ID NO: 15), and GGATTTA (SEQ ID NO: 16). In an alternative embodiment, a sequence that induces translational slippage in yeast is CTTAGGC (SEQ ID NO: 17) or GCGAGTT (SEQ ID NO: 18). In yet another embodiment a sequence that induces translational slippage in mammals is TCCTGAT (SEQ ID NO: 19).

In another embodiment, a translational slippage sequences for use in prokaryotic organisms includes, but is not limited to s sequence selected from the group consisting of AAAAAAG (SEQ ID NO: 20), AAAAAAA (SEQ ID NO: 21), AAAAAAC (SEQ ID NO: 22), GGGAAAG (SEQ ID NO: 23), AAAAGGG (SEQ ID NO: 24), GGGAAAA (SEQ ID NO: 25), TTTAAAG (SEQ ID NO: 26) and AAAGGGG (SEQ ID NO: 27). It is particularly preferred that this translational slippage sequence is positioned about 7 to about 19 nucleotides downstream of a Shine Dalgarno sequence. In an alternative embodiment, a nucleic acid that induces translational slippage in bacterial cells comprises the nucleotide sequence CTT (SEQ ID NO: 28), and is positioned 3 nucleotides upstream of a Shine Dalgarno sequence controlling the expression of the nucleic acid fragment.

A translational slippage sequence is generated using synthetic oligonucleotides, or isolated from a natural or recombinant source, for example the *prfB* gene, the *dnaX* gene, the mammalian ornithine decarboxylase antizyme, in addition to various retroviruses, coronaviruses, retrotransposons, virus-like sequences in yeast, bacterial genes and bacteriophage genes. Such a sequence is isolated using a method that is known in the art, such as for example, restriction enzyme digestion or PCR.

It is preferred that sequences that confer translational slippage are ligated to the 5'-end of the nucleic acid fragment in the same manner as for adaptor addition. Methods of ligating adaptors are known in the art and are described in for example, Ausubel et al (In: Current Protocols in Molecular Biology. Wiley Interscience, ISBN 047 150338, 1987) or (Sambrook et al (In: Molecular Cloning: Molecular Cloning: A Laboratory Manual, Cold Spring Harbor Laboratories, New York, Third Edition 2001).

It is also preferred that the sequences that confer transcriptional or translational slippage are incorporated into the expression vector or gene construct into which the nucleic acid fragment is inserted, such that it is positioned upstream (i.e., 5') of the translational start site in the fragment.

In another embodiment, transcriptional slippage is induced by the introduction of a stretch of nucleotides with a sequence such as, for example, T₉ or A₉. Transcriptional slippage sequences are preferably cloned downstream (i.e., 3') of the site of initiation of transcription. It is also preferred to position a transcriptional slippage sequence upstream (5') of a translational start site in the nucleic acid fragment. Accordingly, the transcriptional slippage sequence is included in the expression vector or gene construct into which the nucleic acid fragment is inserted.

Accordingly, the nucleic acids that form the transcriptional slippage sequence is ligated to the 5' end of a nucleic acid fragment, in conjunction with a translation start site.

It will be apparent from the preceding description that the transcriptional slippage sequence is incorporated into the expression vector or gene construct upstream of the translation start site, and downstream of the site of initiation of transcription.

Preferably, the nucleic acid fragments derived from the prokaryote or compact eukaryote genome are inserted into a gene construct in both the forward and/or reverse orientation, such that 1 or 2 or 3 or 4 or 5 or 6 open reading frames of said nucleic acid fragments are utilized. Methods of bi-directionally inserting fragments into vectors are known in the art.

It will be apparent to the skilled artisan that, by sub-cloning the nucleic acid fragments in multiple reading frames into a suitable expression vector, it is possible to encode a peptide or protein domain that does not occur in nature, as well as producing a variety of natural peptide domains. Accordingly, the diversity of the nucleic acids of the expression library and their encoded peptides are greatly enhanced in these modified nucleic acid fragment expression libraries.

In a preferred embodiment, the expression libraries are normalized to remove any redundant nucleic acid from the genome. As cited herein the term "redundant nucleic acid" shall be taken to mean those nucleic acid fragments having the same sequence, such as, for example, high copy number or repetitive sequences. Nucleic acid fragments derived from multiple homologous sequences, whether derived from the same or a different species can be subject to normalization to reduce the presence of redundant sequences in the expression library. Similarly, nucleic acid fragments derived from repetitive DNA and nucleic acid fragments derived from pseudogenes can be subject conveniently to normalization. Methods of normalizing libraries to remove redundant nucleic acid are known in the art and are described, for example, by Ausubel et al., In: Current Protocols in Molecular Biology. Wiley Interscience, ISBN 047 150338, 1987, or Sambrook et al., In: Molecular Cloning: Molecular Cloning: A Laboratory Manual, Cold Spring Harbor Laboratories, New York, Third Edition 2001, or Bonaldo et al., Genome Res. 6(9), 791-806, 1997. In one embodiment, the nucleic acid fragments are subjected to hydroxyapatite chromatography to remove redundant or highly repetitive sequences. The success of such a normalization process can be determined, for example, by hybridizing labeled non-normalized and normalized DNA to Southern blots of genomic DNA and comparing the amount of label bound to each blot. The amount of bound label is comparable to the amount of hybridized DNA. A reduced hybridization signal for normalized libraries indicates that iterative sequences have been reduced in the normalized pool.

In one embodiment the nucleic acids used to produce the expression libraries are isolated from a single organism. In this case, nucleic acid fragments are generated from nucleic acid derived from a distinct prokaryote.

In another embodiment of the present invention the nucleic acids are derived from two or more prokaryotes including any and all combinations thereof.

It is preferred that the prokaryote(s) used to produce expression libraries from combined genomes are evolutionally diverse organisms. As used herein the term "evolutionary diverse" shall be taken to mean those organisms that when compared at the genetic level, show a significant degree of genetic diversity. As used herein the term "significant degree of genetic diversity" shall be taken to mean, that the genes of the prokaryotes differ by at least about 10% to 30% at the nucleic acid level. More preferably the genetic sequences of the prokaryotes differ by at least about 30% to 40% at the nucleic acid level. More preferably the genetic sequences of the prokaryotes differ by at least about 50% at the nucleic acid level. More preferably the genetic sequences of the prokaryote differ by at least about 70% at the nucleic acid level, or more preferably at least about 80% at the nucleic acid level or 90% at the nucleic acid level.

In determining whether or not two nucleotide sequences fall within these defined percentage identity limits, those skilled in the art will be aware that it is possible to conduct a side-by-side comparison of the nucleotide sequences. In such comparisons or alignments, differences will arise in the positioning of non-identical residues depending upon the algorithm used to perform the alignment. In the present context, references to percentage identities and similarities between two or more nucleotide sequences shall be taken to refer to the number of identical and similar residues respectively, between said sequences as determined using any standard algorithm known to those skilled in the art. In particular, nucleotide identities and similarities are calculated using software of the Computer Genetics Group, Inc., University Research Park, Maddison, Wisconsin, United States of America, e.g., using the GAP program of Devereaux et al., Nucl. Acids Res. 12, 387-395, 1984, which utilizes the algorithm of Needleman and Wunsch, J. Mol. Biol. 48, 443-453, 1970. Alternatively, the CLUSTAL W algorithm of Thompson et al., Nucl. Acids Res. 22, 4673-4680, 1994, is used to obtain an alignment of multiple sequences, wherein it is necessary or desirable to maximize the number of identical/similar residues and to minimize the number and/or length of sequence gaps in the alignment. Nucleotide sequence alignments can also be performed using a variety of other commercially available sequence analysis programs, such as, for example, the BLAST program available at NCBI.

In an alternative embodiment, the genetic sequences of the prokaryotes fail to cross hybridize in a standard Cot analysis. The skilled artisan will be aware that standard Cot analyzes determine the similarity between two nucleotide sequences at the nucleotide level by using renaturation-kinetics of the corresponding nucleic acids (e.g., Britten and Kohne Science, 161, 529-540, 1968).

Where more than one substantially sequenced genome used to produce the expression library, it is also preferred that the fragments from each distinct prokaryote are used in an amount proportional to the complexity and size of the genome of said prokaryote. As the genomes of the prokaryotes are substantially sequenced the approximate size of said genome is determined. Accordingly, library is normalized to ensure that the amount of nucleic acids from all of the incorporated genomes to the final expression library is equal. In a particularly preferred embodiment, the nucleic acid fragment expression libraries are normalized such that nucleic acid fragments from each of the prokaryotes or compact eukaryotes are incorporated in equimolar amounts. In one exemplified embodiment, the sizes (in Mbp or molecular weight) of the genomes to be used in the expression library are compared and nucleic acid from each genome is used in an amount that is proportional to the ration of genome size to the size of the smallest contributing genome for the library. For example, the genome of *T. rubripes* is about 400Mb in size, compared to the genome of *A. thaliana,* which is only about 120Mb. Accordingly, for a combination of genomic *T. rubripes* and *A. thaliana* nucleic acid fragments, the ratio of *T. rubripes* nucleic acid fragments to *A. thaliana* nucleic acid fragments would be about 4:1.2 (w/w). A library comprising nucleic acid from, for example, *Bordetella pertussis, Borrelia burgdorferi and Haemophilus influenzae* would include the following ratio of nucleic acid from each organism 4.07:1:1.91, respectively The relative contributions of nucleic acid fragments for constructing expression libraries from multiple genomes are readily calculated from the information presented in Table 1.

**TABLE 1**

| Sizes of genomes of organisms from which nucleic acid fragments are derived for construction of expression libraries | |
|---|---|
| Source of nucleic acid fragments | Approx. genome size (Mb) |
| *Actinobacillus pleuropneumoniae* | 2.2 |
| *Aeropyrum pernix* | 1.6-1.7 |
| *Agrobacterium pernix* | 1.67 |
| *Anopheles gambiae* | 26-27 |
| *Arabidopsis thaliana* | 120 |
| *Aquifex aeolicus* | 1.5-1.6 |
| *Archaeoglobus fulgidis* | 1.7 |
| *Bacillus anthracis* | 5.09 |
| *Acillus cereus* | 5.4 |
| *Bacillus halodurans* | 4.2 |
| *Bacillus subtilis* | 4.2 |
| *Bacteroides thetaiotaomicron* | 6.2 |
| *Bdellovibrio bacteriovorus* | 3.8 |
| *Bifidobacterium longum* | 2.3 |
| *Bordetella bronchiseptica* | 5.34 |
| *Bordetall parapertusis* | 4.77 |
| *Bordetella pertussis* | 3.91 |
| *Borellia afzelii* | 0.95 |
| *Borellia garinii* | 0.95 |
| *Borrelia burgdorferi* | 0.91-0.96 |
| *Bradyrhizobium japonicum* | 9.11 |
| *Brucella melitensis* | 3.2 |
| *Brucella suis* | 3.29 |
| *Brugia malayi* | 100 |
| *Buchnera aphidicola* | 0.64 |
| *Caenorhabditis elegans* | 97-102 |
| *Campylobacter jejuni* | 1.64 |
| *Candidalus blochmannia floridanus* | 0.7 |
| *Caulobacter crescentus* | 4.01 |
| *Chlamydia muridarum* | 1.07 |
| *Chlamydia pneumoniae* | 1.22 |
| *Chlamydia trachomatis* | 1.0-1.1 |
| *Chlamydophila caviae* | 3.53 |
| *Chlamydophila pneumoniae* | 1.23 |
| *Chlorobium tepidum* | 2.1 |
| *Chlostridium acetobutylicum* | 4.1 |
| *Chromobacterium violaceum* | 4.8 |
| *Clostridium acetobutylicum* | 3.94 |
| *Clostridium perfringens* | 3.03 |
| *Clostridium tetani* | 4.1 |
| *Corynebacterium diphtheriae* | 2.49 |
| *Corynebacterium efficiens* | 3.15 |
| *Corynebacterium glutamicum* | 3.31 |
| *Coxiella burnetii* | 2.0 |
| *Danio rerio* | 1700 |
| *Dechloromonas aromatica* | 4.50 |
| *Deinococcus radiodurans* | 3.28 |
| *Drosophila melanogaster* | 120 |
| *Eimeria acervulina* | 70 |
| *Eimeria tenella* | 70 |
| *Entamoeba hystolitica* | 40 |
| *Emerococcus faecalis* | 3.36 |
| *Escherichia coli* | 4.6-5.6 |
| *Fusobacterium nucleatum* | 4.33 |
| *Geobacter sulfurreducens* | 3.85 |
| *Gloebacter violaceus* | 4.7 |
| *Haemophilus ducreyi* | 1.7 |
| *Haemophilus influenzae* | 1.83 |
| *Halobacterium sp.* | 2.57 |
| *Helicobacter hepaticus* | 1.8 |
| *Helicobacter pylori* | 1.66 |
| *Lactobacillus johnsonii* | 2.0 |
| *Lactobacillus plantarum* | 3.3 |
| *Lactococcus lactis* | 2.36 |
| *Leptospira interrogans serovar lai* | 4.6 |

| Source of nucleic acid fragments | Approx. genome size (Mb) |
|---|---|
| *Listeria innocua* | 3.01 |
| *Listeria monocytogenes* | 2.94 |
| *Mesorhizobium loti* | 7.59 |
| *Methanobacterium thermoautotrophicum* | 1.75 |
| *Methanocaldococcus jannaschii* | 1.66 |
| *Methanococcoides burtonii* | 2.6 |
| *Methanopyrus kandleri* | 1.69 |
| *Methanosarcina acetivorans* | 5.75 |
| *Methanosarcina mazei Goe1* | 4.1 |
| *Methanothermobacter thermautotrophicus* | 1.75 |
| *Mycobacterium avium sp.* | 4.96 |
| *Mycobacterium bovis* | 4.35 |
| *Mycobacterium leprae* | 2.8 |
| *Mycobacterium tuberculosis* | 4.4 |
| *Mycoplasma gallisepticum strain R* | 1.0 |
| *Mycoplasma genitalium* | 0.58 |
| *Mycoplasma penetrans* | 1.36 |
| *Mycoplasma pneumoniae* | 0.81 |
| *Mycoplasma pulmonis* | 0.96 |
| *Nanoarchaeum equitans Kin4* | 0.49 |
| *Neisseria meningitidis* | 2.18-2.27 |
| *Nitrosomonas europaea* | 2.81 |
| *Nostoc sp.* | 6.41 |
| *Oceanobacillus iheyensis* | 3.6 |
| *Onion yellows phytoplasma* | 0.86 |
| *Oryza sativa* | 400 |
| *Pasturella multocida* | 2.4 |
| *Photorhabdus luminescens sp.* | 5.7 |
| *Pirellula sp.* | 7.1 |
| *Porphyromonas gingivalis* | 2.34 |
| *Plasmodium berghei* | 25 |
| *Plasmodium falciparum* | 25 |
| *Plasmodium yoelii* | 23 |
| *Plasmodium vivax* | 30 |
| *Prochlorococcus marinus str.* | 2.41 |
| *Pseudomonas aeruginosa* | 6.3 |
| *Pseudomonas putida* | 6.1 |
| *Pseudomonas syringae* | 6.4 |
| *Pyrobaculum aerophilum* | 2.2 |
| *Pyrococcus abyssi* | 1.77 |
| *Pyrococcus furiosus* | 1.91 |
| *Pyrococcus horikoshii* | 1.74 |
| *Ralstonia solanacearum* | 5.80 |
| *Rhodopseudomonas palustris* | 5.46 |
| *Ricketsia conorii* | 1.27 |
| *Ricketsia prowazekii* | 1.1 |
| *Ricketsia rickettsii* | 1.3 |
| *Saccharomyces cerevesiae* | 13.0 |
| *Salmonella enterica* | 4.8 |
| *Salmonella typhimurium* | 4.8 |
| *Sarcocystis cruzi* | 201 |
| *Schizosaccharomyces pombe* | 13.8-14.0 |
| *Schistosoma mansoni* | 270 |
| *Shewanalla oneidensis* | 5.14 |
| *Shigella flexneri* | 4.7 |
| *Sinorhizobium meliloti* | 6.7 |
| *Staphylococcus aureus* | 2.8 |
| *Staphylococcus epidermidis* | 2.6 |
| *Streptococcus agalactiae* | 2.21 |
| *Streptococcus mutans* | 2.03 |
| *Streptococcus pneumoniae* | 2.2 |
| *Streptococcus pyogenes* | 1.85 |
| *Streptomyces avermitilis* | 9 |
| *Streptomyces coelicolor* | 8.7 |
| *Sulfolobus solfataricus* | 2.99 |
| *Sulfolobus tokodaii* | 2.81 |
| *Synechococcus sp.* | 2.43 |
| *Synechocystis PCC 6803* | 3.57 |
| *Takifugu rubripes* | 400 |
| *Thermoplasma volcanium* | 1.56-1.58 |
| *Thermoanaerobacter tengcongensis* | 2.69 |
| *Thermoplasma acidophilum* | 1.56 |
| *Thermoplasma volcanium* | 1.58 |
| *Thermotoga maritime* | 1.80 |
| *Thermotoga pallidum* | 1.14 |
| *Toxoplasma gondii* | 89 |
| *Treponema denticola* | 3.06 |
| *Treponema pallidum* | 1.14 |
| *Tropheryma whipplei* | 0.93 |
| *Trypanosoma brucei* | 35 |
| *Trypanosoma cruzi* | 40 |
| *Ureaplasma urealyticum* | 0.75 |
| *Vibrio cholerae* | 4 |
| *Vibro parahaemolyticus* | 5.2 |
| *Vibrio vulnificus* | 5.1 |
| *Wigglesworthia brevipa*/*vis* | 0.7 |
| *Wolbachia endosymbiont of Drosophila melanogaster* | 1.27 |
| *Wolinella succinogenes* | 2.1 |
| *Xanthomonas axonopodis* | 5.17 |
| *Xanthomonas campestris* | 5.07 |
| *Xvlella fastidiosa* | 2.68 |
| *Yersinia pestis* | 4.65 |

To increase the diversity of the peptides encoded by the expression library nucleic acid fragments are selected that are from mixtures of organisms, preferably those organisms that are not normally found together in nature.

More preferably, nucleic acid is selected from organisms in which the phenotype of interest does not occur in nature. For example, should the phenotype occur in a mammalian cell, peptides derived from a plurality of bacterial cells are preferred for performance of the invention.

The nucleic acid fragments or cDNA or amplified DNA derived therefrom are inserted into a suitable vector or gene construct in operable connection with a suitable promoter for expression of each peptide in the diverse nucleic acid sample. The construct used for the expression of the diverse nucleic acid fragment library is determined by the system that will be used to screen for those peptides that have a conformation sufficient for binding to a target protein or nucleic acid. Thus, consideration is generally given to an expression format suitable for screening the library.

In a preferred embodiment, the nucleic acid fragments of the present invention are expressed in a cell in which they are screened. As will be apparent to the skilled artisan, to facilitate expression of the nucleic acid fragment(s) in a cell, the fragment may be placed in operable connection with a promoter to produce an expression construct.

The term "gene construct" or "expression construct" is to be taken in its broadest context and includes a promoter sequence that is placed in operable connection with a nucleic acid fragment of the present invention. The nucleic acid comprising the promoter sequence is isolated using a technique known in the art, such as for example PCR or restriction digestion. Alternatively, the nucleic acid comprising the promoter sequence is synthetic, e.g., an oligonucleotide. Methods for producing an oligonucleotide are known in the art and are described, for example, in Oligonucleotide Synthesis: A Practical Approach (M. J. Gait, ed., 1984) IRL Press, Oxford, whole of text, and particularly the papers therein by Gait, ppl-22; Atkinson *et al*., pp35-81; Sproat *et al*., pp 83-115; and Wu *et al*., pp 135-151.

The term "promoter" is to be taken in its broadest context and includes the transcriptional regulatory sequences of a genomic gene, including the TATA box or initiator element, which is required for accurate transcription initiation, with or without additional regulatory elements (i.e., upstream activating sequences, transcription factor binding sites, enhancers and silencers) which alter gene expression in response to developmental and/or external stimuli, or in a tissue specific manner. In the present context, the term "promoter" is also used to describe a recombinant, synthetic or fusion molecule, or derivative which confers, activates or enhances the expression of a nucleic acid molecule to which it is operably linked, and which encodes the peptide or protein. Preferred promoters can contain additional copies of one or more specific regulatory elements to further enhance expression and/or alter the spatial expression and/or temporal expression of said nucleic acid molecule.

Placing a nucleic acid molecule under the regulatory control of, i.e., "in operable connection with", a promoter sequence means positioning said molecule such that expression is controlled by the promoter sequence. Promoters are generally positioned 5' (upstream) to the coding sequence that they control. To construct heterologous promoter/structural gene combinations, it is generally preferred to position the promoter at a distance from the gene transcription start site that is approximately the same as the distance between that promoter and the gene it controls in its natural setting, i.e., the gene from which the promoter is derived. As is known in the art, some variation in this distance can be accommodated without loss of promoter function. Similarly, the preferred positioning of a regulatory sequence element with respect to a heterologous gene to be placed under its control is defined by the positioning of the element in its natural setting, i.e., the gene from which it is derived. Again, as is known in the art, some variation in this distance can also occur.

Typical promoters suitable for expression in bacterial cells, such as, for example, a bacterial cell selected from the group comprising *E. coli, Staphylococcus sp, Corynebacterium sp., Salmonella sp., Bacillus sp.,* and *Pseudomonas sp.,* include, but are not limited to, the *lacz* promoter, the Ipp promoter, temperature-sensitive λ_{L} or λ_{R} promoters, T7 promoter, T3 promoter, SP6 promoter or semi-artificial promoters such as the IPTG-inducible *tac* promoter or lacUV5 promoter. A number of other gene construct systems for expressing the nucleic acid fragment of the invention in bacterial cells are known in the art and are described, for example, in Ausubel et al (In: Current Protocols in Molecular Biology. Wiley Interscience, ISBN 047 150338, 1987) and (Sambrook et al (In: Molecular Cloning: Molecular Cloning: A Laboratory Manual, Cold Spring Harbor Laboratories, New York, Third Edition 2001). Such promoters are available in the form of expression vectors, such as, for example, PKC30 (Shimatake and Rosenberg, Nature 292, 128, 1981); pKK173-3 (Amann and Brosius, Gene 40, 183, 1985), pET-3 (Studier and Moffat, J. Mol. Biol. 189, 113, 1986); the pCR vector suite (Invitrogen), pGEM-T Easy vectors (Promega), the pL expression vector suite (Invitrogen).

Preferably, a peptide of the present invention is expressed in a yeast cell. Typical promoters suitable for expression in yeast cells, such as, for example, a yeast cell selected from the group comprising *Pichia pastoris, Saccharomyces cerevisiae* or *Schizosaccharomyces pombe,* include, but are not limited to, an *ADH1* promoter, a *GAL1* promoter, a *GAL4* promoter, a *CUP1* promoter, a *PHO5* promoter, a *nmt* promoter, a *RPR1* promoter, or a *TEF1* promoter. Typical expression vectors useful for the expression of a peptide in a yeast cell include, for example, the pACT vector (Clontech), the pDBleu-X vector, the pPIC vector suite (Invitrogen), the pGAPZ vector suite (Invitrogen), the pHYB vector (Invitrogen), the pYD1 vector (Invitrogen), and the pNMT1, pNMT41, pNMT81 TOPO vectors (Invitrogen), the pPC86-Y vector (Invitrogen), the pRH series of vectors (Invitrogen), pYESTrp series of vectors (Invitrogen).

Typical promoters suitable for expression in insect cells, or in insects, include, but are not limited to, the OPEI2 promoter, the insect actin promoter isolated from *Bombyx muri*, the *Drosophila sp. dsh* promoter (Marsh et al Hum. Mol. Genet. 9: 13-25, 2000) and the inducible metallothionein promoter. Preferred insect cells for expression of the recombinant polypeptides include an insect cell selected from the group comprising, BT1-TN-5B1-4 cells, and *Spodoptera frugiperda* cells (e.g., sf19 cells, sf21 cells). Suitable insects for the expression of the nucleic acid fragments include but are not limited to *Drosophila sp.* The use of *S. frugiperda* is also contemplated.

Promoters for expressing peptides in plant cells are known in the art, and include, but are not limited to, the *Hordeum vulgare* amylase gene promoter, the cauliflower mosaic virus 35S promoter, the nopaline synthase (NOS) gene promoter, and the auxin inducible plant promoters P1 and P2.

In another preferred embodiment, a peptide of the present invention is expressed in a mammalian cell, preferably, a human cell, more preferably, a human cell line (e.g., a cancer cell line). Typical promoters suitable for expression in a mammalian cell, mammalian tissue or intact mammal include, for example a promoter selected from the group consisting of, retroviral LTR elements, the SV40 early promoter, the SV40 late promoter, the cytomegalovirus (CMV) promoter, the CMV IE (cytomegalovirus immediate early) promoter, the EF_{1α} promoter (from human elongation factor 1α), the EM7 promoter, the UbC promoter (from human ubiquitin C). Expression vectors that contain suitable promoter sequences for expression in mammalian cells or mammals include, but are not limited to, the pcDNA vector suite supplied by Invitrogen, the pCI vector suite (Promega), the pCMV vector suite (Clontech), the pM vector (Clontech), the pSI vector (Promega) or the VP16 vector (Clontech).

Following production of a suitable gene construct, said construct is introduced into the relevant cell. Methods for introducing the gene constructs into a cell or organism for expression are known to those skilled in the art and are described for example, in Ausubel et al (In: Current Protocols in Molecular Biology. Wiley Interscience, ISBN 047 150338, 1987) and (Sambrook et al (In: Molecular Cloning: Molecular Cloning: A Laboratory Manual, Cold Spring Harbor Laboratories, New York, Third Edition 2001). The method chosen to introduce the gene construct in depends upon the cell type in which the gene construct is to be expressed. Means for introducing recombinant DNA into a cell includes, but is not limited to electroporation or chemical transformation into cells previously treated to allow for said transformation, PEG mediated transformation, microinjection, transfection mediated by DEAE-dextran, transfection mediated by calcium phosphate, transfection mediated by liposomes such as by using Lipofectamine (Invitrogen) and/or cellfectin (Invitrogen), transduction by adenoviuses, herpesviruses, togaviruses or retroviruses and microparticle bombardment such as by using DNA-coated tungsten or gold particles (Agacetus Inc., WI,USA).

Accordingly, it is preferred that the peptides screened in the method of the invention are produced by a method comprising:
(i) producing nucleic acid fragments from nucleic acids derived from two or more prokaryotes containing compact genomes having a genome size of less than 1700 Mbp, each of said prokaryotes having a substantially sequenced genome;
(ii) inserting the nucleic acid fragments at (i) into a suitable expression construct in an amount proportional to the size of the genome from which the fragments were derived thereby producing recombinant constructs, wherein each fragment is in operable connection with a promoter sequence that is capable of conferring expression of that fragment; and
(iii) expressing the fragments at (ii) in a cell, tissue or non-human organism that expresses the phenotype to be modulated, wherein the cell is a yeast, insect, plant or mammlian cell.

In one embodiment, the cell, tissue or animal comprises a complex genome.

In another preferred embodiment, the nucleic acid fragments are derived from two or more bacterium.

The present disclosure provides a method for identifying a peptide capable of modulating a phenotype in a cell, tissue or animal comprises:
(i) producing nucleic acid fragments from nucleic acids derived from two or more bacterium (e.g., each of said bacterium having a substantially sequenced genome);
(ii) inserting the nucleic acid fragments at (i) into a suitable expression construct in an amount proportional to the size of the genome from which the fragments were derived thereby producing recombinant constructs, wherein each fragment is in operable connection with a promoter sequence that is capable of conferring expression of that fragment;
(iii) expressing the fragments at (ii) in a cell, tissue or animal other than a bacterium, said cell tissue or organism capable of expresses the phenotype to be modulated;
(iv) selecting a cell, tissue or animal from (iii) in which the phenotype is modulated; and
(v) identifying an introduced peptide that modulates the phenotype in the selected cell or animal, wherein the peptide does not modulate the peptide in its native environment.

In embodiments of the invention, the phenotype is death of a cell, tissue or non-human organism. In accordance with this embodiment, it is preferred that the the peptide reduces or prevents death and/or enhances or induces growth of the cell, tissue or non-human organism.

Clearly, the present invention also contemplates a library of cells and/or peptides and/or nucleic acids produced by the methods described herein.

The present disclosure provides an expression library comprising nucleic acid fragments derived from two or more microorganisms selected from the group consisting of *Archaeoglobus fulgidus, Aquifex aeolicus, Aeropyrum pernix, Bacillus subtilis, Bordetella pertussis, Borrelia burgdorferi, Chlamydia trachomatis, Escherichia coli K12, Haemophilus influenzae, Helicobacter pylori, Methanobacterium thermoautotrophicum*., *Methanococcus jannashii, Neisseria meningitidis, Pyrococcus horikoshii, Pseudomonas aeruginosa, Synechocystis PCC 6803, Thermoplasma volcanicum, Thermotoga maritima, Acidobacterium capsulatum, Halobacterium salinarum, Desulfobacterium autotrophicum, Haloferax volcanii, Rhodopirellula baltica, Thermus thermophilus HB27 and Prochlorococcus marinus MED4,* and wherein the nucleic acid fragments are inserted into an expression vector thereby producing recombinant constructs wherein each fragment is in operable connection with a promoter sequence that is capable of conferring expression of that fragment.

Preferably, the nucleic acid fragments of the library comprise an open reading frame having an average length of at least about 10 to 200 nucleotide residues and/or encode a protein domain. Preferably, the nucleic acid fragments do not encode an entire polypeptide.

The present disclosure additionally provides the expression library *supra* when used in a screening method described herein.

In another embodiment, the candidate peptide is produced by recombinant means and then administered to the cell, tissue or organism. Methods for the production of a recombinant peptide are known in the art and described, for example, in Ausubel et al (In: Current Protocols in Molecular Biology. Wiley Interscience, ISBN 047 150338, 1987) and (Sambrook et al (In: Molecular Cloning: Molecular Cloning: A Laboratory Manual, Cold Spring Harbor Laboratories, New York, Third Edition 2001). Again, such a method involves the insertion of a nucleic acid fragment into an expression construct. Suitable expression constructs are known in the art and/or described herein.

In one embodiment, the peptide is expressed as a fusion with a polypeptide that facilitates isolation of the peptide of interest. Such "tags" include, but are not limited to influenza virus hemagglutinin (HA), Simian Virus 5 (V5, polyhistidine (e.g. 6xHis), c-myc, FLAG, epitope tags as described by Slootstra et al. Mol Divers 2(3):156-164, 1997, GST (glutathione-S-transferase), MBP (maltose binding protein), GAL4, β-galactosidase. Alternatively, the peptide encoded by a nucleic acid fragment is labeled with a protein that directly associates with another known protein, such as for example, biotin, strepavidin or Strep-Tag, an 8 amino acid strepavidin binding peptide (available from Sigma-Genosys, Sydney, Australia).

Methods for isolating a protein from a cellular source are known in the art and described, for example, in Scopes (In: Protein Purification: Principles and Practice, Third Edition, Springer Verlag, 1994). For example, a peptide, polypeptide or protein is isolated using affinity purification. For example, an antibody or ligand capable of binding to the fusion protein is coupled to a solid support. Cell medium or cell lysate comprising the peptide fusion of interest is then passed over the solid support. Following washing, the fusion peptide is eluted using a method known in the art.

In one embodiment, the polypeptide that facilitates isolation of the peptide of interest is a cleavable tag. As used herein, the term "cleavable tag" shall be taken to mean that the fusion polypeptide is capable of being removed from the peptide of interest, for example, by cleavage with a protease. For example, Hopp, et al. Biotechnology 6: 1204-1210, 1988 describe a FLAG peptide that is cleavable using enterokinase.

Alternatively, the IMPACT System available from New England Biolabs is useful for isolation of a recombinant peptide using a chitin column. The self-cleavable intein tag is induced to self-cleave in the presence of DTT, facilitating isolation of the peptide fused to the tag.

In another embodiment, the peptide is produced is produced using synthetic means, for example BOC or FMOC chemistry. Synthetic peptides are prepared using known techniques of solid phase, liquid phase, or peptide condensation, or any combination thereof, and can include natural and/or unnatural amino acids. Amino acids used for peptide synthesis may be standard Boc (Nα-amino protected Nα-t-butyloxycarbonyl) amino acid resin with the deprotecting, neutralization, coupling and wash protocols of the original solid phase procedure of Merrifield, J. Am. Chem. Soc., 85:2149-2154, 1963, or the base-labile Nα-amino protected 9-fluorenylmethoxycarbonyl (Fmoc) amino acids described by Carpino and Han, J. Org. Chem., 37:3403-3409, 1972. Both Fmoc and Boc Nα-amino protected amino acids can be obtained from various commercial sources, such as, for example, Fluka, Bachem, Advanced Chemtech, Sigma, Cambridge Research Biochemical, Bachem, or Peninsula Labs.

Synthetic peptides may also be produced using techniques known in the art and described, for example, in Stewart and Young (In: Solid Phase Synthesis, Second Edition, Pierce Chemical Co., Rockford, Ill. (1984) and/or Fields and Noble (Int. J. Pept. Protein Res., 35:161-214, 1990), or using automated synthesizers. Accordingly, peptides of the invention may comprise D-amino acids, a combination of D- and L-amino acids, and various unnatural amino acids (e.g., β-methyl amino acids, Cα-methyl amino acids, and Nα-methyl amino acids, etc) to convey special properties. Synthetic amino acids include ornithine for lysine, fluorophenylalanine for phenylalanine, and norleucine for leucine or isoleucine.

In one embodiment, a peptide that is to be administered to a cell, tissue or organism is administered with and/or conjugated to a compound or peptide that facilitates uptake of the peptide (e.g. a peptide that facilitates a peptide crossing a membrane).

In one embodiment the peptide encoded by the nucleic acid fragment of the present invention is expressed as a fusion protein or produced by chemical or synthetic means with a peptide sequence capable of enhancing, increasing or assisting penetration or uptake of the peptide by cells either in vitro or in vivo. For example, the peptide sequence capable of enhancing, increasing or assisting penetration or uptake is the Drosophila penetratin targeting sequence. This peptide sequence at least comprises the amino acid sequence:
CysArgGlnIleLysIleTrpPheGlnAsnArgArgMetLysTrpLysLys (SEQ ID NO. 29) further comprising (Xaa)n after the final Lys residue and followed by Cys wherein Xaa is any amino acid and n has a value greater than or equal to 1. Alternatively, a homologue, derivative or analogue of said sequence is used.

In an alternative embodiment, the peptide encoded by the nucleic acid fragment of the present invention is mixed with a peptide capable of enhancing, increasing or assisting penetration or uptake by cells in vitro or in vivo. A peptide sequence that is able to increase or assist penetration or uptake of cells is the synthetic peptide Pep 1, which at least comprises the amino acid sequence:
LysGluThrTrpTrpGluThrTrpTrpThrGluTrpSerGlnLysLysLysLysArgLysVal (SEQ ID NO. 30).

The Pep1 peptide does not need to be conjugated to the peptide encoded by the nucleic acid fragments of the present invention. Furthermore, Pep1 dissociates from the peptide encoded by the expression library of the present disclosure. Thus Pep1 will not interfere with the peptide forming a conformation sufficient for binding to a target protein or nucleic acid.

Alternative protein transduction domains are known in the art, and include, for example, TAT fragment 48-60 (GRKKRRQRRRPPQ, SEQ ID NO: 31), signal sequence based peptide 1 (GALFLGWLGAAGSTMGAWSQPKKKRKV, SEQ ID NO: 32), signal sequence based peptide 2 (AAVALLPAVLLALLAP, SEQ ID NO: 33), transportan (GWTLNSAGYLLKINLKALAALAKKIL, SEQ ID NO: 34), amphiphilic model peptide (KLALKLALKALKAALKLA, SEQ ID NO: 35), polyarginine (e.g., RRRRRRRRRRR, SEQ ID NO: 36)

In another embodiment, a peptide is selected or identified that is capable of modulating the phenotype in a cell, tissue or animal without necessarily penetrating or entering a cell. Such a peptide may, for example, bind to and activate or suppress activation of a cell surface receptor.

In another embodiment, a peptide is selected or identified that is capable of penetrating or entering a cell and modulating the phenotype of interest in a cell, tissue or animal.

The present disclosure also provides a method for identifying a peptide capable of modulating a phenotype in a cell, tissue or animal comprises:
(i) producing nucleic acid fragments from nucleic acids derived from two or more microorganisms or eukaryotes containing compact genomes, each of said microorganisms or eukaryotes having a substantially sequenced genome;
(ii) inserting the nucleic acid fragments at (i) into a suitable expression construct in an amount proportional to the size of the genome from which the fragments were derived thereby producing recombinant constructs, wherein each fragment is in operable connection with a promoter sequence that is capable of conferring expression of that fragment;
(iii) expressing the fragments at (ii) to produce candidate peptides;
(iii) introducing the candidate peptides (iii) into a cell, tissue or animal from a different kingdom to that/those from which the nucleic acid fragment/s were derived, said cell tissue or organism capable of expresses the phenotype to be modulated;
(iv) selecting a cell, tissue or animal from (iii) in which the phenotype is modulated; and
(v) identifying an introduced peptide that modulates the phenotype in the selected cell or animal, wherein the peptide does not modulate the peptide in its native environment.

The present disclosure also provides a method for identifying a peptide capable of modulating a phenotype in a cell, tissue or animal, wherein the phenotype is death or reduced or prevented growth of the cell, tissue or organism comprises:
(i) producing nucleic acid fragments from nucleic acids derived from two or more microorganisms or eukaryotes containing compact genomes, each of said microorganisms or eukaryotes having a substantially sequenced genome;
(ii) inserting the nucleic acid fragments at (i) into a suitable expression construct in an amount proportional to the size of the genome from which the fragments were derived thereby producing recombinant constructs, wherein each fragment is in operable connection with a promoter sequence that is capable of conferring expression of that fragment;
(iii) expressing the fragments at (ii) to produce candidate peptides;
(iii) introducing the candidate peptides (iii) into a cell, tissue or animal capable of expressing the phenotype to be modulated;
(iv) selecting a cell, tissue or animal that survives and/or is capable of growing; and
(v) identifying an introduced peptide that induces survival or growth of the cell, tissue or organism wherein the peptide does not induce survival of the cell tissue or organism in its native environment.

In another embodiment, the peptide is expressed in a first cell as a fusion with a secretory signal peptide. The first cell is then bought into contact with the same culture or incubation medium as a cell in which a screen is to be performed (e.g., the first cell may be a feeder layer of cells). The peptide is then secreted from the first cell and may bind to a membrane protein or an extracellular domain of a protein of the second cell thereby modulating a phenotype of interest.

Alternatvely, the peptide may be fused to or conjugated to a protein transduction domain such that the secreted peptide can be translocated into the cell being screened and bind to an intracellular target thereby modulating the phenotype.

In accordance with the embodiments described in the previous two paragraphs the signal peptide is preferably cleaved with the expressed peptide is secreted.

In a particularly preferred embodiment, the method of the present invention involves screening a plurality of peptides (i.e. a library of peptides) to determine a peptide capable of modulating a phenotype of interest. In screening a library, it is preferred that each peptide is screened individually to determine whether or not it is capable of modulating an allele and/or a phenotype of interest.

In one embodiment, the method of the present invention screens a pool (or a plurality of peptides or library of peptides) to determine a pool of peptides that are capable of modulating a phenotype of interest. Preferably, the pooled library is an arrayed library. As used herein "arrayed expression library" shall be taken to mean that the library is assembled in such a way that an individual peptide and/or nucleic acid encoding same is readily identified. For example, each candidate peptide produced in the method of the present invention is produced individually (i.e., in isolation from other peptides), a number or a plurality of different peptides are then pooled. Two or more of these pools of peptides are then pooled, and if necessary, this process is repeated. Accordingly, pools of several thousands or millions of peptides may be produced. The largest of these pools is then screened to determine whether or not it comprises a peptide capable of modulating a phenotype of interest. Should it comprise such a peptide, one or more groups of smaller pools (i.e. sub-pools) of peptides are screened to determine which comprise the peptide of interest. Clearly this process can be iteratively repeated with pools of descending size until the individual peptide of interest is isolated (i.e., the pool of peptides is deconvoluted). Alternatively, a pool of a smaller number of peptides (e.g. 10 or 100) may be directly screened to determine which, if any, of the peptides are capable of modulating a phenotype of interest.

It is also possible to discriminate individual peptides from mixtures of up to about 100 peptides by mass spectrometry during the screening process. Similarly, small pools of cells expressing different peptides can be readily discriminated by mass spectrometry. The individual peptides can then be readily synthesized using standard methods from the mass spectrometry data and their efficacy validated. Methods for validating a peptide will be apparent to the skilled person, e.g., using a method described herein. For example, the peptide is administered to a cell, tissue or organism and its effect on the phenotype of interest determined. Alternatively, or in addition, the peptide is administered to an animal (e.g., an animal model of a disease) and its effect on the phenotype of interest (e.g., the disease phenotype) is determined along with any other phenotypes that the peptide may modulate (e.g., toxicology screening).

As will be apparent to the skilled artisan the present disclosure clearly encompasses the production of multiple different libraries. Accordingly, the present disclosure also includes pooled libraries. For example, the present disclosure encompasses the pooling of two or more libraries. The libraries may be derived from the same organism/s. The libraries may be derived from different organisms (e.g. a library derived from eukaryotes comprising a compact genome, and another library derived from bacteria).

### Suitable phenotypes

Clearly, any phenotype is encompassed by the present disclosure. Preferably, the phenotype is detectable and more preferably, measurable. For example, a phenotype encompassed by the present invention is an intra-cellular event such as, for example, expression of a gene, expression of a protein, modification of a protein (e.g., phosphorylation or glycosylation), activation of a protein, cleavage of a protein, signal transduction, endocytosis or exocytosis amongst others; to cellular events, such as for example, cell death, cell survival, cell signaling (e.g., neuronal signalling), cell structure (mediated by intracellular scaffolds), differentiation, dedifferentiation or cell movement amongst others; to phenotypes such as, for example, tissue organization, growth of an organism, development of an organism, neurodegeneration, obesity, diabetes, cancer, metastasis of a cancer, an immune response, inflammation, allergy or death of an organism.

In embodiments of the invention, the phenotype is cell death and/or an increased level of cell death. In accordance with this embodiment, the peptide preferably reduces cell death or prevents cell death. Preferably, the reduced or prevented cell death and/or an increased level of cell death is caused by and/or associated with an allele in the cell, tissue or non-human organism.

In another preferred embodiment, the phenotype is death of the cell, tissue or non-human organism and the identified peptide induces survival of the cell, tissue or non-human organism and wherein said allele induces the phenotype in the absence of a substrate or compound that is converted into a cytotoxic or cytostatic compound.

With regard to determining a peptide capable of modulating the expression of a nucleic acid or a protein in a cell, it is preferable that the nucleic acid or protein the expression of which is modulated is endogenous to the cell. Preferably, the nucleic acid or protein is not a reporter molecule.

The phenotype of interest may be naturally occurring, e.g., a plant may have a resistance to some forms of insecticide and it is desirable to enhance the resistance to enable greater concentrations of the insecticide to be used. Alternatively, a cancer cell is resistant to a particular chemotherapy drug (for example, as occurs in a cancer cell with a mutation in an ATP-binding cassette superfamily protein), and it is desirable to reduce the resistance of the cell to enhance treatment of the cancer. Accordingly, selecting a cell expressing the phenotype of interest may involve isolating a cancer cell line or alternatively, screening a number of cancer cell lines to determine a line that is resistant to the chemotherapeutic drug.

In accordance with the present embodiment, the allele that causes the mutation need not be known, but rather, the cell, tissue or animal may be selected by its phenotype. For example, the cell is selected by its inability to grow in the absence of a specific compound or protein, e.g., a growth factor or cytokine. Alternatively, a cell is selected that is unable to grow when a specific gene is expressed in the cell. Alternatively, a cell, tissue or organism is selected that is resistant to a specific compound. Methods for selecting such a cell will be apparent to the skilled person.

For example, a cell that is sensitive to a compound is selected by exposing the cell to the compound and determining the level of cell growth and/or cell death. Methods for determining the level of cell growth and/or cell death are known in the art and/or described herein.

Preferably, the phenotype is inherited in such a way as to suggest that the phenotype has a genetic source (e.g., is caused by an allele). Such a phenotype need not necessarily be associated with a deleterious mutation, but may be associated with or caused by a natural polymorphism in the population.

For example, cells expressing the angiotensin converting enzyme (ACE) polymorphism caused by an Alu element insertion, ACE II have reduced ACE activity and increased cell survival. Accordingly, a peptide that mimics the effect of the ACE-II mutation on other polymorphisms (i.e. ACE-ID or ACE-DD) may have similar effects on cell survival, with implications in ageing and longevity.

Alternatively, the phenotype may be associated with a mutation that has occurred in a cell or an organism. For example, many cancers are associated with a mutation in the p53 gene, thereby aiding the cell to develop uncontrolled cell growth.

In methods of the present disclosure, the phenotype can be associated with a mutation in a cell that induces a cancer, or alternatively, induces a phenotypic change that enables a transition to a cancerous or tumorigenic state. By "induces a transition" is meant that the mutation is one of several mutations that are required for development of a cancer and that the mutation causes one or more phenotypes associated with a tumorigenic state. Studying such a phenotype facilitates the identification of other proteins that are involved in developing a cancer, thereby enabling identification of a drug target. By way of example, the present inventors have studied a cell line that exhibits cytokine-induced cell growth. Accordingly, these cells grow uncontrollably in the presence of specific cytokines. By determining a peptide capable of inducing escape for the cytokine dependence, and subsequently identifying a protein to which the peptide binds, the present inventors are capable of identifying those proteins that are involved in the transformation of such a cell line, with these proteins representing attractive drug targets.

In methods of the present disclosure, the phenotype can be associated with a gene or protein that is involved in inflammation. In this regard, the phenotype need not necessarily be an inflammatory response that results in, for example, cell death or reduced or inhibited cell growth. Rather, the phenotype may be, for example, the dependence of a cell on the presence of a gene or protein that is associated with inflammation for the continued growth and/or survival of the cell.

The gene or protein involved in inflammation can be a cytokine gene or protein. Suitable cytokines will be apparent to the skilled person. For example, a pro-inflammatory cytokine is, for example, a cytokine selected from the group consisting of interleukin (IL)-1, IL-6, IL-8, IL-11, IL-12, tumor necrosis factor (TNF)-α, transforming growth factor(TGF)-β, interferon-α, interferon-β, leukemia inhibitory factor, oncostatin M, ciliary neurotrophic factor, platelet factor 4, platelet basic protein, neutrophil activating protein-2, macrophage inflammatory protein (MIP)-1β, monocyte chemoactractant protein (MCP)-1, MCP-2, MCP-3, lymphotactin, granulocyte-colony stimulating factor (G-CSF), granulocyte macrophage-colony stimulating factor (GM-CSF), erythropoietin (EPO) and regulated upon activation normal T expressed and presumable secreted chemokine (RANTES).

The cytokine can be an anti-inflammatory cytokine, such as, for example, IL-4, IL-10, IL-13 or IL-16.

Methods of the present disclosure, the phenotype of interest may be mediated by the presence or absence of a receptor of a protein involved in inflammation, for example, a cytokine receptor. Exemplary cytokine receptors include, for example, IL-2 receptor, IL-3 receptor, IL-4 receptor, IL-5 receptor, IL-6, receptor, IL-7 receptor, interferon-α receptor, interferon β receptor, soluble TNF-α receptor, TNF-β receptor or RANTES receptor.

As will be apparent from the foregoing, the present disclosure provides method for identifying a peptide capable of inducing cell growth on a cell that is dependent on the presence of a cytokine for cell growth, said method comprising:
(i) selecting or obtaining a cell that is dependent on the presence of a cytokine for cell growth;
(ii) expressing in the cell or introducing into the cell or contacting the cell with a candidate peptide that mimics the structure of a domain or subdomain of a protein;
(iii) maintaining the cell in the absence of the cytokine for a time sufficient for cell growth to occur;
(iii) selecting a cell capable of growing at (iii); and
(iv) identifying the expressed or introduced peptide that induces cell growth, wherein the peptide does not induce growth of the cell in its native environment.

### Suitable cells, tissues and/or non-human organisms

Suitable cells, tissues and/or non-human organisms for performance of the invention capable of expressing a phenotype of interest.

Such cells may already exist and/or be characterized. For example, as exemplified herein, a screen is performed using a cell that is dependent upon the presence of IL-3 for survival. Such a cell line is useful for identifying a peptide that induces IL-3 signaling thereby inducing survival of the cell. A peptide identified using this method is then useful for inducing a stem cell to proliferate and differentiate into a T cell to thereby assist in an inflammatory response. Accordingly, the phenotype of interest is the growth and differentiation of hematopoietic stem cells, however, the assay is performed in a cell in which there is reduced growth in the absence of IL-3.

Other suitable cells will be apparent to the skilled artisan. For example, cytokine dependent cells are known in the art as are cells that cannot grow in the presence of some cytokines. The former cells are useful for determining a compound that induces signaling of a specific cytokine. The latter cells are useful for determining an inhibitor of cytokine signaling. The following is a list of cells useful for screening using the method of the invention (the dependence and/or utility of each cell is indicated in brackets following the name of the cell): 1xN/2b (dependent on IL-7), 2D6 (dependent on IL12, IL2 , IL4 and/or IL7), 2D9 (IFN-gamma dependent), 2E8 (IL-7 dependent), 4-1.10 (useful for identifying inhibitors of oncostatin M resistance), 7TD1 (IL-6 dependent), 32D (IL3 and/or G-CSF dependent), 32D-G (G-CSF dependent), 32D-Epo (Epo dependent), 32D-GM (GM-CSF dependent), A9.12 (IL-2 dependent), A375 (IL1 and/or Oncostatin M and/or IL6 dependent), A375-R (useful for detection of TNF-alpha resistance inhibitors), A431 (EGF dependent), AKR-2B (TGF-alpha and/or TGF-beta dependent), AML-193 (IL-3 and/or G-CSF and/or GM-CSF dependent), ANBL-6 (IL-6 dependent), AP-16 (EGF dependent), AS-E2 (Epo dependent), ATH8 (IL-2 dependent), B6SUt-A (IL3 and/or GM-CSF and/or Epo dependent), B9 (IL6 and/or IL-11 dependent), B9-11 (IL-11 dependent), B9-1-3 (IL-13 dependent), B9-TY1 (IL-11 dependent), B13 (IL5 and/or IL3 dependent), BAC1.2F5 (M-CSF and/or GM-CSF dependent), BaF3 (IL-3 dependent), BALM-4 (IL-4 dependent), BC-1 ( IL10 dependent), BCL1 (IL-5 dependent), BT-20 (bFGF and/or GM-CSF and/or IL3 and/or TNF dependent), CCL-39 (alpha-thrornbin and/or bFGF and/or aFGF and/or insulin and/or EGF dependent), CCL-64 (TGF-beta and/or HGF dependent), CCL-185 (IL4 dependent), CESS (BCDF and/or TRF and/or IL6 dependent), CRL 1395 (bFGF dependent), CT.4S (IL4 dependent), CT6 (IL2 and/or IL4 and/or TNF-alpha and/or IL7 dependent), CTL44 (IL4 dependent), CTLL-2 (IL-2 and/or IL-4 dependent), D10 (IL-1 dependent), D36 (IL-10 dependent), Da (LIF and/or IL3 and/or GM-CSF and/or Epo and/or IL4 dependent), DAUDI (IFN-alpha dependent), DW34 (IL7 dependent), Ea3.17 (IL-3 dependent), EL4 (IL-1 dependent), EML C1 (SCF dependent), FBHE (aFGF and/or bFGF dependent), FDCPmix (CSF and/or IL3 dependent), FDCP1 (CSF and/or IL3 dependent), FDCP2 (IL2 and/or GM-CSF dependent), FL5.12 (IL-3 dependent), GF-D8 (GM-CSF and/or IL3 dependent), GM/SO (GM-CSF dependent), GNFS-60 (G-CSF and/or M-CSF and/or IL6 dependent), HCD57 (Epo dependent), HFB-1 (BCDF dependent), HL-60 (IFN-gamma and/or LIF and/or Activin A and/or G-CSF dependent), HT-2 (IL-2 dependent), HT55 (scatter factor and/or HGF dependent), HT115 (scatter factor and/or HGF dependent), IC-2 (IL-3 and/or GM-CSF and/or Epo and/or IL-4 dependent), INA-6 (IL-6 dependent), J774 (M-CSF dependent), JR-2-82 (BCDF dependent), KD83 (IL-6 dependent), KG-1 (CSF and/or TGF-beta and/or IL18 dependent), Kit225 (IL-2 dependent), KMT-2 (IL-3 dependent), KT-3 (IL-2 and/or IL-4 and/or IL6 dependent), KYM-1D4 (TNF-alpha an/or TNF-beta dependent), L4 (BCDF and/or IL-4 dependent), L138.8A (IL-3 and/or IL4 and/or IL9 dependent), L929 (TNF dependent), LBRM-33 (IL-1 dependent), L-M (TNF dependent), LyD9 (IL-3 and/or IL-7 dependent), M1 (LIF and/or IL-6 dependent), MC/9 (IL-3 dependent), MDBK (IFN-alpha dependent), MEB5 (EGF dependent), MH11 (IL-7 and/or SCF dependent), MH60-BSF-2 (IL-6 dependent), MLA-144 (IL-2 dependent), MO7E (IL-3 and/or GM-CSF and/or SCF dependent), Mono Mac 6 (IL-1 beta and/or IL6 dependent), MPC-11 (Activin A dependent), MV-3D9 (TGF-beta dependent), Nb2 (IL-7 dependent), NBFL (CNTF and/or LIF and/or Oncostatin M dependent), NFS-60 (G-CSF and/or IL-3 dependent), NKC3 (IL-2 dependent), NRK-49F (TGF dependent), PIL-6 (IL-6 dependent), PK15 (TNF dependent), Pno (IL-7 dependent), PT-18 (IL-3 and/or GM-CSF dependent), Ramos (IL-4 dependent), RAW264.7 (murine IFN-gamma dependent), RINm5F (IL1-alpha and/or IL1-beta dependent), RPMI 1788 (IL-1 dependent), S21 (for detecting inhibitors of IL-3), SAS-1 (GM-CSF or IL3 dependent), Sez627 (human IL-2 and/or human IL-4 dependent), SFME (EGF dependent), SKW6-Cl4 (BCDF and/or TRF dependent), SR-4987 (bFGF dependent), T10 (IL-11 dependent), T88 (IL-5 dependent), T88-M (IL-3 and/or IL5 dependent), T1165 (IL-6 and/or IL-11 dependent), TALL-103 (GM-CSF and/or IL5 dependent), TF-1 (IL-3 and/or IL-4 and/or IL-5 and/or IL-13 and/or GM-CSF and/or Epo and/or SCF dependent), TMD2 (IL-3 dependent), TS1 (IL-9 dependent), TSGH9201 (EGF dependent), UT-7 (Epo and/or IL-3 and/or GM-CSF dependent), XG-1 (IL-6 dependent), Y16 (IL-5 dependent) or YAPC (IL-1-alpha dependent).

A suitable source of such a cell will be apparent to the skilled person, and includes, for example, the ATCC.

In a preferred embodiment, the cell is dependent on the presence of a cytokine selected from the group consisting of cytokine is selected from the group consisting of interleukin-3 (IL-3), interferon, erythropoietin, granulocyte-colony stimulating factor (G-CSF), granulocyte/macrophage-colony stimulating factor (GM-CSF) and mixtures thereof.

In another embodiment, the phenotype of interest is induced in a cell. For example, the cell is contacted with a compound that induces the phenotype (e.g., a toxin) or, alternatively, the cell is genetically modified to express the phenotype of interest. Accordingly, a suitable cell is a cell that is sensitive to the compound or that is readily genetically modified. Suitable cells will be apparent to the skilled person and/or described herein.

In a preferred embodiment, the cell comprises an allele that induces the phenotype of interest. Clearly, such an allele may be characterized (e.g., in the case of an induced genetic mutation) or uncharacterized (e.g., in the case of some of the cells described *supra.* In accordance with this embodiment, the allele may induce the phenotype itself or, alternatively, render a cell resistant or sensitive to a compound. Preferably, the allele induces the phenotype itself (i.e., in the absence of a compound).

### Methods for producing a cell, tissue or non-human organism comprising the allele that induces the phenotype

### Spontaneous mutation

In one embodiment of the invention, the phenotype is caused by or associated with an induced spontaneous mutation. As used herein, the term "induced spontaneous mutation" shall be taken to mean a random mutation is produced in the genome of an organism, using, for example a mutagenic substance, e.g. N-ethyl-N-nitrosourea (ENU) or ethylmethanesulphonate (EMS).

In one embodiment, such a mutation is associated with reduced or prevented growth and/or death of a cell/tissue or non-human organism in which the induced spontaneous mutation occurs. Accordingly, the induced spontaneous mutation is the allele that induces the phenotype of interest. Such a cell is useful for, for example, a rescue screen.

However, the mutation may be responsible for any phenotype of interest, e.g., transformation of a cell, expression of a gene of interest, differentiation of a cell, dedifferentiation of a cell, sensitivity of a cell to a compound or an environment, resistance of a cell to a compound amongst others.

For example, EMS mutagenesis is used to produce mutations of interest in cells and/or animals, and those cells or animals with a phenotype of interest are selected. Without determining the causative mutation the method of the present invention is used to determine a peptide capable of modulating the induced phenotype of interest.

Methods for inducing a mutation using a mutagenic substance will be apparent to the skilled person. For example, a cell, e.g., a stem cell or any other cell of interest is incubated in a sufficient amount of a mutagen, such as, for example, EMS or ENU to induce a desired level of mutation without killing the cell. Suitable methods for inducing a mutation in a cell *in vitro* using a mutagen, such as, for example, EMS or ENU are described, for example, in Browning et al., Genomics, 73: 291-298, 2001; Stopper and Lutz, Mutagenesis, 17: 177-181, 2002; or Lee et al., J. Mol. Biol. 223: 617-626, 1992.

In the case of, for example, an ES cell this cell may be used to produce an animal that comprises a suitable mutation. Alternatively, a suitable mutation may be produced in a non-human animal using a method known in the art. For example, non-human animals are injected with a suitable dose of a mutagen to induce mutation in the reproductive cells of the animal (usually sperm cells). Following sufficient time for spermatogenesis to commence, non-human animals are bred to produce the first generation of mutant animals. Non-human animals may then be screened to identify those with a suitable phenotype for use in the method of the invention. Such a non-human animal is then useful for performance of the method of the invention, or, alternatively, a suitable cell or tissue may be isolated from the non-human animal to perform the screening process. Suitable methods for producing mutagenized animals are described, for example, in Wu et al., J. Clin. Invest. 113: 434-440, 2004 or Zan et al., Nature Biotechnol., 21: 645-651, 2003.

Accordingly, in one embodiment, the cell, tissue or non-human organism with the phenotype of interest is produced by contacting or introducing into a cell, tissue or non-human organism a mutagenic compound for a time and under conditions sufficient to induce a mutation and selecting a cell with the phenotype of interest. In the case of a non-human animal, the non-human animal may be bred prior to selecting a non-human animal with a phenotype of interest.

Clearly, the present invention additionally contemplates the use of non-human animals and/or cells that have a phenotype suitable for the screening of the present invention. For example, the phenotype of such a cell or non-human animal may have been induced by a spontaneous mutation. Such non-human animals and/or cells are available from, for example, Jackson Laboratories, Bar Harbor, ME, USA or ATCC, Manassas, VA, USA. Alternatively, or in addition, the mutation or phenotype may be induced, for example, by gene trapping. Cells and/or animals having a phenotype induced by gene trapping are available from, for example, Baygenomics at University of California Davis Mutant Mouse Regional Resource Center, CA, USA.

### Genetic modification

In a preferred embodiment, the mutation is produced or induced in a cell, tissue or non-human animal by genetic modification. Accordingly, the method of the present invention may additionally comprise providing or producing a cell, tissue or non-human animal expressing the phenotype to be modulated. Mutations or alterations to the genome or genetic makeup of a cell, tissue or non-human organism include, for example, expression of a heterologous protein in the cell, tissue or non-human organism. For example, as exemplified herein by overexpressing human Aurora-A kinase in yeast cells, cell death is induced. A peptide capable of modulating this phenotype is then selected by screening for a yeast cell that expresses Aurora-A kinase and survives. Such a peptide is of particular interest, as Aurora-A kinase overexpression is associated with various forms of cancer in humans.

Accordingly, in a preferred embodiment, the phenotype of interest (e.g., cell death) is caused by or induced by expression of a heterologous peptide, polypeptide or protein that induces phenotype (e.g., the cell, tissue or non-human organism to die).

Methods for producing a cell, tissue or non-human animal that expresses a protein of interest will be apparent to the skilled person and/or described herein and/or, described, for example, in Ausubel et al (In: Current Protocols in Molecular Biology. Wiley Interscience, ISBN 047 150338, 1987); (Sambrook et al (In: Molecular Cloning: Molecular Cloning: A Laboratory Manual, Cold Spring Harbor Laboratories, New York, Third Edition 2001); or Hogan et al (In: Manipulating the Mouse Embryo. A Laboratory Manual, 2nd Edition or Porteus et al., Mol. Cell. Biol., 23: 3558-3565, 2003.

For example, a nucleic acid that encodes a polypeptide that induces a phenotype of interest is operably linked to a promoter that is operative in a cell of interest, e.g., in an expression construct or vector. Preferably, the promoter is an inducible promoter so as to enable the induction of the phenotype (e.g., cell death or reduced or prevented cell growth) at a desirable stage, e.g., following introduction or expression of a candidate peptide. The expression construct is then introduced into a cell or non-human animal using a method known in the art and/or described herein.

Clearly, the protein of interest need not necessarily be overexpressed in yeast cells. The present invention encompasses such overexpression in any cell, tissue or non-human organism. For example, methods for producing mammalian cells overexpressing a protein of interest are known in the art, and/or described herein.

The present invention also encompasses overexpression of an endogenous protein in a cell, tissue or non-human animal. Such overexpression may be induced, for example, by introducing multiple copies of the gene or a minigene or expression construct that encodes the protein of interest into the cell, tissue or non-human animal using a method known in the art and/or described herein.

In a preferred embodiment, the introduced nucleic acid (or allele) induces death of a cell, tissue or non-human organism in which it is expressed. Preferably, cells used for the screening method of the invention are genetically modified to induce increased or enhanced cell death compared to an unmodified cell.

In one embodiment, the increased cell death is induced by increased expression of Aurora-A kinase. Preferably, the Aurora-A kinase is overexpressed in a yeast cell. In accordance with this embodiment, the cell is selected or obtained by, for example, overexpressing human Aurora-A kinase using a method known in the art and/or described herein. As used herein, the term "Aurora-A kinase" shall be taken to mean a polypeptide comprising an amino acid sequence at least about 80% identical to the sequence set forth in SEQ ID NO: 38. More preferably, the degree of sequence identity is at least about 85% to 90%, more preferably, at least about 90% to 95% and even more preferably, 99%. In a particularly preferred embodiment, the Aurora-A kinase is human Aurora-A kinase. Accordingly, the allele with which the phenotype of interest is associated is the Aurora-A kinase.

In another embodiment, an Aurora-A kinase is encoded by a nucleic acid comprising a nucleotide sequence at least about 80% identical to the sequence set forth in SEQ ID NO: 37. More preferably, the degree of sequence identity is at least about 85% to 90%, more preferably, at least about 90% to 95% and even more preferably, 99%. In a particularly preferred embodiment, the Aurora-A kinase is human Aurora-A kinase.

Accordingly, one embodiment of the present invention provides a method for identifying a peptide capable of inhibiting cell death induced by expression of Aurora-A kinase in a yeast cell, said method comprising:
(i) obtaining or producing a yeast cell capable of overexpressing Aurora-A kinase;
(ii) expressing in the cell or introducing into the cell or contacting the cell with a candidate peptide that mimics the structure of a domain or subdomain of a protein;
(iii) selecting a cell capable of growing at (ii); and
(iv) identifying the expressed or introduced peptide that inhibits cell death, wherein the peptide does not inhibit death of the cell in its native environment.

In another preferred embodiment, the increased cell death is induced by overexpressing cyclin E in a cell. Preferably, the cell is a yeast cell. In accordance with this embodiment, a cell overexpressing cyclin E is obtained by genetically modifying the cell by, for example, the introduction of an expression construct that encodes cyclin E. As used herein, the term "cyclin E" shall be taken to mean a polypeptide comprising an amino acid sequence at least about 80% identical to the sequence set forth in SEQ ID NO: 40. More preferably, the degree of sequence identity is at least about 85% to 90%, more preferably, at least about 90% to 95% and even more preferably, 99%. In a particularly preferred embodiment, the cyclin E is human cyclin E. Accordingly, the allele with which the phenotype of interest is associated is the cyclin E.

In another embodiment, a cyclin E is encoded by a nucleic acid comprising a nucleotide sequence at least about 80% identical to the sequence set forth in SEQ ID NO: 39. More preferably, the degree of sequence identity is at least about 85% to 90%, more preferably, at least about 90% to 95% and even more preferably, 99%. In a particularly preferred embodiment, the cyclin E is human cyclin E.

Accordingly, in another embodiment the present invention provides a method for identifying a peptide capable of inhibiting cell death induced by expression of Aurora-A kinase in a yeast cell, said method comprising:
(i) obtaining or producing a yeast cell capable of overexpressing Aurora-A kinase;
(ii) expressing in the cell or introducing into the cell or contacting the cell with a candidate peptide that mimics the structure of a domain or subdomain of a protein;
(iii) selecting a cell capable of growing at (ii); and
(iv) identifying the expressed or introduced peptide that inhibits cell death, wherein the peptide does not inhibit death of the cell in its native environment.

Preferably, a cell that overexpresses cyclin E also expresses cyclin dependent kinase 2 (CDK2). As used herein the term "cyclin dependent kinase 2" or "CDK2" shall be taken to mean a polypeptide comprising an amino acid sequence at least about 80% identical to the sequence set forth in SEQ ID NO: 42. More preferably, the degree of sequence identity is at least about 85% to 90%, more preferably, at least about 90% to 95% and even more preferably, 99%. In a particularly preferred embodiment, the CDK2 is human CDK2.

In another embodiment, a CDK2 is encoded by a nucleic acid comprising a nucleotide sequence at least about 80% identical to the sequence set forth in SEQ ID NO: 41. More preferably, the degree of sequence identity is at least about 85% to 90%, more preferably, at least about 90% to 95% and even more preferably, 99%.

In a particularly preferred embodiment, the cell, tissue or non-human organism is genetically modified to express both cyclin E and CDK2.

In methods of the present disclosure, reduced or prevented cell growth and/or cell death can be induced by silencing expression of a gene. Such a method is useful, for example, for determining a peptide that complements or rescues a phenotype (e.g., a disease) that is characterized by reduced or prevented gene expression. Such gene silencing may be induced using, for example "knock-out" technology, for example, as described in Hogan et al (In: Manipulating the Mouse Embryo. A Laboratory Manual, 2nd Edition or Porteus et al., Mol. Cell. Biol., 23: 3558-3565, 2003.

For example, a cell or animal in which a gene of interest is knocked-out is produced using a replacement vector. This form of construct contains two regions of homology to the target gene located on either side of a heterologous nucleic acid (for example, encoding one or more positive selectable markers, such as, for example, a fluorescent protein (e.g. enhanced green fluorescent protein), β-galactosidase, an antibiotic resistance protein (e.g. neomycin resistance or zeocin resistance) or a fusion protein (e.g. β-galactosidase - neomycin resistance protein, β-geo). The vector is introduced into a cell of interest and the vector homologlously recombines with the target gene.

Homologous recombination proceeds by at least two recombination events (or a double cross-over event) that leads to the replacement of target-gene sequences with the replacement-construct sequences. More specifically, each region of homology in the vector induces at least one recombination event that leads to the heterologous nucleic acid in the vector replacing the nucleic acid located between the regions of homology in the target gene.

Alternative methods for knocking out a gene of interest will be apparent to the skilled person, for example, using recombination (e.g., recombination of nucleic acid located between two LoxP sites using the enzyme Cre).

Alternatively, gene silencing may be induced using, for example, RNA interference (Hannon and Conklin, Methods Mol. Biol. 257: 255-266, 2004), antisense, ribozymes (e.g. Bartel and Szostak, Science 261, 1411-1418, 1993), nucleic acid capable of forming a triple helix (e.g. Helene, Anticancer Drug Res. 6, 569-584, 1991) or PNAs (Hyrup et al., Bioorganic & Med. Chem. 4, 5-23, 1996; O'Keefe et al., Proc. Natl Acad. Sci. USA 93, 14670-14675, 1996).

### Induction of a phenotype using a compound or biological molecule

In another embodiment, the phenotype of interest is induced by contact a cell, tissue or non-human organism with a compound or administering to a non-human organism a compound that induces the phenotype of interest. Clearly, the use of any compound that induces a phenotype of interest is encompassed by the present invention.

For example, a peptide that modulates the response of a cell, tissue or non-human organism to oxidative stress is determined, for example, by contacting a cell expressing or comprising a candidate peptide with, for example, hydrogen peroxide or a superoxide dismutase inhibitor, such as, for example, diethylthiocarbamate.

Alternatively, a peptide that induces cell division is determined, for example, by contacting a cell with a cell cycle inhibitor, such as, for example, a purine derivative, e.g., Roscovitine. Cell cycle inhibition may also be induced, for example, by exposing a cell to ultraviolet radiation.

Alternatively, a peptide that protects a cell against transformation is determined by contacting a cell expressing a peptide with a compound that induces transformation. For example, Miller et al., Environmental Health Perspectives 106: 465-471, 1998 describe the transformation of osteoblasts to a tumorigenic state using depleted uranium-uranyl chloride.

The present invention is also useful, for example, for determining a peptide that prevents infection of a cell, tissue or organism, for example, by a virus. For example, a cell expressing a peptide of the invention is contacted with a virus (e.g., HCV or HIV) and the level viral infection and/or growth and/or the production of viral proteins by the cell is determined.

### Determining a peptide that modulates an allele that determines the phenotype of interest

In some methods of the present disclosure, a peptide identified by the method of the present invention enhances the phenotype of interest (or enhances the level of a phenotype of interest).

In one embodiment, a peptide identified by the method of the present invention reduces or suppresses the phenotype of interest (or suppresses the level of a phenotype of interest).

In the case of a phenotype that is conferred or induced by an allele, a peptide that modulates the phenotype of interest may do so by directly interacting with the allele that determines the phenotype of interest, or interacts with the an expression product associated with said allele. For example, the peptide may directly modulate expression of a gene of interest. Alternatively, the peptide interacts with a mutant protein that determines the phenotype of interest and inhibits the activity of the protein that confers the phenotype.

In another embodiment, a peptide that modulates the phenotype of interest induced by an allele does not directly interact with the allele that determines the phenotype of interest or an expression product directly associated with said allele. Without being bound by a mode of action, such a peptide may, for example, enhance or suppress the expression or activity of a protein that interacts with the allele or an expression product thereof, or, alternatively, modulate the level of expression or activity of a protein or a number of proteins that are "downstream" of the allele or an expression product thereof. By "downstream" is meant, for example, a cellular component that is a component of a signaling cascade that is modulated by virtue of the activity of the allele or an expression product thereof. Clearly, a peptide may also activate or suppress a protein or a number of proteins that do not interact with the allele or an expression product thereof or a protein downstream of the allele or an expression product thereof, yet is capable of modulating the phenotype of interest. By way of example, cell death is mediated by several pathways, with apoptosis having several different pathways and necrotic cell death also being another pathway. A cell that has blocked, for example, the apoptotic pathway (e.g., a tumor cell that has enhanced expression of bcl-2) may be killed, or induced to die by a peptide that activates the necrotic cell death pathway (e.g., by activating the RIP-FADD necrotic pathway, e.g., by activating RIP).

In a preferred embodiment, a peptide of the present invention is capable of complementing a phenotype in a cell, tissue or organism. Complementation is to be understood to include the modulation of a phenotype of a cell, tissue or animal (wherein the phenotype of the cell is not a wild-type phenotype) such that the phenotype returns to or becomes the same as a wild-type cell, tissue or animal. For example, the present inventors have identified a peptide that is capable of complementing the cell-death phenotype of a cell overexpressing Aurora-A kinase. Accordingly, the peptide is capable of suppressing the cell death induced by overexpression Aurora-A kinase and enable the cell to grow in a manner similar to a cell that does not express Aurora-A kinase (i.e., a wild-type cell).

The term "complementation" or "complement" or grammatical equivalent shall also be understood to encompass a peptide that rescues a cell from a phenotype.

As will be apparent to the skilled person a method or assay for determining a change in a phenotype will depend upon the phenotype that is being modulated. Such an assay will be apparent to the skilled artisan.

### Rescue assays - cell survival and/or growth

### Cell survival

In methods of the invention, the phenotype being assayed is cell death. Accordingly, in the absence of a modulatory peptide of interest a cell is induced to die. As will be apparent to the skilled artisan, it is preferable that to assay a peptide of the present invention in such circumstances, the phenotype of interest is inducible. Accordingly, the peptide of the present invention is expressed or introduced into the cell, tissue or non-human animal prior to expression of the phenotype. A phenotype may be induced using, for example, an inducible promoter to control expression of a gene that causes or is associated with the phenotype. Inducible promoters or enhancer/suppressor elements are known to those skilled in the art and/or described herein. Alternatively, the phenotype is induced, for example, by contact a cell with a toxic compound

In one embodiment, a peptide that is capable of modulating the level of cell death in a cell is determined by cell survival in the presence of the allele that induces the cell death phenotype, For example, a cell expressing a peptide of the present invention (or preferably, a plurality of cells each expressing a peptide of the present invention) are grown under conditions sufficient for expression of the phenotype of interest (e.g., cell death). Any cell that survives and preferably grows is considered to express a peptide capable of modulating (in this case, suppressing) the phenotype. Preferably, the cells are grown under conditions sufficient for observation of cell growth.

For example, the present inventors have overexpressed either Aurora-A kinase or cyclin E in yeast cells. As this overexpression is toxic to yeast cells, the expression of the Aurora-A kinase gene or cyclin E gene is placed under control of an inducible promoter. Cells are transformed with an expression construct that encodes a peptide that mimics the structure of a protein domain and grown for a time and under conditions sufficient for expression of said peptide. Following expression of the peptide, the expression of Aurora-A kinase or cyclin E is induced. Those cells that express a peptide capable of modulating the cell death phenotype induced by expression of Aurora-A kinase or cyclin E, and preferably suppress the phenotype, are identified by growing the cells for a time and under conditions for colonies to form. Nucleic acid encoding a peptide that modulates/rescues/complements the cell death phenotype are then isolated from the yeast cells and identified using, for example, sequencing.

Accordingly, cell survival may simply be detected by maintaining the cells for a sufficient time for a visible colony of cells to form. Clearly, this provides a simple method for high-throughput screening of peptides as peptides capable of inducing cell survival are easily recovered from the colony of cells.

Other methods for assessing cell survival will be apparent to the skilled artisan, for example, a cell growth and/or proliferation assay described herein.

Methods of the present disclosure may be performed *in vivo*. Clearly, such an assay may be performed in any model organism, such as, for example, a mouse, a rat, a sheep, a monkey, a fish, a fly or a nematode. However, larger model organisms are usually preferred for confirming the ability of a peptide of interest to modulate a phenotype.

High throughput methods of screening a compound *in vivo*, for example, in a zebrafish, are described, for example, in International application No. PCT/GB2003/005239. In adapting the methods described therein to the current invention a zebrafish is genetically modified to express a protein that is lethal to the fish. For example, the protein is under control of an inducible promoter or a life stage specific promoter or causes progressive degeneration. As zebrafish are relatively small they may be maintained in a 96 well format plate. Peptides (e.g., conjugated to a protein transduction domain) are introduced to each well of the plate (e.g., individually or in pools) and the survival of the fish is determined. In the case of an inducible promoter the inducer of expression (e.g., Tet) may be added to or removed from each well of the plate following introduction of the peptide. Clearly, such methods allow for relatively high-throughput *in vivo* screening of peptides.

Similar methods using, for example, nematodes or *Drosophila* will be apparent to the skilled artisan.

In another embodiment, cell death is assayed using a method for the detection of cellular components associated with cell death, such as, for example apoptosis. Such an assay is useful, for example, for rapid screening mammalian cells transfected or transduced with an expression construct expressing a peptide that mimics a protein domain that is capable of suppressing or enhancing cell death. This is because, mammalian cells grow at a reduced rate compared to, for example yeast cells.

Methods for detecting cell death in a cell are known in the art. For example, APOPTEST (available from Immunotech) stains cells early in apoptosis, and does not require fixation of the cell sample (Martin *et al*., 1994). This method utilizes an annexin V antibody to detect cell membrane re-configuration that is characteristic of cells undergoing apoptosis. Apoptotic cells stained in this manner can then sorted either by fluorescence activated cell sorting (FACS), ELISA or by adhesion and panning using immobilized annexin V antibodies.

Alternatively, a terminal deoxynucleotidyl transferase-mediated biotinylated UTP nick end-labeling (TUNEL) assay is used to determine the level of cell death. The TUNEL assay uses the enzyme terminal deoxynucleotidyl transferase to label 3'-OH DNA ends, generated during apoptosis, with biotinylated nucleotides. The biotinylated nucleotides are then detected by using streptavidin conjugated to a detectable marker. Kits for TUNEL staining are available from, for example, Intergen Company, Purchase, NY.

Alternatively, or in addition, an activated caspase, such as, for example, Caspase 3 is detected. Several caspases are effectors of apoptosis and, as a consequence, are only activated to significant levels in a cell undergoing programmed cell death. Kits for detection of an activated caspase are available from, for example, Promega Corporation, Madison WI, USA. Such assays are useful for both immunocytochemical or flow cytometric analysis of cell death.

In the case of assays in which the cell is fixed or killed, it is preferred that a record of which peptide or nucleic acid was introduced into or expressed is maintained to facilitate rapid identification of peptides that rescue a cell from cell death.

### Cell growth/proliferation

In some methods of the present disclosure, the phenotype of interest is cell survival and/or growth. For example, the present inventors have assayed a library of peptides using cytokine-dependent cell lines to determine those peptides capable of overcoming the cytokine dependence of these cells. Upon growth factor withdrawal, the cytokine dependent cells stop growing and eventually die. By transfecting cells with a library of peptides of the present invention and then withdrawing the relevant cytokine a peptide capable of overcoming the cytokine dependence by growing the cells for a sufficient time for a colony of clonal cells (each expressing the same peptide) to develop. Following growth of the cells, nucleic acid encoding the peptide that modulated the cytokine dependent phenotype was isolated and characterized using sequencing.

Again, maintaining cells for a time and under conditions sufficient for cells to grow and proliferate sufficiently to produce a visible colony is perhaps the simplest assay to determine a modulatory peptide.

As an alternative to growing cells for a time sufficient for growth of a detectable colony of cells, a cell viability or cell metabolism assay may be detected and/or assayed. By way of example, non-fluorescent resazurin is added to cells cultured in the presence of a peptide of the present invention. Viable cells reduce resazurin to red-fluorescent resorufin, easily detectable, using, for example microscopy or a fluorescent plate reader. This marker of cell viability is useful for a variety of different cell types, from bacteria to higher eukaryotes. Kits for analysis of cell viability are available, for example, from Molecular Probes, Eugene, OR, USA.

Other assays for cell viability include for example, assays that detect WST-8 reduction to formazan salt in live cells (Alexis Biochemicals), staining of live cells with cell-permeable calcein acetoxymethyl (calcein AM) which is converted to fluorescent calcein by intracellular esterases, detection of XTT reduction to formazan salt (Intergen), MTS reduction to formazan salt (Promega Corporation).

Yeast cell plasma membrane integrity and metabolic function are required to convert the yellow-green-fluorescent intracellular staining of FUN 1 into red-orange-fluorescent intravacuolar structures. An assay for the detection of viable yeast cells based on this compound is available from Molecular Probes (Eugene, OR, USA).

In yet another method of the present disclosure, the phenotype of interest is cellular proliferation. Methods for determining cellular proliferation are known in the art.

For example, incorporation of ³H-thymidine or ¹⁴C-thymidine into DNA as it is synthesized is an assay for DNA synthesis associated with cell division. In such an assay, a cell is incubated in the presence of labeled thymidine for a time sufficient for cell division to occur. Following washing to remove any unincorporated thymidine, the label (e.g. the radioactive label) is detected, e.g., using a scintilation counter. Assays for the detection of thymidine incorporation into a live cell are available from, for example, Amersham Pharmacia Biotech.

Cellular proliferation can be measured using a MTT assay. The yellow tetrazolium MTT (3-(4, 5- dimethylthiazolyl-2)-2, 5-diphenyltetrazolium bromide) is reduced by metabolically active cells, in part by the action of dehydrogenase enzymes, to generate reducing equivalents such as NADH and NADPH. The resulting intracellular purple formazan is then solubilized and quantified by spectrophotometric means. Assay kits for MTT assays are available from, for example, American Type Culture Collection.

Alternative assays for determining cellular proliferation, include, for example, measurement of DNA synthesis by BrdU incorporation (by ELISA or immunohistochemistry, kits available from Amersham Pharacia Biotech), expression of proliferating cell nuclear antigen (PCNA) (by ELISA, FACS or immunohistochemistry, kits available from Oncogen Research Products) or a Hoechst cell proliferation assay that detects DNA synthesis (available from Trevigen Inc.).

Alternatively, the growth rate of the cell is determined, for example, manually, by, for example observing or measuring the size of a colony of cells over a period of time or, alternatively or in addition counting the number of cells over a period of time.

Clearly, cell proliferation changes are also useful, for example, for determining a peptide that suppresses proliferation (e.g., of a cancer cell).

### Gene expression changes

In one method of the present disclosure, the phenotype of interest is the modulation of expression of one or more genes. Detecting a change in expression of a gene by detecting encoded nucleic acid, e.g., RNA, mRNA or cDNA derived therefrom are known in the art and described, for example, in Ausubel et al (In: Current Protocols in Molecular Biology. Wiley Interscience, ISBN 047 150338, 1987)and (Sambrook et al (In: Molecular Cloning: Molecular Cloning: A Laboratory Manual, Cold Spring Harbor Laboratories, New York, Third Edition 2001).

For example, the level of expression of a nucleic acid is detectable using Northern blotting (described in Ausubel et al (In: Current Protocols in Molecular Biology. Wiley Interscience, ISBN 047 150338, 1987) and Sambrook et al (In: Molecular Cloning: Molecular Cloning: A Laboratory Manual, Cold Spring Harbor Laboratories, New York, Third Edition 2001)). Essentially this method comprises immobilizing nucleic acid (RNA or mRNA) on a solid support, such as, for example, a membrane. A probe or primer that hybridizes to the nucleic acid of interest that is labeled with a detectable marker (such as, for example, a fluorescent label or a radioactive label) is then brought into direct contact with the membrane for a time and under conditions sufficient for hybridization to occur (preferably, under moderate and more preferably high stringency conditions). Following washing to remove any non-specifically bound probe, the detectable marker is detected. Methods of detection will vary with the detectable marker used, but include, for example, densitometry. Using a control, such as, for example GAPDH or actin, a normalized level of expression of the nucleic acid of interest is determined.

The level of expression of a nucleic acid can be determined using an amplification reaction, such as, for example, quantitative RT-PCR, for example using "kinetic analysis" described in Higuchi et al., BioTechnology 10, 413-17, 1992, and Higuchi et al., BioTechnology 11, 1026-30, 1993. The principle of this form of analysis is that at any given cycle within the exponential phase of PCR, the amount of product is proportional to the initial number of template copies.

Methods of Quantitative PCR often rely upon an internal standard that is not modulated by the experimental procedures. For example, a mRNA the expression of which is not modulated be a peptide of the present invention. Such mRNA include, for example, 18S ribosomal subunit, GAPDH or actin.

Alternatively, quantitative PCR is performed in the presence of an internally quenched fluorescent oligonucleotide (TaqMan probe) complementary to the target sequence, the probe is cleaved by the 5'-3' endonuclease activity of Taq DNA polymerase and a fluorescent dye released in the medium (Holland et al., Proc. Natl. Acad. Sci. U.S.A. 88, 7276-80, 1991). As the fluorescence emission increases in direct proportion to the amount of the specific amplified product, the exponential growth phase of PCR product can be detected and used to determine the initial template concentration (Heid et al., Genome Res. 6, 986-94, 1996, and Gibson et al., Genome Res. 6, 995-1001, 1996).

The level of expression of a nucleic acid can be determined using, for example, microchip or a chip. In such an assay a series of oligonucleotide probes or short nucleic acid probes that hybridize to specific nucleic acid in a sample (e.g. mRNA) are affixed to a solid support. A biological sample of interest (preferably, comprising nucleic acid such as, for example, mRNA, cDNA or cRNA) is then contacted with the DNA "chip" and hybridization is detected. Preferably, the sample nucleic acid is labeled with a detectable marker to facilitate detection of hybridization. Methods used in the generation and screening of DNA arrays are known in the art and are described in for example, Schena (In: Microarray Analysis, John Wiley and Sons, ISBN: 0471414433, 2002).

One form of chip assay is a GeneChip assay(Affymetrix, Santa Clara, Calif.; described, for example, in U.S. Patent Nos. 6,045,996; 5,925,525; and 5,858,659). The GeneChip technology uses miniaturized, high-density arrays of oligonucleotide probes affixed to a "chip." Probe arrays are manufactured by Affymetrix's light-directed chemical synthesis process, which combines solid-phase chemical synthesis with photolithographic fabrication techniques employed in the semiconductor industry. Using a series of photolithographic masks to define chip exposure sites, followed by specific chemical synthesis steps, the process constructs high-density arrays of oligonucleotides, with each probe in a predefined position in the array. These arrays are then useful for the detection of a expression of a large number of nucleic acids. Accordingly, such an array is useful for determining a gene expression profile in response to a peptide of the present invention.

The level of expression of a peptide, polypeptide or protein can be determined in a cell, tissue or organ in response to a peptide of the present invention. Accordingly, the level of expression of the peptide, polypeptide or protein is the phenotype to be modulated.

The level of a polypeptide in a sample can be determined using an enzyme-linked immunosorbent assay (ELISA) or a fluorescence linked immunosorbent assay (FLISA). Methods of performing an ELISA or a FLISA , e.g., a direct ELISA, an indirect ELISA, a capture ELISA or FLISA, a sandwich ELISA or FLISA or a competitive ELISA are known in the art and/or described, for example, in Scopes (*In*: Protein Purification: Principles and Practice, Third Edition, Springer Verlag, 1994). For example, an antibody or ligand that specifically binds to a polypeptide of interest is adsorbed or conjugated to a solid support, such as, for example, a polycarbonate or polystyrene microtitre plate. A biological sample is then brought into direct contact with the antibody or ligand under conditions to allow binding of the polypeptide in the sample by the antibody or ligand. Following washing, a suitable, labeled secondary antibody is added to the plate. For example, a suitable secondary antibody binds to the polypeptide of interest at a different epitope to that bound by the first antibody. The secondary antibody is labeled with, for example, a fluorescent label (e.g., a Texas Red label, FITC or a fluorescent semiconductor nanocrystal (as described in US 6,306,610)) in the case of a FLISA or an enzymatic label (e.g. horseradish peroxidase or alkaline phosphatase) in the case of an ELISA. Alternatively, the secondary antibody may be labeled with a marker, such as, for example, biotin. The secondary antibody is then detected with a labeled tertiary antibody or molecule, for example, streptavidin. The amount of label that is subsequently detected is indicative of the amount of the polypeptide of interest in the biological sample.

Alternatively, the amount of a polypeptide of interest in a sample may be determined using a biosensor or optical immunosensor system. In general an optical biosensor is a device that uses optical principles quantitatively to convert the binding of a ligand or antibody to a target polypeptide into electrical signals. These systems can be grouped into four major categories: reflection techniques; surface plasmon resonance; fibre optic techniques and integrated optic devices. Reflection techniques include ellipsometry, multiple integral reflection spectroscopy, and fluorescent capillary fill devices. Fibre-optic techniques include evanescent field fluorescence, optical fibre capillary tube, and fibre optic fluorescence sensors. Integrated optic devices include planer evanescent field fluorescence, input grading coupler immunosensor, Mach-Zehnder interferometer, Hartman interferometer and difference interfermoter sensors. Fluorescence fluctuation anisotropy is an example of a technique applicable to the analysis of peptide/protein interactions in solution. These examples of optical immunosensors are described in general by G. A. Robins (Advances in Biosensors), Vol. 1, pp. 229-256, 1991. More specific description of these devices are found for example in U.S. Pat. Nos. 4,810,658; 4,978,503; 5,186,897; R. A. Brady et al. Phil. Trans. R. Soc. Land. B 316: 143-160, 1987 and G. A. Robinson et al. (in Sensors and Actuators, Elsevier, 1992).

For example, surface plasmon resonance is used to detect the amount of a protein of interest in a sample. Surface plasmon resonance detects changes in the refractive index of a solution close to the surface of a sensor device, or a chip. A surface plasmon resonance sensor comprises of a transparent material having a metal layer deposited thereon. An antibody or ligand capable of specifically binding the polypeptide is immobilized on the metal surface layer of the sensor. A light source generates polarized light that is directed through a prism, or diffraction grating, striking the metal layer-transparent material interface. A detector detects light reflected from the metal surface. A biological sample is then brought into direct contact with the sensor, e.g. by injection in a controlled flow over the surface containing the bound antibody. Any change in the surface concentrations resulting from an interaction between the antibody or ligand and the polypeptide is spectroscopically detected as a surface plasmon resonance signal by the shifting of relative reflective intensity signals. As more of the polypeptide is bound by the antibody or ligand the degree of change of the reflective intensity signals increases. Accordingly, such assays are quantitated by determining the degree of change in the reflective intensity signal in a test sample relative to a control sample, such as, for example, a sample comprising a known amount of the polypeptide of interest.

Preferably, the sensor detects the enthalpic heat released upon binding of an antibody to the target molecule. Such 'isothermal calorimetry' methods are known for identifying or characterizing interactions.

The amount of one or more proteins in a sample can be determined using a protein chip. To produce protein chips, the proteins, peptides, polypeptides, antibodies or ligands that are able to bind specific antibodies or proteins of interest are bound to a solid support such as for example glass, polycarbonate, polytetrafluoroethylene, polystyrene, silicon oxide, metal or silicon nitride. This immobilization is either direct (e.g. by covalent linkage, such as, for example, Schiff's base formation, disulfide linkage, or amide or urea bond formation) or indirect. Methods of generating a protein chip are known in the art and are described in for example U.S. Patent Application No. 20020136821, 20020192654, 20020102617 and U.S. Patent No. 6,391,625. To bind a protein to a solid support it is often necessary to treat the solid support so as to create chemically reactive groups on the surface, such as, for example, with an aldehyde-containing silane reagent. Alternatively, an antibody or ligand may be captured on a microfabricated polyacrylamide gel pad and accelerated into the gel using microelectrophoresis as described in, Arenkov et al. Anal. Biochem. 278:123-131, 2000.

Preferably, a protein sample to be analyzed using a protein chip is attached to a reporter molecule, such as, for example, a fluorescent molecule, a radioactive molecule, an enzyme, or an antibody that is detectable using methods well known in the art. Accordingly, by contacting a protein chip with a labeled sample and subsequent washing to remove any unbound proteins the presence of a bound protein is detected using methods well known in the art, such as, for example, using a DNA microarray reader.

Alternatively, the amount of a protein of interest bound to a protein chip is detected using a labeled secondary or even tertiary antibody or ligand.

Alternatively, biomolecular interaction analysis-mass spectrometry (BIA-MS) is used to rapidly detect and characterize a protein present in complex biological samples at the low- to sub-fmole level (Nelson et al. Electrophoresis 21: 1155-1163, 2000). One technique useful in the analysis of a protein chip is surface enhanced laser desorption/ionization-time of flight-mass spectrometry (SELDI-TOF-MS) technology to characterize a protein bound to the protein chip. Alternatively, the protein chip is analyzed using ESI as described in U.S. Patent Application 20020139751.

### Detection of cell signaling

In another method of the present disclosure, the phenotype of interest is activation of a signal transduction pathway, for example TNFα activation of the NFκB signaling pathway, or activation of the activator protein 1 (AP-1 pathway) or any other signal transduction pathway.

To identify a peptide useful for, for example, inhibiting TNFα activation of the NFκB signaling pathway an expression vector encoding a detectable marker the expression of which is operably under control of a promoter controlled by a NFκB response element. Accordingly, the detectable marker is only expressed in the presence of NFκB. Cells are administered with a peptide that mimics the structure of a protein domain or transfected with an expression construct encoding same and then treated with TNFα. The level of the detectable marker is then determined in the cell with the peptide compared to a cell that does not comprise the peptide. Using such a system a peptide is determined that inhibits or reduces TNFα activation of the NFκB signaling pathway (or conversely enhances TNFα activation of the NFκB signaling pathway). Suitable detectable markers include, for example luciferase, a fluorescent protein (e.g., green fluorescent protein), β-galactosidase or alkaline phosphatase.

Vectors comprising response elements for the detection of signal transduction activation (e.g., a nuclear factor κB (NFκB) response element, a cyclic AMP response element (CRE), a serum response element (SRE), an activator protein 1 (AP-1) response element or a serum response factor (SRF) response element) in operable connection with a reporter gene (i.e., encoding a detectable marker) are available from commercial sources, such as, for example Clontech or Stratagene, or alternatively may be produced using methods known in the art.

Alternative assays for monitoring the level of signal transduction include, for example, a cyclic AMP activation assay, e.g., a cAMP enzyme linked immunosorbent assay that directly measures cyclic AMP in a cell (e.g. as available from Amersham Pharmacia).

Alternatively, an assay to detect the activity of a protein kinase, an assay to determine the activity of a kinase may be used to assess the effect of a peptide on the activity of a kinase. For example, The Mercury *In Vivo* Kinase Assay Kits (Clontech) are useful for assessing specific signal transduction pathway activation *in vivo*. Cells are transfected with an expression construct encoding a peptide of the present invention and a transactivator vector (e.g., a vector encoding ELK, ATF, Jun, or CREB fused to the tetracycline repressor protein (TetR)). The cell is also transfected with a reporter vector containing a reporter gene under the control of a tetracycline-responsive element (TRE), consisting of seven repeats of the tet operator sequence and a vector that expresses a known kinase or a target gene that you want to test for kinase activity.

In the absence of doxycycline/tetracycline the Tet R fusion protein binds to the TRE in the reporter plasmid. Should the transactivation protein (i.e., the fusion protein) have been phosphorylated, the reporter gene will be activated. Accordingly, this system is useful for determining a peptide that enhances or suppresses the activity of a kinase.

Assay systems are also available, for example, for determining phosphatase activity or G-protein coupled receptor activity.

### Cell differentiation

In a still further method of the present disclosure, the phenotype of interest is differentiation of a cell. A peptide that induces differentiation of a cell is a putative therapeutic for the treatment of a cancer, as cancer cells are considered to be relatively undifferentiated or pluripotent cells (Dinnen et al., Cancer Res.53: 1027-1033). Methods for determining a cell that has differentiated include, for example, detecting a marker that is associated with a specific differentiated cell type, e.g. integrin α6, nestin, NCAM-L1, Pax6, glucagon, GLUT2, albumin, α-smooth muscle actin, bone specific alkaline phosphatase, osteonectin, CD45 or GMSCF Rα (antibodies to which are available from R&D Systems).

Alternatively, a cell cultured in the presence of a peptide of the present invention or transfected with an expression construct encoding same is monitored for loss of expression of a marker of expression of undifferentiated cells (e.g. Stage-Specific Embryonic Antigens 1 and 4 (SSEA-1 and SSEA-4) and Tumor Rejection Antigen 1-60 and 1-81 (TRA-1-60, TRA-1-81)). Alternatively, or in addition an undifferentiated cell is cultured in the presence of a peptide of the present invention or transfected with an expression construct encoding same and is monitored for the formation of an embroid body.

### In vivo analysis

In a still further method of the present disclosure, a peptide of the present invention is assayed to determine its effect on, for example an animal model of a human disease. The peptide is administered to or expressed in a mouse that carries a mutation or has been genetically modified to mimic a human disease. Methods for producing a mouse expressing a recombinant protein are known in the art and are described, for example, in Hogan et al (In: Manipulating the Mouse Embryo. A Laboratory Manual, 2nd Edition. Cold Spring Harbour Laboratory. ISBN: 0879693843, 1994).

By comparing the phenotype observed in a mutant mouse comprising a peptide of the invention to a mutant mouse that does not comprise a peptide of the invention, a modulator of the disease phenotype is determined. Furthermore, by comparing the phenotype observed in a mutant mouse comprising a peptide of the invention to the phenotype of a wild-type mouse a peptide that complements or rescues the disease phenotype is determined. Such assays are useful, as not only do they determine a peptide that modulates the phenotype of interest, but they also provide information against activity of the peptide on, e.g., pathways other than that being studied, e.g., toxicity.

Such an assay is useful for studying a variety of human disorders, such as, for example, obesity, cancer, neurodegeneration, osteoporosis, osteopetrosis, stroke, allergy, inflammatory disease, amongst many others.

For example, a peptide is administered to or expressed in a mouse model of a human disease, e.g. a neurodegenerative disease, eg. Huntington's Disease, for example, the R6/2 model of Huntington's Disease (Mangiarini et al., Cell, 87: 493-506, 1996). The R6/2 mice show various neurological defects, such as reduced ability to maintain balance on a rotating rod, and behavioral defects such as, for example circling behavior, in addition to progressive inability to use the limbs and progressive weight loss. Following administration of a peptide of the present invention, mice are monitored for phenotypic changes compared to R6/2 mice that do not comprise a peptide of the invention and/or a wild-type mouse to determine a peptide that rescues all or one aspect of the Huntington's Disease pathology observed.

The peptide can be administered to or expressed in a rodent temporary occlusion model. For example, the rat temporary occlusion of the MCA model is used to induce transient focal ischemia. Induction of focal ischemia involves placing a monofilament nylon suture to occlude the middle cerebral artery (MCA) for 45 minutes and maintaining blood pressure at 90 mmHg, followed by reperfusion. MCA occlusion and re-establishment of blood flow is monitored, for example, using Laser Doppler. Following reperfusion, a peptide is administered to an animal to determine its ability to reduce the effect of the reperfusion injury (similar to the injury induced by a stroke). The effect of the peptide, for example, in infarct size is determined, by incubating coronal brain sections in triphenyltetrazolium chloride, which stains mitochondrial dehydrogenase activity.

Behavioural testing may also be performed to determine the effect of the peptide on preventing damage caused by a reperfusion injury, e.g., stroke. Suitable behavioral tests include, for example, paw extension, body positioning, touch response, circling behavior and/or the presence of seizures or no spontaneous movement.

### Determining a peptide with a novel activity

A peptide identified using the method of the present invention is further assayed to determine whether or not it is capable of modulating (i.e. enhancing or suppressing) phenotype in its native environment.

The known function/s of the polypeptides isolated in the method of the present invention are determined, for example, using sequence analysis software as is available from, for example NCBI, or Prosite.

As used herein the term "Prosite" shall be understood to mean the Prosite protein database which is a part of the ExPasy proteomics server provided by the Swiss Institute of Bioinformatics at CMU-Rue Michel - Servet 1 1211 Genève 4 Switzerland.

Accordingly, those polypeptides that are known to modulate or mediate the phenotype of interest in their native environment are excluded from any further analysis.

Furthermore, analysis of the bioinformatic information available, for example, at NCBI aids in determining the native function of a protein. Such analysis will determine if, for example, the pathway or phenotype being modified exists in an organism from which a peptide is identified or if a target protein or nucleic acid is found in any of the organisms used to generate an expression library.

In a preferred embodiment, nucleic acid fragments used to produce the candidate peptides of the present invention are produced from an organism that does not express the phenotype to be modulated.

Even more preferably, the nucleic acid fragments used to produce the candidate peptide are from an organism with a compact genome and the phenotype is expressed by a cell, tissue or organism having a complex genome.

As exemplified herein, the present inventors have studied the effect of overexpression of Aurora-A kinase, which produces a cancer phenotype in human cells. The nucleic acid fragments used to identify a peptide useful for modulating the activity of Aurora-A kinase were isolated form a variety of single celled microorganisms, i.e., organisms that do not suffer from cancer. Accordingly, it is unlikely that a peptide isolated in a screen using such nucleic acid fragments will modulate Aurora-A kinase in its native environment.

It is particularly preferred that an expression library is generated using nucleic acid fragments isolated from organisms that are distinct from the organism in which the phenotype naturally occurs or in which an allele that causes or is associated with the phenotype naturally occurs. For example, to identify a nucleic acid that encodes a peptide that modulates the ability of a human cell line to escape cytokine dependence, an expression library is generated from the organisms *Aeropyrum pernix, Aquifex aeolicus, Archaeoglobus fulgidis, Bacillus subtilis, Bordetella pertussis*, *Borrelia burgdorferi, Chlamydia trachomatis, Desulfobacterium autotrophicum, Escherichia coli, Haemophilus influenzae, Halobacterium salinarium, Haloferax volcanii Helicobacter pylori, Methanobacterium thermoautotrophicum, Methanococcus jannaschii, Mycoplasma pneumoniae, Neisseria meningitidis, Pirellula Species 1 (rhodopirellula baltica), Pseudomonas aeruginosa, Pyrococcus horikoshii, Synechocystis PCC 6803, Thermoplasma volcanium and Thermotoga maritima.Escherichia coli, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis PCC 6803, Thermoplasma volcanium* and *Thermotoga maritima.* This will reduce the likelihood of identifying a peptide that modulates cytokine dependence in a human cell line in its native environment.

In another embodiment, where the cellular component to which the modulatory peptide/s bind/s is known or determined (e.g., using a method described herein) a peptide is preferably selected that is not related in structure to a peptide or protein or protein domain that naturally binds to the cellular component. Accordingly, in one embodiment the method of the invention comprises:
(i) determining a of peptide that modulates a phenotype of interest;
(ii) determining a cellular component to which the peptide binds to thereby modulate the phenotype;
(iii) determining the structure of a peptide, polypeptide or protein or protein domain that binds to the cellular component in nature; and
(iv) selecting a peptide that is unrelated in structure to the peptide, polypeptide or protein or protein domain that binds to the cellular component in nature.

In one embodiment, the method comprises determining a plurality of modulatory peptides and selecting that or those peptides that is/are unrelated in structure to the peptide, polypeptide or protein or protein domain that binds to the cellular component in nature.

Methods for determining the structure of a cellular component and/or a peptide, polypeptide or protein or protein domain are known in the art. For example, the three dimensional structure of a peptide or polypeptide is determined using X-ray crystallography.

Alternatively, methods for predicting the 3 dimensional structure of a peptide are known in the art, and are described, for example, in US Patent Application No 20020150906 (California Institute of Technology), or using a computer program or algorithm, such as, for example, MODELLER, (Sali and Blundell, J. Mol. Biol. 234, 779-815, 1993). These techniques rely upon aligning the sequence of a peptide with the sequences of peptides or proteins that have a characterized structure. Such alignment algorithms are known in the art and are accessed through software packages such as, for example BLAST at NCBI. Structural information, i.e., three-dimensional structure, of a query peptide is then be predicted based upon structural information corresponding to the sequence or subsequences aligned in the proteins or peptides that have previously been characterized. This information is used to determine those sequences that is adopt a conformation sufficient for binding to a target protein or nucleic acid that is different to the structure adopted by a peptide or protein that binds to the target in nature.

In a preferred embodiment, the method of the present invention additionally comprises isolating and/or providing and/or purifying and/or synthesizing a peptide that is capable of modulating an allele and/or phenotype of interest. Methods for the isolation/production/purification and/or synthesis of a peptide are known in the art and or described herein.

In another embodiment, the method of the present invention additionally comprises isolating and/or providing and/or purifying and/or synthesizing a nucleic acid fragment that encodes a peptide that is capable of modulating an allele and/or phenotype of interest. Methods for the isolation/production/purification and/or synthesis of a nucleic acid fragment are known in the art and or described herein.

### Providing or producing a modulatory peptide or nucleic acid encoding same

One embodiment of the invention provides a method for identifying and/or obtaining a nucleic acid that encodes a peptide of the invention. This method comprises, for example:
(i) identifying a peptide that capable of modulating a phenotype in a cell, tissue or animal by performing the method essentially as described herein; and
(ii) identifying a nucleic acid encoding said peptide.

In one embodiment, the method additionally comprises obtaining the nucleic acid.

Methods for identifying and/or obtaining a nucleic acid that encodes a modulatory peptide will be apparent to the skilled person. For example, as the peptide may be expressed by a nucleic acid fragment, a cell having a phenotype of interest may be lysed and the nucleic acid fragment amplified using, for example, PCR or RT-PCR. Such amplified nucleic acid may then be sequenced. Suitable methods for amplifying nucleic acid and/or sequencing nucleic acid will be apparent to the skilled person and/or described in Dieffenbach (ed) and Dveksler (ed) (In: PCR Primer: A Laboratory Manual, Cold Spring Harbour Laboratories, NY, 1995); Ausubel et al (In: Current Protocols in Molecular Biology. Wiley Interscience, ISBN 047 150338, 1987) or (Sambrook et al (In: Molecular Cloning: A Laboratory Manual, Cold Spring Harbor Laboratories, New York, Third Edition 2001).

The present invention also clearly encompasses the use of any *in silico* analytical method and/or industrial process for carrying the screening methods described herein into a pilot scale production or industrial scale production of a compound identified in such screens. This invention also provides for the provision of information for any such production. Accordingly, a further aspect of the present invention provides a process for identifying or determining a peptide or nucleic acid encoding same *supra*, said method comprising:
(i) performing a method as described herein to thereby identify or determine a peptide capable of modulating a phenotype of interest or a nucleic acid encoding same;
(ii) optionally, determining the amount of the peptide;
(iii) optionally, determining the structure of the peptide; and
(iv) providing the compound or the name or structure of the peptide such as, for example, in a paper form, machine-readable form, or computer-readable form.

As used herein, the term "providing the peptide" or "providing the nucleic acid" shall be taken to include any chemical or recombinant synthetic means for producing said peptide or nucleic acid (with or without derivitisation) or alternatively, the provision of a peptide or nucleic acid that has been previously synthesized by any person or means.

In a preferred embodiment, the peptide or nucleic acid or the name or structure of the compound is provided with an indication as to its use e.g., as determined by a screen described herein.

A further aspect of the present invention provides a process for producing a compound *supra*, said method comprising:
a process for identifying or determining a peptide or nucleic acid *supra*, said method comprising:
   (i) performing a method as described herein to thereby identify or determine a peptide capable of modulating an allele and/or a phenotype of interest or a nucleic acid encoding same;
   (ii) optionally, determining the amount of the peptide or nucleic acid;
   (iii) optionally, determining the structure of the peptide or nucleic acid;
   (iv) optionally, providing the name or structure of the peptide or nucleic acid such as, for example, in a paper form, machine-readable form, or computer-readable form; and
   (v) providing the peptide or nucleic acid.

Preferably, the method further comprises providing a chemical derivative of the peptide by protection of the amino-or carboxy-terminus, cyclisation of the peptide or construction of the peptide as a retroinvertopeptide.

In a preferred embodiment, the synthesized peptide or the name or structure of the peptide or nucleic acid is provided with an indication as to its use e.g., as determined by a screen described herein.

A method of manufacturing a medicament comprising a peptide identified by a method or nucleic acid encoding same for use in medicine is disclosed, comprising:
(i) performing a method as described herein to thereby identify or determine a peptide capable of modulating an allele and/or a phenotype of interest or a nucleic acid encoding same; and
(ii) using the peptide in the manufacture of a therapeutic or prophylactic for use in medicine.

The method may comprise the additional step of isolating the peptide. Alternatively, a compound is identified and is produced for use in the manufacture of a compound for use in medicine.

As exemplified herein, the present inventors have performed screens to identify peptides capable of rescuing a yeast cell from cell-death caused by Aurora-A kinase over-expression. As overexpression of Aurora-A kinase is observed in a variety of human cancers, such peptides are of use in the treatment of such cancer. Accordingly, the present disclosure provides a method for treating a cancer, preferably a colorectal cancer or a breast cancer, comprising administering an effective amount of a peptide capable of inhibiting cell death caused by overexpression of Aurora-A kinase in a yeast cell.

The present disclosure also provides for the use of a peptide capable of modulating capable of inhibiting cell death caused by overexpression of Aurora-A kinase in a yeast cell or nucleic acid encoding same identified using the method of the present invention in the manufacture of a medicament for the treatment of a cancer, preferably, a breast cancer or a colorectal cancer.

The present inventors have also screened a library of peptides to determine a peptide that is capable of rescuing a yeast cell from cell death caused by overexpression of cyclin E. Cyclin E overexpression is also associated with cancer. Accordingly, the present disclosure provides a method for treating a cancer comprising administering an effective amount of a peptide capable of inhibiting cell death caused by overexpression of cyclin E in a yeast cell.

The present disclosure also provides for the use of a peptide capable of modulating capable of inhibiting cell death caused by overexpression of cyclin E kinase in a yeast cell or nucleic acid encoding same identified using the method of the present invention in the manufacture of a medicament for the treatment of a cancer, preferably, a breast cancer or a colorectal cancer.

The present inventors have also performed a screen to identify a peptide that modulates oxidative stress. Accordingly, the present disclosure additionally provides a method for manufacturing a medicament comprising a peptide or nucleic acid identified by the method of the present invention for the treatment of a disease or disorder associated with aberrant oxidative stress, e.g., a stroke.

Preferably, a peptide identified using the method of the present disclosure or a nucleic acid encoding same is administered in the form of a composition. More preferably, a pharmaceutical composition. Preferably, the composition or pharmaceutical composition is for use in the treatment of a disease.

The quantities of reagents necessary for effective therapy will depend upon many different factors, including means of administration, target site, physiological state of the patient, and other medicaments administered. Thus, treatment dosages should be titrated to optimize safety and efficacy. Typically, dosages used *in vitro* may provide useful guidance in the amounts useful for *in situ* administration of these reagents. Animal testing of effective doses for treatment of particular disorders will provide further predictive indication of human dosage. Various considerations are described, e.g., in Gilman, et al. (eds.) (1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Pa. Methods for administration are discussed therein, e.g., for oral, intravenous, intraperitoneal, or intramuscular administration, transdermal diffusion, and others. See also Langer (1990) Science 249:1527-1533. Pharmaceutically acceptable carriers will include water, saline, buffers, and other compounds described, e.g., in the Merck Index, Merck & Co., Rahway, N.J. Dosage ranges would ordinarily be expected to be in amounts lower than 1 mM concentrations, typically less than about 10 µM concentrations, usually less than about 100 nM, preferably less than about 10 pM (picomolar), and most preferably less than about 1 fM (femtomolar), with an appropriate carrier. Slow release formulations, or a slow release apparatus will often be utilized for continuous administration.

Therapeutic formulations may be administered in any conventional dosage formulation. While it is possible for the active ingredient to be administered alone, it is preferable to present it as a pharmaceutical formulation. Formulations typically comprise at least one active ingredient, e.g. a peptide identified using the method of the present invention, together with one or more acceptable carriers thereof. Each carrier should be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by any methods well known in the art of pharmacy. See, e.g., Gilman, et al. (eds.) (1990) Goodman and Gilman's: The Pharmacoloaical Bases of Therapeutics, 8th Ed., Pergamon Press, Parrytown, N.Y.; Remington's Pharmaceutical Sciences, 17th ed. (1990), Mack Publishing Co., Easton, Pa.; Avis, et al. (eds.)(1993) Pharmaceutical Dosage Forms: Parenteral Medications 2d ed., Dekker, N.Y.; Lieberman, et al. (eds.)(1990) Pharmaceutical Dosage Forms: Tablets 2d ed., Dekker, NY; and Lieberman, et al. (eds.)(1990) Pharmaceutical Dosage Forms: Disperse Systems Dekker, N.Y. The therapy of this disclosure may be combined with or used in association with other chemotherapeutic or chemopreventive agents.

### Identification of a putative drug target

As exemplified herein, the present inventors have screened a library of peptides to determine or identify a peptide that is capable of overcoming the cytokine dependence of a human cancer cell line. Overcoming cytokine dependence by mutations in genes is thought to be one mechanism by which some forms of cancer, e.g., leukemia. Accordingly, by identifying proteins with which a peptide that overcomes cytokine dependence interacts identifies a putative drug target for the treatment of cancer.

Accordingly, in another embodiment, the method of the present invention additionally comprises determining or identifying a cellular component (e.g., a protein or modified form thereof, a nucleic acid, a carbohydrate, a lipid or a phosphate) to which a peptide identified using the method of the present invention binds. Preferably, the method comprises determining or identifying a peptide, polypeptide or protein to which a peptide identified using the method of the present invention binds.

Methods for determining and/or identifying a peptide, polypeptide or protein to which a peptide identified using the method of the present invention binds are known in the art.

For example, a peptide, polypeptide or protein to which a peptide identified using the method of the present invention binds is isolated using an immunoaffmity purification technique, for example, as described *supra.* In one form of such a method, the purified peptide is immobilized on a solid support and cellular lysate (preferably, from cells in which the screen was performed) is contacted with the peptide. Following washing, any bound peptide, polypeptide or protein is eluted and then identified.

Prior to identification, a sample may be, for example, electrophoresed to isolate individual peptides, polypeptides or proteins in the sample. In one embodiment, an isolated protein is isolated using reducing one-dimensional gel electrophoresis, using methods known in the art, and described, for example, in Scopes (In: Protein purification: principles and practice, Third Edition, Springer Verlag, 1994). In accordance with this embodiment, proteins are separated by their molecular weight.

In another embodiment, a sample comprising an isolated protein are electrophoresed using two-dimensional gel electrophoresis. For example proteins are separated in one dimension using isoelectric focusing. Using such a method, proteins are separated by their isoelectric point, that is the pH at which the net charge of a protein is equal to zero. In order to separate proteins by their isoelectric point a sample is electrophoresed in a gel that comprises a pH gradient. Under such conditions, a protein will move to a position on said gradient where its net charge is equal to zero. Following isoelectric focusing proteins are separated according to their mass, using standard gel electrophoresis.

Following gel electrophoresis proteins are identified, for example, using Edman sequencing, mixed peptide sequencing, mass spectrometry including MALDI, TOF, ESI and ion trap analysis. Edman sequencing is described by Edman, Arch. Biochem. Biophys., 22, 475-483, 1949; mixed-peptide sequencing is described in Damer et al, J. Biol. Chem. 273, 24396-24405, 1998; electrospray ionisation (ESI) is described by, for example Fenn et al, Science, 246, 64-71, 1989 and Wilm et al, Nature, 379, 466-469, 1996; matrix assisted laser desorption/ionisation (MALDI) is described by, for example, Karas and Hillenkamp, Anal. Chem., 60, 2299-2301, 1988; quadrupole mass analysis, or a linear quadripole, is described in Burlingame et al, Anal. Chem. 70, 674R-716R; an ion trap mass analyzer is in Cooks et al, Chem. Eng. News, 69, 26, 1991; time of flight (TOF) analysis is described by Yates, J. Mass Spectrom. 33, 1-19, 1998; Fourier transform ion cyclotron mass spectrometry is described in US Patent No. 3,937,955; a triple quadripole is described in Hunt et al, Proc. Natl. Acad. Sci. USA, 83, 6233-6237, 1986; quadripole-TOF is described in Morris et al, Rapid Commun. Mass Spectrom., 10, 889-896, 1996; and MALDI-QqTOF is described in Loboda et al, Rapid Commun. Mass Spectrom. 14, 1047 - 1057, 2000.

In a preferred embodiment, the interacting protein is identified using N-hybrid analysis.

In one embodiment a polypeptide that binds to a peptide of the present invention is identified that is able to bind a target protein or peptide using the two-hybrid assay described in US Patent No. 6,316,223 to Payan et al and Bartel and Fields, The Yeast Two-Hybrid System, New York, NY, 1997. The basic mechanism described requires that the binding partners are expressed as two distinct fusion proteins in an appropriate host cell, such as for example bacterial cells, yeast cells, and mammalian cells. In adapting the standard two-hybrid screen to the present purpose, a first fusion protein consists of a DNA binding domain fused to the a protein that is derived from a cell in which the interaction occurs that modulates the phenotype of interest, and a second fusion protein consists of a transcriptional activation domain fused to the peptide of the present invention. The DNA binding domain binds to an operator sequence which controls expression of one or more reporter genes. The transcriptional activation domain is recruited to the promoter through the functional interaction between the peptide of the present invention and the target protein. Subsequently, the transcriptional activation domain interacts with the basal transcription machinery of the cell, thereby activating expression of the reporter gene(s), the expression of which can be determined.

Other modifications of the two-hybrid screens are known in the art, such as for example the PolIII two hybrid system, the Tribrid system, the ubiquitin based split protein sensor system and the Sos recruitment system as described in Vidal and Legrain Nucl. Acid Res. 27(4), 919-929 (1999). All of these systems are particularly contemplated.

The present invention is described further in the following non-limiting examples.

### EXAMPLE 1

### Production of a gene fragment expression library

Nucleic acid was isolated from the following bacterial species:

| | |
|---|---|
| *1* | *Archaeoglobus fulgidis* |
| *2* | *Aquifex aeliticus* |
| *3* | *Aeropyrum pernix* |
| *4* | *Bacillus subtilis* |
| *5* | *Bordetella pertussis TOX6* |
| *6* | *Borrelia burgdorferi* |
| *7* | *Chlamydia trachomatis* |
| *8* | *Desulfobacterium autotrophicum* |
| *9* | *Escherichia coli Kl2* |
| *10* | *Haemophilus influenzae (rd)* |
| *11* | *Halobacterium salinarium* |
| *12* | *Haloferax volcanii* |
| *13* | *Helicobacter pylori* |
| *14* | *Methanobacterium thermoautotrophicum* |
| *15* | *Methanococcus, jannaschii* |
| *16* | *Mycoplasma pneumoniae* |
| *17* | *Neisseria meningitides* |
| *18* | *Pirellula Species 1 (rhodopirellula baltica)* |
| *19* | *Pseudomonas aeruginosa* |
| *20* | *Pyrococcus horikoshii* |
| *21* | *Synechosistis PCC 6803* |
| *22* | *Thermoplasma volcanium* |
| *23* | *Thermotoga maritime* |

Nucleic acid fragments were generated from each of these genomes using multiple consecutive rounds of Klenow primer extension using tagged random oligonucleotides.

In the final round of PCR, the sequence of the oligonucleotide primer comprised the sequence:
5'-AGAGGAATTCAGGTCAGACTACAAGGAGGACGACGACAAG-3' (SEQ ID NO: 43).

The primer extension products generated were then used as a template for PCR reactions using the following oligonucleotides:
5'-CAG**AAGCTT**AAGGACGACGACGACAAG-3' (SEQ ID NO: 44);
5'-CAG**AAGCTT**AAGGACGACGACGACAAG-3' (SEQ ID NO: 45);
5'-CA**GGAATTC***C*AAGGACGACGACGACAAG-3' (SEQ ID NO: 46); and
5'-CAG**GAATTC***AC*AAGGACGACGACGACAAG-3' (SEQ ID NO: 47),
wherein the underlined sequence in SEQ ID Nos: 44-47 permits amplification of the PCR products. Furthermore, the sequence shown in bold highlights a *Hind*III restriction endonuclease recognition site or *Eco*RI recognition site. Furthermore, note the addition of one or two nucleotides after the *Eco*RI restriction site in SEQ ID Nos: 46 and 47, respectively (shown in italics). These nucleotides allow expression of amplified nucleic acid in multiple forward reading frames.

Each DNA template was amplified by "one armed" (i.e. using only 1 oligonucleotide primer) PCR, with each of the oligonucleotides (i.e., SEQ ID Nos: 44-47) in separate reactions (i.e. 76 reactions).

Each PCR reaction contained:

| | |
|---|---|
| Template DNA | 1µl |
| Taq buffer (10x) (Promega) | 5µl |
| MgCl₂ (25mM) | 4µl |
| dNTP (2mM) | 5µl |
| a primer selected from the group consisting of SEQ ID Nos: 14-17 (10pmol/µl) | 10µl |
| Taq DNA polymerase (Promega 5U/µl) | 0.4µl |
| H₂O | to 50µl |

Reactions were then cycled in a Perkin Elmer thermocycler PE 9700 or PE 2400 using the following program:
5 min at 94°C, followed by 30 cycles wherein each cycle consists of 30 sec at 94°C, followed by 30 sec at 55°C, and followed by 1 min at 72°C], followed by 5 min at 72°C.

A sample of the resulting PCR products was analyzed by electrophoresis using a 2% agarose/TAE gel. The amount of nucleic acid in each of the PCR products was also determined using the picogreen method following instructions provided by the manufacturer.

PCR products generated with each of the oligonucleotides SEQ ID Nos: 44 to 47 were pooled. DNA from each organism was added in an equimolar amount when compared to the amount of nucleic acid added to the pool from the organism with the smallest genome.

Subsequently, the pools generated from PCR products amplified using the oligonucleotides SEQ ID NO: 45, SEQ ID NO: 46 or SEQ ID NO: 47 were combined in equal ratios (i.e. equal amounts of nucleic acid) to form one pool.

The pooled PCR products were then purified using QIAquick PCR purification columns (QIAGEN) as per manufacturer's instructions. This step removes any unincorporated oligonucleotides, dNTPs and contaminating proteins.

Each of the pools of PCR products (6µg) was then divided into 3 equal parts and each part digested with a different one of the restriction enzymes AluI, HaeII or RsaI (NEB) in the following reaction:

| | |
|---|---|
| PCR product (2µg) | |
| Restriction endonuclease buffer (10x) (NEB) | 4µl |
| Restriction endonuclease | 1µl |
| H₂O | to 40µl |

Reactions were allowed to proceed for 2 hours at 37°C, before being heat inactivated by incubating at 65°C for 20 minutes. Restriction digests were then re-pooled and purified using QIAquick PCR purification columns (QIAGEN) as per manufacturer's instructions.

Each of the enzymes AluI, HaeII and RsaI produce blunt ends. Accordingly, it is possible to ligate blunt end adaptors to the restriction digested PCR products to allow directional cloning into the pMF4-5 vector (Phylogica Ltd, Perth, Australia). Oligonucleotides encoding the blunt-end adaptors were generated comprising the following sequences:
5'-AATTCGAACCCCTTCG-3' (SEQ ID NO: 48)
5'-CGAAGGGGTTCG-3' (SEQ ID NO: 49)
5'-AATTCGAACCCCTTCGC-3' (SEQ ID NO: 50)
5'-GCGAAGGGGTTCG-3' (SEQ ID NO: 51)
5'-AATTCGAACCCCTTCGCG-3' (SEQ ID NO: 52)
5'- CGCGAAGGGGTTCG-3' (SEQ ID NO: 53)
5'-AGCTCGAAGGGGTTCG-3' (SEQ ID NO: 54)
5'-CGAACCCCTTCG-3' (SEQ ID NO: 55).

The adaptor pairs SEQ ID Nos: 48 and 49; SEQ ID Nos: 50 and 51; SEQ ID NOs: 52 and 53; SEQ ID NOs: 54 and 55 were then annealed to one another. This process was completed in H₂O with each of the oligonucleotides at a concentration of 50µM. Pairs of adaptors were incubated at 94°C for 10 minutes and then allowed to cool to room temperature slowly.

The annealed adaptors were then ligated to the pool of amplified PCR products in separate ligation reactions. The adaptor formed through annealing of SEQ ID NOs: 52 and 53 was ligated to the pool of PCR products amplified using the oligonucleotides set forth in SEQ ID NO: 53, SEQ ID NO: 54 and SEQ ID NO: 55.

Ligations were carried out in the following reactions:

| | |
|---|---|
| Pooled PCR product (average length of 200bp) | 2 pmol |
| Annealed adaptor | 150 pmol |
| Ligation buffer (10x) (Promega) | 1µl |
| T4 DNA ligase (3U/µl) (Promega) | 1µl |
| H₂O | to 10µl |

Samples were then incubated at 4°C overnight before being heat inactivated through incubation at 65°C for 20 minutes.

Samples were then phosphorylated using T4 polynucleotide kinase (Promega) in the following reaction:

| | |
|---|---|
| Ligation buffer (10x) (Promega) | 1µl |
| rATP (10mM) | 2µl |
| T4 polynucleotide kinase (5U/µl) | 1µl |
| H₂O | 20µl |

Samples were incubated at 37°C for 30 minutes followed by incubation at 65°C for 20 minutes to heat inactivate the T4 polynucleotide kinase.

Following ligation and phosphorylation each of the three reactions comprising nucleic acid amplified using the oligonucleotide SEQ ID NO: 44 were combined in equal ratios, i.e. equal amounts of nucleic acid to form one pool.

The nucleic acids originally amplified with SEQ ID NO: 44 were then digested with the restriction endonuclease *Hin*dIII in the following reaction:
PCR product (2 µg)

| | |
|---|---|
| HindIII buffer (10x) (Promega) | 8µl |
| HindIII (10U/µl) (Promega) | 1 µl |
| H₂O | to 80µl |

The nucleic acids in the pool originally amplified by one of SEQ ID Nos: 45-47 were digested with the restriction endonuclease *Eco*RI in the following reaction:

| | |
|---|---|
| PCR product (2 µg) | |
| EcoRI buffer (10x) (Promega) | 8µl |
| EcoRI (10U/µl) (Promega) | 1µl |
| H₂O | to 80µl |

Samples were then purified using a QIAquick PCR purification column (QIAGEN) as per manufacturer's instructions. Nucleic acid concentration was then determined by spectrophotometry measuring UV absorption at 260nm.

Both pools of nucleic acid fragments (i.e. those digested with EcoRI and those digested with HindIII) were then combined in equal ratios, i.e. equal amounts of nucleic acid, to form one pool. This pool of nucleic acid fragments was then suitable for cloning into the expressing vector PMF4-5.

The nucleic acid fragments were then ligated into the pMF4-5 vector using the following reaction:

| | |
|---|---|
| Ligation buffer (10x) (Novagen) | 0.5µl |
| rATP (10mM) | 0.5µl |
| DTT (10mM) | 0.5µl |
| PMF4-5(0.02pmol) | 1µl |
| Nucleic acid fragments | |
| (0; 0.02; and 0.06 pmol in independent reactions) | |
| H₂O | to 5µl |

Reactions were incubated at 16°C overnight.

### EXAMPLE 2

### Screening a peptide expression library to identify a peptide that inhibits a phenotype associated with overexpression of Aurora-A kinase

Aurora-A kinase (Aurora 2) is cloned into the pDD vector (Phylogica Ltd, Perth, Australia) essentially as described in Bischoff et al., EMBO J. 17: 3052-3065, 1998, thereby placing expression of this protein under control of the galactose inducible promoter GAL1. The expression construct is then transformed into the yeast strain SKY 473 (*MAT*α*, his3, trp1, ura3, 4 LexA-LEU2, lys2::3 clop-LYS2, CANR* ). Yeast are grown in the presence of glucose to suppress expression of the Aurora-A kinase that is toxic to yeast.

The yeast strain PRT51 (*MAT*α*, his3, trp1, ura3, 6 LexA-LEU2, lys2:3 clop-LYS2, CYH2R, ade2: G418-pZero-ade2, met15:Zeo-pBLUE-met15, his5::hygro*) are then transformed with the pMF4-5 expression construct described in Example 1. The library is then mass mated with the SKY 473 yeast strain and plated onto trp- and his- selective media with galactose to induce expression of the Aurora-A kinase and Phylomer expression library.

As a positive control Aurora Interacting Protein (SEQ ID NO: 56) was amplified using RT-PCR with the primers comprising the sequences set forth in CGC TGC CGA TCG GGG CCG ACT (SEQ ID NO: 58) and CGC TGC CGA TCG GGG CCG ACT (SEQ ID NO: 59) using mRNA from HeLa cells and cloned into the pMF4-5 vector. This vector is also transformed into the PRT51 strain of yeast and mated with the SKY 473 yeast strain carrying the Aurora-A kinase expression construct. This peptide Aurora Interacting Protein inhibits the toxic effects of Aurora-A kinase on yeast cells.

Those colonies that grow are considered to express a peptide that rescues the yeast strain from the toxic effect of Aurora-A kinase expression. To confirm results, plasmids are rescued and retransformed.

Some peptides that rescue the phenotype are expected to do so by direct interaction with Aurora-A kinase, while others are expected to do so by interaction with other proteins in the yeast cell.

### EXAMPLE 3

### In vitro analysis of an inhibitor of Aurora-A kinase

Peptides identified in the screen described in Example 2 are synthesized using the multipin Pepset format by Mimotopes, Melbourne, Australia.

Recombinant Aurora-A kinase is purchased from Proquinase GmbH (Freiburg, Germany). Following pretreatment with inhibitor peptides (1µM in Buffer A(20 mM HEPES, 20 mM MgCl₂, 20 mM β-glycerophosphate, pH 7.6, containing 500 µM dithiothreitol, 100 µM sodium orthovanadate) for 10 min, 30 °C)), Aurora-A activity is assayed by incubation in Buffer A supplemented with 20 µM ATP, 100 µMof [γ-³²P]ATP, and a protein substrate (0.5 mg/ml Histone H3). The reaction is performed for 30 min at 30 °C, and then the phosphorylated substrate is separated by SDS-PAGE, visualized by autoradiography, and quantitated by Cerenkov counting.

These peptides are capable of directly modulating the activity of Aurora-A kinase, i.e. modulating an allele that is associated with a phenotype.

Using lower concentrations of each of the previously identified inhibitory peptides enables determination of the IC₅₀ of each peptide.

### EXAMPLE 4

### Ex vivo assessment of an inhibitor of Aurora-A kinase

Nucleic acid encoding Aurora-A kinase is cloned into the pcDNA 3.1 vector (Invitrogen) for high level expression under the control of cytomegalovirus enhancer promoter (essentially as described in Zhou et al., Nat. Genet. 20: 189-193, 1998.. For stable transfection, 1 µg is mixed with lipofectamine reagent (12 µl; Gibco BRL) to 3x10⁵ cells in a 60-mm dish. After 5 h incubation in serum-free medium, complete medium with serum is added to the cells and incubated them for 48 h. Stable clones are selected with 600 µg/ml G418.

Expression of Aurora-A kinase is then analyzed using Western blotting. Cell extracts are prepared by lysis by sonication with five volumes of extraction buffer (80 mM Na β-glycerophosphate, 20 mM EDTA, 15 mM MgCl₂, 1 mM DTT, 1 mM ATP, 1 µM okadaic acid) and protease inhibitor (10 µg/ml of each; leupeptin, pepstatin A and chymostatin; Boehringer). Total protein concentrations are determined by Bradford analysis. A polyclonal anti-Aurora-A kinase antibody raised against a carboxy-terminal peptide in rabbit described in Zhou et al., Nat. Genet. 20: 189-193, 1998 is used to detect protein expression.

Cells that stably express Auroa-A kinase are then transfected with an expression vector encoding a peptide positively identified in Example 3. Nucleic acid encoding a positively identified peptide is cloned in frame into the pIRES-hrGFP vector (Stratagene). This vector allows for high level expression (from a CMV promoter and enhancer) of the peptide and a GFP protein by virtue of an internal ribosome entry site. Again, DNA (1 µg) is mixed with lipofectamine reagent (12 µl; Gibco BRL) to 3x10⁵ cells in a 60-mm dish. After 5 h incubation in serum-free medium, complete medium with serum is added to the cells and incubated them for 48 h. Cells that express GFP are then sorted using FACS essentially as described in Bierhuizen et al., Biochem Biophys Res Commun. 234: 371-5, 1997.

Cells expressing both Aurora-A kinase and a previously identified peptide inhibitor are studied to determined foci formation, essentially as described in Zhou et al., Nat. Genet. 20: 189-193, 1998. For 3T3 focus formation assay, 1x10⁶ cells previously described clones are grown in a 100-mm dish in medium containing bovine calf serum. Aurora-A kinase expressing cells (without a peptide inhibitor) form foci after about 10 d. Those peptides that inhibit Aurora-A kinase inhibit formation of foci formation.

### EXAMPLE 5

### Determining the effect of a peptide inhibitor of Aurora-A kinase on breast cancer cell proliferation.

Various breast cancer cell lines (low grade: MCF7; and high grade BT474 and MDA468) cells are expressed with the pIRES-hrGFP vector containing a nucleic acid encoding a previously identified peptide essentially as described in Example 4.

The level of Aurora-A kinase expression is determined using a Western blot essentially as described in Example 4. Following this, cellular proliferation is determined using the CellTiter Assay (Promega Corporation). Essentially this assay involves, incubation of the cells in a 96 well plate with the Cell-Titer Blue reagent for 1-4 hours. Actively dividing cells convert the Resazurin in the buffer to Rezorufin, that emits a fluorescent signal that is phase shifted compared to Resazurin. Plates are then read at 560/590nm and results compared to cells that are not actively dividing and cells that are known to be dividing. Using this protocol, a peptide that inhibits proliferation of a breast cancer cell line is determined. Furthermore, using various concentrations of the peptide inhibitor an IC₅₀ value is determined.

### EXAMPLE 7

### Identification of a peptide inhibitor of yeast cell death caused by overexpression of cyclin E

In mammalian cells cyclin E, in association with, is a positive regulator of the G1-to-S phase transition of the cell cycle (major cell cycle transitions). Cyclin E is often found to be overexpressed in human cancers, and cell culture models suggest that cyclin E overexpression causes genomic instability (Spruck et al., Nature, 401: 297-300). A mouse model of Cyclin E overexpression has shown that deregulation of this protein is associated with loss of heterozygosity at the p53 tumor suppressor locus.

Human cyclin E was identified in a genetic screen by virtue of its ability to rescue a deficiency of G1 cyclin function in the budding yeast Saccharomyces cereviseae (Lew et al., Cell, 66: 1197-1206, 1991). However, over-expression of human cyclin E in yeast, genetically modified to co-express human Cdk2, is lethal to yeast cells. Accordingly, by screening cells overexpressing cyclin E and CDK2 peptide inhibitors of cyclin E are determined.

The human cyclin E cDNA is cloned into the pDD vector as an in frame fusion with the LexA protein. The construct is then transformed into the yeast starin AZ-1 (*Mat*α*, ade1, his2, leu2-3, 112trp-la, ura3,* huCDK2::*his2*). These cells constitutively express the human CDK2 gene under control of the glyceraldehyde 3-phosphate dehydrogenase promoter (GAP) (Won and Reed, EMBO J., 15: 4182-4193, 1996). Transformants are grown on appropriate selection media in the presence of glucose to suppress expression of cyclin E, which is toxic to yeast cells in the presence of CDK2.

As a positive control cDNA encoding the p21^{CIP1} gene (SEQ ID NO: 60) is cloned as an in-frame fusion into the pMF4-5 plasmid, as the p21^{CIP1} protein has been shown to rescue the lethal phenotype in yeast.

The library of nucleic acid fragments in the pMF4-5 vector (Example 1) are then transformed into the yeast strain PRT 51. These yeast are then mass mated with the AZ-1 yeast carrying the cyclin E expression construct. Cells are then grown on media containing an appropriate level of galactose to induce expression of cyclin E thereby killing any yeast cells that do not express a peptide inhibitor of the cyclin E lethal phenotype. Media is also leu-, thereby selecting for peptides that interact with cyclin E (causing expression of the LEU 2 reporter gene) and inhibit the cyclin E lethal phenotype.

Any positive colonies are isolated and plasmids rescued. Plasmids are retransformed and screened to confirm a positive finding.

### EXAMPLE 8

### Identification of a peptide capable of complementing cytokine dependence of a human cancer cell line.

Peptides of the present invention are screened to identify those that are capable of rescuing a cell from cytokine dependence.

A library of nucleic acid fragments is produced essentially as described in Example 1. However the fragments are cloned into the MICR1 retroviral vector (Koh, et al.,. Nucleic Acids Research 30:e142). This IRES-GFP retroviral vector is based on MSCV2.2 virus. Retroviral supernatants are produced and subsequent infections of target cells are performed essentially as described in (Koh, et al.,. Nucleic Acids Research 30:e142).

Culturing of the murine IL-3-dependent BaF/3 and 32D cells is performed essentially as described in Klucher et al.,Blood, 91,3927-3934. 1998. Human GM-CSF-dependent TF-1 cells engineered to contain ecotropic receptor are cultured essentially as described in Kitamura et al., J. Cell Physiol., 140, 323-334.1989). The eco-TF-1 cells are grown in RMPI 1640 containing 10% fetal calf serum (FCS) and 4 ng/ml of human GM-CSF (Peprotech). 293T cells (DuBridge et al., Mol. Cell. Biol., 7, 379-387, 1987) are grown in DMEM containing 10% FCS and penicillin-streptomycin (10 U/mL and 10 mg/mL). All cells are incubated at 37°C with 5% CO₂.

Following transduction, cells are grown in the presence of IL3 for 4 days to allow expression of the retrovirus, at which point the efficiency of transduction is determined by FACS analysis as described *supra.* The IL3 is then removed from the media by two sequential washes and the cells outgrown for 10-30 days until colonies emerge. Any colonies that emerge are considered to express a peptide capable of rescuing the cytokine dependent phenotype of the cells. The inserts from the retroviral integration sites in these rescued clones are then isolated by PCR .

### EXAMPLE 9:

### A Screen For Agonists of The Human Interferon Type I (IFN) Receptor

Type I interferons are polypeptides ranging in size between 17-20 kDa. These proteins are currently used in the treatment of Hepatitis, hairy cell leukemia, condyloma acuminatum, multiple sclerosis, and Kaposi sarcoma. However, the type I interferons are inherently unstable and relatively expensive to produce. Because of this, high doses are required to obtain an effect in patients, and this in turn increases the cost of treatment significantly.

A screen is performed to identify smaller and more stable peptide agonists of the interferon type I receptor for treatment.

### 9.1 production ofpooled recombinant biodiverse expression libraries

A biodiverse gene fragment expression library is produced essentially as described in Example 1. However this library is cloned into a modified pYTB vector (Phylogica Ltd, Perth, Australia). The pYTB vector is modified to include a HIS tag in place of the FLAG tag already incorporated into this vector. The HIS tag facilitates peptide purification. The pYTB vector comprises a T7 promoter thereby facilitating *in vitro* expression of a cloned fragment.

The that comprise a genome fragment are arrayed in a 96 well format, thereby producing 1000 pools of 100 encoded peptides. An example of such an array procedure is shown in Figure 1. Each of the vectors are linearized and the peptides encoded by the cloned genomic fragments expressed using a bacterial *in vitro* transcription/translation system (RTS100HY, Roche Applied Systems).

The RTS100HY system produces approximately 20µg of protein (i.e. approximately 200ng of each individual peptide). While the level of protein produced/peptide is relatively low, this provides a selection for only those peptides that are potent agonists of an interferon receptor.

Expressed peptides are purified from the reaction using high throughput magnetic beads purification, Dynabeads™ TALON™ system (Dynal) - for his-tagged protein purification. This system used magnetic beads to purify the peptides from the *in vitro* expression extract and is suitable for 96-well format purification.

### 9.2 Screening for peptides that inhibit neutrophil apoptosis

Neutrophils are small immune cells that spontaneously apoptose 24-48 hours after leaving the bone marrow. However, the cells can be prevented from apoptosing in the presence of type I interferons.

Neutrophils are isolated from human subjects to assess the ability of the peptides to suppress apoptosis. Twenty to 100 ml of venous blood is taken from healthy volunteers, and neutrophils are isolated on Percoll density gradients essentially as described in Affordet al.. J. Biol. Chem. 267:21612, 1992. Neutrophil preparations containing >98% neutrophils are resuspended in RPMI 1640 medium (Life Technologies, Gaithersburg, MD), supplemented with 10% heat-inactivated FCS (Sera-Lab, Loughborough, U.K.) and containing 100 U/ml penicillin and 100 µg/ml streptomycin (Sigma-Aldrich, St. Louis, MO). Neutrophils are either used immediately as healthy control cells or were cultured in a humidified 5% CO₂ atmosphere in the presence or the absence of recombinant human IFN-β (BioSource, Camarillo, CA) or human type 1 IFN purified from fibroblast tissue culture supernatant (Sigma-Aldrich) or in the presence of a pool of expressed peptides.

To determine the effect of each of the peptides on spontaneous neutrophil apoptosis in vitro cytospin preparations (3 min, 10 x *g*; Cytospin 2; Shandon, Pittsburgh, PA) are made of freshly isolated or neutrophils cultured for up to 20 h in medium alone or in the presence of a range of concentrations of pooled peptides. Cytospins are then differentially stained using a commercial May-Grunwald Giemsa stain (Diff-Quick; Gamidor, Abingdon, Oxfordshire, U.K.) and assessed for apoptotic morphology. Morphological assessments are confirmed by measurement of annexin V binding using a commercial kit (R&D Systems, Minneapolis, MN) and flow cytometric analysis.

Pools of peptides that are capable of inhibiting neutrophil cell death are then further analyzed (i.e. using sub-pools comprising fewer peptides) to identify those specific peptides that are capable of inhibiting neutrophil cell death.

### 9.3 Screening for peptides capable of binding to and activating a chimeric interferon receptor

A cell line expressing a chimeric interferon receptor is produced essentially as described in Carroll et al., Proc. Soc. Exp. Biol. Med., 206: 289-294, 1994. Essentially an expression construct is produced that encodes an extracellular domain of interferon α or an extracellular domain of interferon P that is fused with the cytoplasmic domain of the IL-3 cell line. This construct is then transfected into Ba/F3 cell line essentially as described in Example 8. By culturing these cells in the presence of a peptide identified in the primary screens, and in the absence of interferon or IL-3, those peptides capable of binding to and activating the chimeric receptor (i.e. binding to and activating an interferon receptor) are identified. Only those cells that are capable of activating the receptor are capable of growing in the absence of IL-3.

Any peptides identified in the primary screen and/or secondary screen are then assayed in a standard viral bioassay for interferon activity, essentially as described in Pestka (Ed) (1986) "Interferon Standards and General Abbreviations," in Methods in Enzymology..), Academic Press, New York 119, 14-23].

### EXAMPLE 10

### Screening for agonists of the growth factor receptors Erythropoietin (Epo), G-CSF or GMCSF.

The murine haematopoietic cell line 32D was originally described as predominantly a basophil/mast cell line that retains the capacity to give rise to cells which proliferate and differentiate in response to Epo, GM-CSF, and/or G-CSF. (Greenberger, J et al., Proc. Natl. Acad. Sci USA 80:2931-2935, 1983). More recently subclones of the 32D line have been developed which are differentially responsive to Epo (e.g. line 32D Epol), GM-CSF (e.g. line 32D GM1) or G-CSF (e.g. line 32D G1). Migliaccio et al J Cell Biol.;109:833-41, 1989. These subcloned cell lines are useful for determining a peptide capable of binding to and/or activating a particular growth factor receptor (is. Epo, GM-CSF, and/or G-CSF) using a screen for stimulation of 32D proliferation.

32D cells are grown by biweekly passage in McCoys medium (Gibco, NY) supplemented with antibiotics, L-glutamine and 1% pyruvic acid (Gibco, NY) and 10% horse serum with IL3 added exogenously.

The library described in Example 9 in the modified pYTB3 vector is produced and pools of recombinant peptides are produced as described previously.

The purified pools of peptides are then cultured with the 32D cells under the following conditions: 1ml of FBS-deprived medium and approximately 20 µg of each peptide pool in semisolid medium (Iscove's modified dulbeccos's medium: 0.8% methylcellulose beta mercaptoethanol (75 micromolar), supplemented with the following mixture of nutrients which replaced serum: BSA (200 micromolar), BSA-absorbed cholesterol (12 micrograms/ml), soybean lecithin (36 micrograms/ml) trasferrin (9 micromolar) bovine insulin (1.7 micromolar) nucleosides (10 micrograms/ml each, sodium pyruvate (100 micromolar) and L-glutamine (2 millimolar). Cells are cultured at 37°C for 8 days and the number of cells per well or per colony scored. Those wells that include colonies with more than 500 cells and significantly more cells than negative control wells (i.e. an in vitro transcribed/translated vector control sample, purified in parallel with the test samples) are isolated from further analysis are considered to activate one or more of the Epo, G-CSF or GM-CSF receptors and are selected for further analysis.

Pools of peptides that are capable of inducing colony formation in 32D cells are then assayed using the cell lines 32D Epol, 32D GM1, or 32D G1 to determine which of the receptors the peptides are capable of activating. Essentially the ability of each pool of peptides to induce colony formation is assessed as described above for the 32D cell line.

Those pools of peptides that are capable of inducing formation of a colony are then further studied, by determining which specific peptide/s in each pool are capable of inducing colony formation in the 32D cell line and/or one or more of the cell lines 32D Epol, 32D GM1, or 32D G1.

### EXAMPLE 11

### Production of gene fragments for an expression library

### 11.1. Random amplification of genomic DNA by Klenow polymerase

Small amounts of DNA (1-10 µg) from bacterial strains with fully sequenced genomes were obtained from research groups and culture collections. For the construction of this library DNA from the following 25 bacteria was used:

| | **Organism** | **Genome Size (Kb)** | **Multiplier¹** |
|---|---|---|---|
| 1 | *Archaeoglobus fulgidus* | 2178 | 2.7 |
| 2 | *Aquifex aeolicus* | 1590 | 1.9 |
| 3 | *Aeropyrum pernix* | 1670 | 2.0 |
| 4 | *Bacillus subtilis* | 4214 | 5.2 |
| 5 | *Bordetella pertussis* | 3880 | 4.7 |
| 6 | *Borrelia burgdorferi* | 1230 | 1.5 |
| 7 | *Chlamydia trachomatis* | 1000 | 1.2 |
| 8 | *Escherichia coli K12* | 4639 | 5.7 |
| 9 | *Haemophilus influenzae* | 1830 | 2.2 |
| 10 | *Helicobacter pylori* | 1667 | 2.0 |
| 11 | *Methanobacterium thermoautotrophicum.* | 1751 | 2.1 |
| 12 | *Methanococcus jannashii* | 1664 | 2.0 |
| 13 | *Neisseria meningitidis* | 2157 | 2.6 |
| 14 | *Pyrococcus horikoshii* | 1800 | 2.2 |
| 15 | *Pseudomonas aeruginosa* | 5940 | 7.3 |
| 16 | *Synechocystis PCC 6803* | 3673 | 4.5 |
| 17 | *Thermoplasma volcanicum* | 1700 | 2.1 |
| 18 | *Thermotoga maritima* | 1800 | 2.2 |
| 19 | *Acidobacterium capsulatum* | 2841 | 3.5 |
| 20 | *Halobacterium salinarum* | 2000 | 2.4 |
| 21 | *Desulfobacterium autotrophicum* | 5500 | 6.7 |
| 22 | *Haloferax volcanii* | 4200 | 5.1 |
| 23 | *Rhodopirellula baltica* | 7146 | 8.7 |
| 24 | *Thermus thermophilus HB27* | 1894 | 2.3 |
| 25 | *Prochlorococcus marinus MED4* | 1658 | 2.0 |

| | | | |
|---|---|---|---|
| ¹ Multiplier indicates the size of the genome (kb) relative to the smallest genome used. This figure is used to determine the amount of amplified nucleic acid used to produce a library. | | | |

The DNA samples were individually subjected to four consecutive rounds of "tagged random amplification" by the Klenow fragment of *E. coli* DNA polymerase. The use of a tagged primer with a 3'N9 (instead of a 3'N6) portion led to small, uniform fragments. The primer contains a *Mfe*I restriction site which produces overhangs compatible with *EcoR*I*.* Amplification in the presence of NaCl was found to increase the yield with the tagged N9 primer.

Each DNA sample was used in the following amplification reaction:
100 ng of genomic DNA in a volume of 2 µl was added to 4 µl of the primer T7MfeN9 (SEQ ID NO: 32; 25pmol/µl) and the volume made up to 10 µl with H₂O. Reactions were prepared in 0.2 ml thin-walled PCR tubes and all subsequent incubations were performed in an PE2400 thermocycler (Perkin Elmer).

**First round amplification**: Following incubating the sample at 98°C for 5 min, 3 µl of 10x DNA polymerase buffer (Promega), 6 µl of 50% (w/v) PEG8000, 3 µl of 2 mM dNTP, 3 µl of 1M NaCl and 0.6 µl of Klenow DNA polymerase were added. The volume was made up to 30 µl with H₂O and the samples incubated for 50 min at 22°C and 15 min 37°C.

**Second round amplification**: The 30 µl sample from the first round amplification was incubated at 5 min 98°C to denature double stranded DNA and facilitate new primer binding to the target and newly synthesized DNA. Following this step, 0.5 µl of 10x DNA polymerase buffer (Promega), 0.5 µl of 2 mM dNTP, 0.5 µl of 1M NaCl and 0.5 µl of Klenow DNA polymerase and 2 µl of T7MfeN9 (25µmol/µl) were added. The volume was made up to 35 µl with H₂O and the samples incubated for 50 min at 22°C and 15 min 37°C.

**Third round amplification**: The 35 µl sample from the second round amplification was incubated at 5 min 98°C. Then 0.5 µl of 10x DNA polymerase buffer (Promega), 0.5 µl of 2 mM dNTP, 0.5 µl of 1M NaCl and 0.5 µl of Klenow DNA polymerase and 2 µl of T7MfeN9 (25pmol/µl) were added. The volume was made up to 40 µl with H₂O and the samples incubated for 50 min at 22°C and 15 min 37°C.

**Fourth round amplification**: The 40 µl sample from round #3 was incubated at 5 min 98°C. Then 0.5 µl of 10x DNA polymerase buffer (Promega), 0.5 µl of 2 mM dNTP, 0.5 µl of 1M NaCl and 0.5 µl of Klenow DNA polymerase and 2 µl of the primer T7MfeN9 (SEQ ID NO: 32; 25pmol/µl) were added. The volume was made up to 45 µl with H₂O and the samples incubated for 50 min at 22°C and 15 min 37°C.

**Buffer exchange**: An Amersham S200 spin column was prepared for use essentially according to manufacturer's instructions. The 45 µl fourth round amplification reaction was applied to the column and spun for 2 min at 735 x g (2764 rpm Hettich Micro 20). The purified sample was collected in 1.5 ml reaction tube and stored at -20°C.

### 11.2. Specific PCR amplification of amplified nucleic acid

Each sample was individually amplified with the primer T7Mfe (SEQ ID NO: 63), which specifically binds to the tag introduced by amplification with T7MfeN9 (SEQ ID NO: 62) (see above)

The site of the *Mfe*I cleavage site is indicated by the box.

Amplification was carried out with Pfu DNA polymerase due to its lower error incorporation rate and lower processivity. 2 µl of Klenow amplified S200 purified DNA were added to 2.5 µl of 10x Promega Pfu buffer, 2.5 µl of 2 mM dNTP, 6 µl of T7Mfe primer (SEQ ID NO: 33; 10 pmol/µl), 0.4 µl of Pfu-DNA-polymerase (Promega) and the volume of the reaction was made up to 25 µl with H₂O. Thermocycling conditions were: 5 min at 94°C, followed by 30 cycles of 30 sec at 94°C, 30 sec at 60°C and 1 min at 72°C. Finally samples were incubated for 2 min 72°C and then maintained at 4°C.

The PCR amplified samples were electrophoresed on 2% TAE agarose gels and stained with ethidium bromide. The samples were quantified by comparison with known band intensities of a DNA size standard (100 bp ladder; Promega; quantification on Geldoc; Biorad).

To obtain representative amounts of each of the 25 bacterial genomes, the PCR products were pooled according to concentration and genome size (proportionally higher amounts from bacteria with bigger genomes and smaller amounts from bacteria with smaller genomes). Subsequently, the pool was digested with the restriction enzyme *Mfe*I in the following reaction: 330 µl of pooled T7Mfe PCR products (17 µg) were added to 40 µl of 10x MfeI restriction buffer (NEB buffer 4), 4 µl of BSA (10 mg/ml, 7 µl of MfeI (10U/µl) and made up to 400 µl with H₂O. Restriction was carried out for 2.5 h at 37°C followed by heat inactivation of the enzyme at 65°C for 10 min. 100 µl of MfeI digested DNA was purified by QIAquick® PCR purification (Qiagen) essentially according to the manufacturer's instructions. The sample was eluted with 45 µl of 10 mM Tris/Cl, pH 8.5 from the QIAquick column and stored at -20°C.

### EXAMPLE 12

### Identification of peptide inhibitors of tumor necrosis factor α (TNF-α) signaling

The gene fragments produced in Example 11 are cloned into the *EcoR*I site of the pcDNA3.1 vector (Invitrogen) to produce an expression library.

The cell lines OCI-AML-1 and OCI-AML-11 are transfected with the expression library. TNF-α induces apoptosis in the cell lines OCI-AML-1 and OCI-AML-11.

For transfection, 1 µg of the library is mixed with lipofectamine reagent (Gibco BRL) and added to 3x10⁵ cells in a 60-mm dish. After 5 h incubation in serum-free medium, complete medium with serum is added to the cells and incubated them for 48 h. Transfected clones are selected with 600 µg/ml G418.

Following transfection cells are incubated in the presence of TNF-α and cells that do not die by apoptosis selected. Surviving cells are lysed and the nucleic acid encoding the peptide expressed by the cell amplified using PCR. The amplified nucleic acid is then cloned into the pcDNA3.1 vector for further analysis.

Nucleic acid encoding each of the peptides selected in the first round of selection are transfected into the cell lines HU-3, M-07e and TF-1, essentially as described *supra*. TNF-α prevents apoptosis and induces cellular proliferation in the cell lines HU-3, M-07e and TF-1.

Following transfection and selection for the presence of the pcDNA3.1 expression vector using G418, cells are assessed for proliferation using the cell proliferation assay kit available from Stratagene. Assays are performed essentially according to manufacturer's instructions.

Those cells that proliferate at a significantly lower level than control cells (i.e., cells transfected with an empty pcDNA3.1 vector) are considered to express a peptide that inhibits TNF-α signaling.

Cells with reduced levels of proliferation are grown in the absence of TNF-α and lysed. Nucleic acid encoding the expressed peptide isolated by PCR. Amplified nucleic acid is then sequenced and the amino acid sequence of the encoded peptide is then elucidated from the nucleotide sequence.

### EXAMPLE 13

### Effect of TNF-α signaling inhibitory molecules in a mouse delayed type hypersensitivity (DTH) model

Peptides identified in the screen described n Example 11 are synthesized using the multipin Pepset format by Mimotopes, Melbourne, Australia.

mBSA-induced DTH is induced essentially as described in Zheng et al., Immunity, 3: 9-19, 1995. Briefly, mice are sensitized by injecting 1.25mg/ml mBSA (Sigma) in CFA at the base of the tail. Seven days after sensitization mice are challenged with 200µg/20µl mBSA in the right footpad and 20µl of PBS injected into the left footpad. Footpad swelling is measured using a caliper.

Prior to and/or at the time of challenge with mBSA mice are also administered one of the test peptides suspended in phosphate buffered saline (PBS) in the right footpad. Control mice are administered PBS and no peptide.

A peptide that reduces the degree of footswelling compared to control mice are considered to reduce or inhibit TNF-α signaling *in vivo*.

### EXAMPLE 14

### Isolation of a peptide that complements an TGF-α dependent cell

TGF-α dependent cells are produced essentially as described in Howell et al., Mol. and Cell. Biol., 18: 303-313, 1998. Briefly, HCT116 cells are transfected with a construct (pRC/CMV; Invitrogen) with a cDNA encoding TGF-α cloned in the antisense orientation relative to the CMV promoter. This construct encodes a TGF-α antisense RNA. Cells are transfected by electroporation and selected in the presence of geneticin and TGF-α.

A cell line stably expressing the TGF-α antisense RNA is selected. These cells are then transfected with the expression library described in Examples 11 and 12. Following transfection cells are maintained in the absence of TGF-α. Only those cells that expresses a peptide that is capable of inducing the TGF-α signaling pathway are capable of growing under these conditions. Any colonies that emerge are considered to express a peptide capable of rescuing the cytokine dependent phenotype of the cells.

Any colonies are isolated, lysed and the nucleic acid encoding the peptide that complements the TGF-α dependency of the cells amplified by PCR.

These fragments are then recloned into the pcDNA3.1 vector and retransformed into the HCT116 TGF-α dependent cells to confirm the ability of the peptide to rescue this phenotype.

### EXAMPLE 15

### Determining a peptide that modulates oxidative stress

A screen to identify a peptide that protects a cell against oxidative stress is performed in HEK293 mammalian cells that are stressed with hydrogen peroxide.
HEK293 cells are an adherent human embryonic kidney cell line and are grown in standard media (DMEM supplemented with 10% foetal calf serum (FCS), 2mM L-glutamine, and 50 units/ml penicillin/streptomycin solution) using tissue culture flasks for adherent cells. Cells are incubated in a tissue culture incubator at 37°C, 5% CO₂. On day 1 a confluent T75 flask of HEK293 cells are treated with a trypsin reagent (trypsin:EDTA 1:250 reagent (MultiCel™)) until the cells detach from the surface of the flask. The trypsin reagent is inactivated with transfection media (DMEM supplemented with 10% FCS and L-glutamine). The cells are then split 2/5 into two new T75 flasks and the total volume in each flask made up to 15ml (volume made up with transfection media and incubated overnight.
On day 2 pairs of flasks containing cultures that are 80-90% confluent are transfected with plasmid DNA using Lipofectamine 2000 reagent (Invitrogen) according to the manufacturer's protocol. One flask of cells is transfected with the expression library described in Examples 11 and 12 while the other flask is transfected with pcDNA3 vector as a control. Transfected cells are returned to the incubator and left overnight.
On day 3 the transfection media is removed from both flasks of transfected cells and replaced with 50ml standard media. Hydrogen peroxide is diluted in double-deionised water to make a 40X stock (for the screens with 400µM and 450µM hydrogen peroxide, these are stocks of 16mM and 18mM hydrogen peroxide, respectively). To each flask of transfected cells 1.25ml of hydrogen peroxide stock is added and mixed immediately. Flasks are returned to the incubators and left for 3 days.
On day 6 the transfected and hydrogen peroxide-treated flasks are examined to observe cell death from the hydrogen peroxide treatment. The media is removed from the flasks and surviving cells adhering to the flasks are gently washed with sterile phosphate buffered saline (PBS). Cells from both flasks are trypsinised as described above to detach them from the plastic and are collected by centrifugation in sterile 10ml tubes, which are then place immediately on ice. Total RNA is extracted from the collected cells using Trizol reagent (Invitrogen), following the manufacturer's instructions. RNA is then stored at -80°C.

To identify any peptide/s that protected the surviving HEK293 cells against oxidative stress in the library screen cDNA is made from the extracted total RNA using Omniscript (Qiagen) essentially according to manufacturer'sinstructions. The cDNA encoding the peptide is amplified by PCR using primers specific for pcDNA3 and flanking the insertion site of the nucleic acid fragment encoding the peptide. The amplified DNA is subsequently recloned into the pcDNA3 vector.

The protective effect of any candidate peptide is verified by transforming HEK293 cells with a vector encoding each individual putative protective peptide and subjecting the transformed cells to hydrogen peroxide treatment as described above, at various concentrations of hydrogen peroxide. The percentage of surviving cells in the peptide-expressing cells is compared to the percentage of surviving cells in pcDNA3-transfomed cells to assess the level of oxidative stress protection.

## Claims

1. A non-hybrid screening method for identifying a peptide that rescues a cell, tissue or non-human organism from death induced by expression of a heterologous peptide, polypeptide, protein or allele, said method comprising:
(i) using a cell, tissue or non-human organism which has been obtained or selected, wherein death of the cell, tissue or non-human organism is induced by expression of a heterologous peptide, polypeptide, protein or allele;
(ii) before inducing death of the cell, tissue or non-human organism which has been obtained and selected at (i), expressing in the cell, tissue or non-human organism or introducing into the cell, tissue or non-human organism or contacting the cell, tissue or non-human organism with a candidate peptide wherein said candidate peptide is encoded by a genome of a prokaryote organism having a genome size of less than 1700 mega-base pairs (Mbp)
(iii) inducing death of the cell, tissue or non-human organism and selecting a cell, tissue or non-human organism that survives;
(iv) identifying from a cell, tissue or non-human organism selected at (iii) the expressed or introduced peptide; and
(v) identifying a peptide that is not contained within a protein that induces survival when expressed in a cell, tissue or animal expressing the heterologous peptide, polypeptide, protein or allele,
wherein the cell is a yeast, insect, plant or mammalian cell.

2. The method according to claim 1 wherein the death of the cell, tissue or non-human organism is caused by overexpressing Aurora-A kinase or cyclin E in the cell, tissue or non-human organism.

3. The method according to claim 1 or claim 2 wherein the candidate peptide is produced by a method comprising:
(i) producing fragments from nucleic acid derived from two or more microorganisms, each microorganism having a genome size of less than 1700 mega-base pairs (Mbp) which is at least 60% sequenced;
(ii) inserting the nucleic acid fragments produced at (i) into a suitable expression construct thereby producing recombinant constructs, wherein each fragment is in operable connection with a promoter sequence that is capable of conferring expression of that fragment; and
(iii) expressing the peptide encoded by the recombinant construct (ii), thereby producing a candidate peptide.

4. The method according to claim 3 wherein the microorganisms are selected from the group consisting of *Aeropyrum pernix, Aquifex aeolicus, Archaeoglobus fulgidis, Bacillus subtilis, Bordetella pertussis, Borrelia burgdorferi, Chlamydia trachomatis, Desulfovibrio vulgaris, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis* PCC 6803, *Thermoplasma volcanium, Thermus thermophilus* and Thermotoga maritime or wherein the microorganisms are selected from the group consisting of *Archaeoglobus fulgidus, Aquifex aeolicus, Aeropyrum pernix, Bacillus subtilis, Bordetella pertussis, Borrelia burgdorferi, Chlamydia trachomatis, Escherichia coli K12, Haemophilus influenzae, Helicobacter pylori, Methanobacterium thermoautotrophicum., Methanococcus jannashii, Neisseria meningitidis, Pyrococcus horikoshii, Pseudomonas aeruginosa, Synechocystis PCC 6803, Thermoplasma volcanicum, Thermotoga maritima, Halobacterium salinarum, Desulfobacterium autotrophicum, Haloferax volcanii and Rhodopirellula baltica.*

5. The method according to claim 1 further comprising producing the cell, tissue or non-human organism by expressing a peptide, polypeptide or protein in the cell, tissue or non-human organism from an inducible promoter.

6. The method according to claim 1 for identifying a peptide capable of inhibiting cell death induced by expression of Aurora-A kinase in a yeast cell, comprising:
(i) obtaining or producing a yeast cell capable of overexpressing Aurora-A kinase;
(ii) before overexpressing Aurora-A kinase, expressing in the yeast cell or introducing into the yeast cell or contacting the yeast cell with a candidate peptide, wherein said candidate peptide is encoded by a genome of a prokaryote organism;
(iii) overexpressing Aurora-A kinase in the yeast cell and selecting a yeast cell capable of growing; and
(iv) identifying from a yeast cell selected at (iii) the expressed or introduced peptide, and identifying a peptide that it not contained within a protein that induces survival when expressed in a yeast cell overexpressing Aurora-A kinase.

7. The method according to claim 1 for identifying a peptide capable of inhibiting cell death induced by expression of cyclin-E in a yeast cell, comprising:
(i) obtaining or producing a yeast cell capable of overexpressing cyclin-E and preferably additionally expresses a cyclin dependent kinase-2;
(ii) before overexpressing cyclin-E, expressing in the yeast cell or introducing into the yeast cell or contacting the yeast cell with a candidate peptide, wherein said candidate peptide is encoded by a genome of a prokaryote organism;
(iii) overexpressing cyclin-E in the yeast cell and selecting a yeast cell capable of growing; and
(iv) identifying from a yeast cell selected at (iii) the expressed or introduced peptide, and identifying a peptide that it not contained within a protein that induces survival when expressed in a yeast cell overexpressing cyclin-E.

8. The method according to any one of claims 1 to 7 further comprising identifying a nucleic acid encoding the identified peptide and preferably additionally comprising obtaining or isolating the nucleic acid.

## Patentansprüche

1. Nicht hybrides Screening-Verfahren zur Identifikation eines Peptids, das dazu in der Lage ist, eine Zelle, ein Gewebe oder einen nicht humanen Organismus vor dem durch die Expression eines heterologen Peptids, Polypeptids, Proteins oder Allels induzierten Absterben zu bewahren, wobei das Verfahren umfasst:
(i) Verwendung einer Zelle, eines Gewebes oder eines nicht humanen Organismus, die/das/der selektioniert oder erhalten wurde, wobei das Absterben der Zelle, des Gewebes oder des nicht humanen Organismus durch die Expression eines heterologen Peptids, Polypeptids, Proteins oder Allels induziert wird;
(ii) vor dem Induzieren des Absterbens der Zelle, des Gewebes oder des nicht humanen Organismus, die/das/der gemäß (i) selektioniert oder erhalten wurde, die Expression eines Kandidatenpeptids in der Zelle, dem Gewebe oder dem nicht humanen Organismus oder das Einbringen eines Kandidatenpeptids in die Zelle, das Gewebe oder den nicht humanen Organismus oder das Kontaktieren einer Zelle, eines Gewebes oder eines nicht humanen Organismus mit einem Kandidatenpeptid, wobei das Kandidatenpeptid durch ein Genom eines prokaryotischen Organismus kodiert wird, das eine Genomgröße von weniger als 1.700 Mega-Basenpaaren (Mbp) aufweist;
(iii) Induzieren des Absterbens der Zelle, des Gewebes oder des nicht humanen Organismus und Selektionieren einer überlebenden Zelle, eines überlebenden Gewebes oder eines überlebenden nicht humanen Organismus;
(iv) Identifizieren des exprimierten oder eingebrachten Peptids aus einer Zelle, einem Gewebe oder einem nicht humanen Organismus aus der Selektion gemäß (iii); und
(v) Identifizieren eines Peptids, das nicht in einem Protein enthalten ist, welches das Überleben induziert, wenn es in einer Zelle, einem Gewebe oder einem Tier, welche/welches das heterologe Peptid, Polypeptid, Protein oder Allel exprimiert, exprimiert wird,
wobei es sich bei der Zelle um eine Hefezelle, eine Insektenzelle, eine Pflanzenzelle oder eine Säugerzelle handelt.

2. Verfahren nach Anspruch 1, wobei das Absterben der Zelle, des Gewebes oder des nicht humanen Organismus durch eine Überexpression von Aurora-A-Kinase oder Cyclin E in der Zelle, dem Gewebe oder dem nicht humanen Organismus verursacht ist.

3. Verfahren nach Anspruch 1 oder Anspruch 2, wobei das Kandidatenpeptid durch ein Verfahren hergestellt ist, welches umfasst:
(i) Produzieren von Nukleinsäurefragmenten, die aus zwei oder mehreren Mikroorganismen stammen, wobei jeder Mikroorganismus ein Genom mit einer Größe von weniger als 1.700 Mega-Basenpaaren (Mbp) aufweist, das zu mindestens 60% sequenziert ist;
(ii) Insertieren der gemäß (i) produzierten Nukleinsäurefragmente in ein geeignetes Expressionskonstrukt zum Erhalt von rekombinanten Konstrukten, wobei jedes Fragment in einer funktionellen Verbindung mit einer Promotorsequenz steht, die zu einer Vermittlung der Expression dieses Fragments in der Lage ist; und
(iii) Exprimieren des durch das rekombinante Konstrukt gemäß (ii) kodierten Peptids, wodurch ein Kandidatenpeptid produziert wird.

4. Verfahren nach Anspruch 3, wobei die Mikroorganismen ausgewählt sind aus der Gruppe bestehend aus *Aeropyrum pernix, Aquifex aeolicus, Archaeoglobus fulgidis, Bacillus subtilis, Bordetella pertussis, Borrelia burgdorferi, Chlamydia trachomatis, Desulfovibrio vulgaris, Escherichia coli, Haemophilus influenzae, Helicobacter pylori, Methanobacterium thermoautotrophicum, Methanococcus jannashii, Mycoplasma pneumoniae, Neisseria meningitidis, Pseudomonas aeruginosa, Pyrococcus horikoshii, Synechocystis PCC 6803, Thermoplasma volcanium, Thermus thermophilus* und *Thermotoga maritime* oder wobei die Mikroorganismen ausgewählt sind aus der Gruppe bestehend aus *Archaeoglobus fulgidus, Aquifex aeolicus, Aeropyrum pernix, Bacillus subtilis, Bordetella pertussis, Borrelia burgdorferi, Chlamydia trachomatis, Escherichia coli K12, Haemophilus influenzae, Helicobacter pylori, Methanobacterium thermoautotrophicum, Methanococcus jannashii, Neisseria meningitidis, Pyrococcus horikoshii, Pseudomonas aeruginosa, Synechocystis PCC 6803, Thermoplasma volcanium, Thermotoga maritima, Halobacterium salinarum, Desulfobacterium autotrophicum, Haloferax volcanii* und *Rhodopirellula baltica.*

5. Verfahren nach Anspruch 1, des Weiteren umfassend die Produktion der Zelle, des Gewebes oder des nicht humanen Organismus durch die Expression eines Peptids, Polypeptids oder Proteins in der Zelle, dem Gewebe oder dem nicht humanen Organismus mit einem induzierbaren Promotor.

6. Verfahren nach Anspruch 1 zur Identifikation eines Peptids, das zu einer Inhibition des durch die Expression von Aurora-A-Kinase induzierten Zelltods in einer Hefezelle in der Lage ist, umfassend:
(i) Erhalt oder Produktion einer Hefezelle, die zu einer Überexpression von Aurora-A-Kinase in der Lage ist;
(ii) vor der Überexpression von Aurora-A-Kinase, Expression eines Kandidatenpeptids in der Hefezelle oder Einbringen eines Kandidatenpeptids in die Hefezelle oder Kontaktieren eines Kandidatenpeptids mit der Hefezelle, wobei das Kandidatenpeptid durch ein Genom eines prokaryotischen Organismus kodiert ist;
(iii) Überexprimieren von Aurora-A-Kinase in der Hefezelle und Selektion einer Hefezelle, die zum Wachstum in der Lage ist; und
(iv) Identifizieren des exprimierten oder eingebrachten Peptids aus einer gemäß (iii) selektionierten Hefezelle sowie Identifizieren eines Peptids, das nicht in einem Protein enthalten ist, welches das Überleben induziert, wenn es in einer Hefezelle exprimiert wird, die Aurora-A-Kinase überexprimiert.

7. Verfahren nach Anspruch 1 zur Identifikation eines Peptids, das zur Inhibition des durch die Expression von Cyclin E induzierten Zelltods in der Lage ist, in einer Hefezelle, umfassend:
(i) Erhalten oder Produzieren einer Hefezelle, die zu einer Überexpression von Cyclin E in der Lage ist und bevorzugt zusätzlich dazu eine cyclinabhängige Kinase-2 exprimiert;
(ii) vor dem Überexprimieren von Cyclin E, Exprimieren eines Kandidatenpeptids in der Hefezelle oder Einbringen eines Kandidatenpeptids in die Hefezelle oder Kontaktieren der Hefezelle mit einem Kandidatenpeptid, wobei das Kandidatenpeptid durch ein Genom eines prokaryotischen Organismus kodiert ist; und
(iv) Identifizieren des exprimierten oder eingebrachten Peptids aus einer gemäß (iii) selektionierten Hefezelle sowie Identifizieren eines Peptids, das nicht in einem Protein enthalten ist, welches das Überleben induziert, wenn es in einer Hefezelle exprimiert wird, die Cyclin E überexprimiert.

8. Verfahren nach einem der Ansprüche 1 bis 7, des Weiteren umfassend die Identifikation einer Nukleinsäure, die für das identifizierte Peptid kodiert, und bevorzugt zusätzlich dazu umfassend den Erhalt oder die Isolation der Nukleinsäure.

## Revendications

1. Procédé de criblage non hybride permettant d'identifier un peptide qui protège une cellule, un tissu ou un organisme non humain de la mort induite par l'expression d'un peptide, d'un polypeptide, d'une protéine ou d'un allèle hétérologue, ledit procédé comprenant :
(i) l'utilisation d'une cellule, d'un tissu ou d'un organisme non humain qui a été obtenu(e) ou sélectionné(e), la mort de la cellule, du tissu ou de l'organisme non humain étant induite par l'expression d'un peptide, d'un polypeptide, d'une protéine ou d'un allèle hétérologue ;
(ii) avant l'induction de la mort de la cellule, du tissu ou de l'organisme non humain qui a été obtenu(e) et sélectionné(e) à l'étape (i), l'expression dans la cellule, le tissu ou l'organisme non humain d'un peptide candidat ou l'introduction dans la cellule, le tissu ou l'organisme non humain d'un peptide candidat ou la mise en contact de la cellule, du tissu ou de l'organisme non humain avec un peptide candidat, ledit peptide candidat étant codé par le génome d'un organisme procaryote ayant une taille de génome de moins de 1 700 méga-paires de bases (Mpb)
(iii) l'induction de la mort de la cellule, du tissu ou de l'organisme non humain et la sélection d'une cellule, d'un tissu ou d'un organisme non humain qui survit ;
(iv) l'identification dans une cellule, un tissu ou un organisme non humain sélectionné(e) à l'étape (iii) du peptide exprimé ou introduit ; et
(v) l'identification d'un peptide qui n'est pas contenu dans une protéine qui induit la survie lorsqu'il est exprimé dans une cellule, un tissu ou un animal exprimant le peptide, le polypeptide, la protéine ou l'allèle hétérologue,
dans lequel la cellule est une cellule de levure, d'insecte, de plante ou de mammifère.

2. Procédé selon la revendication 1 dans lequel la mort de la cellule, du tissu ou de l'organisme non humain est causée par la surexpression de la kinase Aurora-A ou de la cycline E dans la cellule, le tissu ou l'organisme non humain.

3. Procédé selon la revendication 1 ou la revendication 2 dans lequel le peptide candidat est produit par un procédé comprenant :
(i) la production de fragments d'acides nucléiques dérivés de deux ou plusieurs microorganismes, chaque microorganisme ayant une taille de génome de moins de 1 700 méga-paires de bases (Mpb), lequel est au moins 60 % séquencé ;
(ii) l'insertion des fragments d'acides nucléiques produits à l'étape (i) dans une construction d'expression adéquate pour ainsi produire des constructions recombinantes, chaque fragment étant lié de manière fonctionnelle à une séquence de promoteur qui est capable de conférer l'expression de ce fragment ; et
(iii) l'expression du peptide codé par la construction recombinante (ii), pour ainsi produire un peptide candidat.

4. Procédé selon la revendication 3 dans lequel les microorganismes sont choisis dans le groupe constitué de *Aeropyrum pernix, Aquifex aeolicus, Archaeoglobus fulgidis, Bacil*/*us subtilis, Bordetella pertussis, Borrelia burgdorferi, Chlamydia trachomatis, Desulfovibrio vulgaris, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis* PCC 6803, *Thermoplasma volcanium, Thermus thermophilus* et Thermotoga maritima ou dans lequel les microorganismes sont choisis dans le groupe constitué de *Archaeoglobus fulgidus, Aquifex aeolicus, Aeropyrum pernix, Bacillus subtilis, Bordetella pertussis, Borrelia burgdorferi, Chlamydia trachomatis, Escherichia coli K12, Haemophilus influenzae, Helicobacter pylori, Methanobacterium thermoautotrophicum, Methanococcus jannashii, Neisseria meningitidis, Pyrococcus horikoshii, Pseudomonas aeruginosa, Synechocystis PCC 6803, Thermoplasma volcanicum, Thermotoga maritima, Halobacterium salinarum, Desulfobacterium autotrophicum, Haloferax volcanii et Rhodopirellula baltica*.

5. Procédé selon la revendication 1 comprenant en outre la production de la cellule, du tissu ou de l'organisme non humain par l'expression d'un peptide, d'un polypeptide ou d'une protéine dans la cellule, le tissu ou l'organisme non humain à partir d'un promoteur inductible.

6. Procédé selon la revendication 1 permettant d'identifier un peptide capable d'inhiber la mort cellulaire induite par l'expression de la kinase Aurora-A dans une cellule de levure, comprenant :
(i) l'obtention ou la production d'une cellule de levure capable de surexprimer la kinase Aurora-A ;
(ii) avant la surexpression de la kinase Aurora-A, l'expression dans la cellule de levure d'un peptide candidat ou l'introduction dans la cellule de levure d'un peptide candidat ou la mise en contact de la cellule de levure avec un peptide candidat, ledit peptide candidat étant codé par le génome d'un organisme procaryote ;
(iii) la surexpression de la kinase Aurora-A dans la cellule de levure et la sélection d'une cellule de levure capable de se développer ; et
(iv) l'identification dans une cellule de levure sélectionnée à l'étape (iii) du peptide exprimé ou introduit, et l'identification d'un peptide qui n'est pas contenu dans une protéine qui induit la survie lorsqu'il est exprimé dans une cellule de levure surexprimant la kinase Aurora-A.

7. Procédé selon la revendication 1 permettant d'identifier un peptide capable d'inhiber la mort cellulaire induite par l'expression de la cycline E dans une cellule de levure, comprenant :
(i) l'obtention ou la production d'une cellule de levure capable de surexprimer la cycline E et de préférence qui exprime en outre une kinase 2 dépendante de la cycline ;
(ii) avant la surexpression de la cycline E, l'expression dans la cellule de levure d'un peptide candidat ou l'introduction dans la cellule de levure d'un peptide candidat ou la mise en contact de la cellule de levure avec un peptide candidat, ledit peptide candidat étant codé par le génome d'un organisme procaryote ;
(iii) la surexpression de la cycline E dans la cellule de levure et la sélection d'une cellule de levure capable de se développer ; et
(iv) l'identification dans une cellule de levure sélectionnée à l'étape (iii) du peptide exprimé ou introduit, et l'identification d'un peptide qui n'est pas contenu dans une protéine qui induit la survie lorsqu'il est exprimé dans une cellule de levure surexprimant la cycline E.

8. Procédé selon l'une quelconque des revendications 1 à 7 comprenant en outre l'identification d'un acide nucléique codant pour le peptide identifié et de préférence comprenant en outre l'obtention ou l'isolement de l'acide nucléique.
